(19) Europäisches Patentamt / European Patent Office / Office européen des brevets

(11) **EP 4 399 025 B1**

(12) **FASCICULE DE BREVET EUROPEEN**

(45) Date de publication et mention de la délivrance du brevet:
**06.08.2025 Bulletin 2025/32**

(21) Numéro de dépôt: **22785938.6**

(22) Date de dépôt: **09.09.2022**

(51) Classification Internationale des Brevets (IPC):
***B01J 31/16*** *(2006.01)* ***B01J 31/18*** *(2006.01)*
***B01J 31/22*** *(2006.01)* ***B01J 31/24*** *(2006.01)*
***C07C 67/36*** *(2006.01)* ***C07C 231/00*** *(2006.01)*
***C07C 231/12*** *(2006.01)*

(52) Classification Coopérative des Brevets (CPC):
(C-Sets disponibles)
**B01J 31/2273; B01J 31/1658; B01J 31/181; B01J 31/2234; B01J 31/2404; C07C 67/36; C07C 231/12;** B01J 2231/341; B01J 2531/0216; B01J 2531/824; B01J 2531/828 (Cont.)

(86) Numéro de dépôt international:
**PCT/EP2022/075152**

(87) Numéro de publication internationale:
**WO 2023/036955 (16.03.2023 Gazette 2023/11)**

(54) **NOUVEAU PROCÉDÉ DE PRÉPARATION DE COMPOSÉS OXALATES ET OXAMIDES PAR VOIE CATALYTIQUE**

NEUES VERFAHREN ZUR KATALYTISCHEN HERSTELLUNG VON OXALAT- UND OXAMIDVERBINDUNGEN

NEW PROCESS FOR CATALYTIC PREPARATION OF OXALATE AND OXAMIDE COMPOUNDS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **10.09.2021 FR 2109523**

(43) Date de publication de la demande:
**17.07.2024 Bulletin 2024/29**

(73) Titulaire: **Fairbrics**
**92110 Clichy (FR)**

(72) Inventeurs:
- **NASRALLAH, Ali**
  **91940 Les Ulis (FR)**
- **KRACHKO, Tetiana**
  **91300 Massy (FR)**
- **NASR ALLAH, Tawfiq**
  **75013 Paris (FR)**

(74) Mandataire: **Cabinet Grosset-Fournier & Demachy**
**54, rue Saint Lazare**
**75009 Paris (FR)**

(56) Documents cités:
**CN-A- 109 746 043** **JP-A- 2014 088 380**
**US-A- 4 005 129** **US-A- 4 614 832**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

(52) Classification Coopérative des Brevets (CPC):
(Cont.)

C-Sets
**C07C 67/36, C07C 69/36;**
**C07C 231/12, C07C 235/80**

## Description

La présente invention concerne un nouveau procédé de préparation de composés oxalates et oxamides par voie catalytique.

## CONTEXTE DE L'INVENTION

Les oxalates et les oxamides sont des matières premières importantes dans l'industrie chimique organique, qui sont utilisées à grande échelle pour produire divers colorants, médicaments, solvants importants, agents d'extraction et divers intermédiaires dans l'industrie de la chimie fine.

Par exemple, l'hydrogénation des oxalates et des oxamides peut produire de l'éthylène glycol qui est une matière première importante dans l'industrie chimique.

Une méthode de production traditionnelle des oxalates utilise l'estérification de l'acide oxalique avec des alcools. Cette technique de production est coûteuse, énergivore, polluante et entraine une utilisation déraisonnable de matières premières.

Une méthode de production traditionnelle des oxamides s'appuie sur la réaction de l'acide oxalique, ou d'un dérivé de celui-ci, avec une amine. Cependant, cette méthode présente des inconvénients, car elle implique l'utilisation de réactifs coûteux ou toxiques et corrosifs. L'utilisation de tels composés de départ hautement réactifs conduit facilement à des réactions secondaires indésirables abaissant la sélectivité de la réaction et les rendements.

Les composés oxalates et oxamides peuvent être synthétisés directement par carbonylation oxydative d'un alcool ou d'une amine respectivement, en présence d'un catalyseur au palladium (Pd) ou au platine (Pt).

Une étape cruciale dans la réaction oxydative industrielle catalysée par le palladium ou le platine est la régénération efficace de l'atome métallique au degré d'oxydation +2 à partir de l'atome métallique au degré d'oxydation 0 qui est réduit lors de la réaction. En général, il est assez difficile de réoxyder directement le Pd(0) en Pd(II) ou Pt(0) en Pt(II) par l'oxygène moléculaire, ainsi un oxydant supplémentaire est généralement utilisé.

La réoxydation du Pd(0) ou Pt(0) peut être réalisée par des couples redox de métaux ou par benzoquinones. Les principaux inconvénients sont :

(1) l'utilisation d'un excès de métaux qui entraine des dommages corrosifs sur les installations et
(2) la difficulté de séparer le produit de réaction du l'oxydant (par exemple lorsque la benzoquinone est utilisée comme oxydant, de l'hydroquinone est formé comme sous-produit). De plus, les procédés de l'art antérieur relatifs à la préparation des oxalates sont effectués dans des conditions anhydres et éventuellement avec un agent déshydratant, puisque la production d'un oxalate est empêchée par l'eau formée in situ lorsque l'oxygène est utilisé comme oxydant ou lorsque l'eau est présente dans les réactifs ou solvants. En effet l'eau désactive le catalyseur.

Une autre approche de la synthèse d'oxalate repose sur l'utilisation de nitrites d'alkyle (RONO), des composés explosifs, qui d'une part agissent comme des agents réoxydants efficaces pour le Pd(0) et d'autre part agissent par leur fonction alcoxy (RO) comme de bons nucléophiles pour le CO. Cependant des réactions secondaires se produisent, en particulier la production d'acide nitrique.

Les méthodes de l'art antérieur divulguent l'utilisation des complexes organométalliques de palladium ou de platine comme catalyseurs homogènes pour la carbonylation oxydative des alcools en oxalates et des amines en oxamides. Les sels de Pd(II) tels que $PdCl_2$, $PdBr_2$, $Pd(acac)_2$, $Pd(OAc)_2$, $PdSO_4$, $Pd(NO_3)_2$ seuls ou en combinaison avec une phosphine telle que $Ph_2EtP$, $PhEt_2P$, $(PhO)_3P$, $Ph_3P$ ou avec d'autres ligands ou les sels de Pt(II) tels que $PtCl_2$ ont notamment été mis en oeuvre. US 4 005 129 A et US 4 614 832 A décrivent des procédés de carbonylation oxydative pour la préparation des oxalates en présence des catalyseurs à base de Pd ou Pt avec des ligands de type phosphites.

Cependant, bien qu'il ait été démontré que la présence de ligands dans les catalyseurs au palladium améliore les performances du catalyseur, en opérant avec des oxydants forts tels que l'$O_2$ et le RONO, les ligands donneurs de Phosphore, les P-donneurs, peuvent être facilement oxydés, entraînant la dégradation du catalyseur en produit toxiques, qui doivent notamment être éliminés par une étape de purification supplémentaire.

Depuis de nombreuses années, on recherche une voie de préparation des oxalates et des oxamides à faible coût et respectueuse de l'environnement.

L'un des buts de l'invention est l'utilisation d'un catalyseur palladium-(carbène *N*-hétérocyclique) ou d'un catalyseur platine-(carbène *N*-hétérocyclique) permettant la préparation d'oxalates ou d'oxamides.

Un autre but de la présente invention est l'utilisation d'un catalyseur palladium-(carbène *N*-hétérocyclique) ou d'un catalyseur platine-(carbène *N*-hétérocyclique) permettant d'opérer dans des conditions d'oxydation avec un oxydant fort tel que le dioxygène.

Un autre but de la présente invention est l'utilisation d'un catalyseur palladium-(carbène *N*-hétérocyclique) ou d'un catalyseur platine-(carbène *N*-hétérocyclique) qui est stable lors de la réaction de carbonylation oxydative permettant

la préparation d'oxalate et d'oxamide et qui peut être récupéré et réutilisé.

Un autre but de l'invention est l'utilisation d'un catalyseur palladium-(carbène N-hétérocyclique) ou d'un catalyseur platine-(carbène *N*-hétérocyclique), avantageusement immobilisé sur un support, permettant d'obtenir un catalyseur sous forme supportée recyclable et performant.

L'un des buts de l'invention dans le cas particulier de la préparation d'oxalates, concerne l'utilisation d'un catalyseur palladium-(carbène *N*-hétérocyclique) ou d'un catalyseur platine-(carbène *N*-hétérocyclique) dans un procédé de carbonylation oxydative, dans des conditions particulières, pouvant permettre d'éviter l'utilisation :

- de réactifs nocifs et toxiques tel que l'oxyde nitrique (NO),
- d'agents déshydratants,
- de co catalyseurs de métal de transition, ou
- de ligands P-donneurs.

Un autre but de l'invention est l'utilisation d'un catalyseur palladium-(carbène *N*-hétérocyclique) ou d'un catalyseur platine-(carbène *N*-hétérocyclique), dans un procédé de carbonylation oxydative d'un alcool ou d'une amine conduisant à un rendement efficace.

Un autre but de l'invention est l'utilisation d'un catalyseur palladium-(carbène *N*-hétérocyclique) ou d'un catalyseur platine-(carbène *N*-hétérocyclique), dans un procédé de carbonylation oxydative, dont la sélectivité est supérieure à 80% en oxalates ou en oxamides. Un autre but de l'invention est un procédé de préparation d'oxalates et d'oxamides, en présence d'un catalyseur palladium-(carbène *N*-hétérocyclique) ou d'un catalyseur platine-(carbène *N*-hétérocyclique).

**Utilisation**

**Un premier objet** de la présente invention est l'utilisation d'un catalyseur M-NHC, dans lequel M représente Pd ou Pt et NHC représente un groupement carbène *N*-hétérocyclique, comprenant au moins un atome M lié à au moins un ligand carbène *N*-hétérocyclique, dans la mise en oeuvre d'un procédé de préparation sélective d'oxalates ou d'oxamides, à partir de monoxyde de carbone, d'un oxydant, en particulier l'oxygène moléculaire ou l'air, et d'un alcool ou d'une amine respectivement, éventuellement en présence d'un promoteur.

Au sens de la présente invention, on entend par « oxalate » le dialkyloxalate correspondant à l'alcool utilisé.

On entend par « oxamide » le dérivé d'oxamide 1,1'-oxalyl diamine correspondant à l'amine utilisée.

On entend par catalyseur de M-NHC, un complexe organométallique comprenant au moins un centre métallique M constitué par un atome de palladium ou de platine comme site catalytique, ledit centre métallique étant lié à au moins un ligand carbène *N*-hétérocyclique.

On entend par « carbène *N*-hétérocyclique » ou NHC, une espèce moléculaire possédant un carbone divalent à 6 électrons de valence compris dans un hétérocycle contenant au moins un atome d'azote.

À titre d'exemples non limitatifs, les catalyseurs suivants entrent aussi dans la définition selon l'invention :

- un complexe qui comprend un centre M lié à un ligand NHC,
- un complexe qui comprend un centre M lié à deux ligands NHC,
- un complexe qui comprend deux centres M et deux ligands NHC,
- un complexe qui comprend deux centres M et 4 ligands NHC,
- un complexe qui comprend n centres M et n ligands NHC, *n* étant un nombre entier de 1 à 1000, notamment de 1 à 10, et
- un complexe qui comprend *n* centre M et *2n* ligands NHC, *n* étant un nombre entier de 1 à 1000, notamment de 1 à 10.

On comprend au vu de la valeur du nombre entier *n*, que le catalyseur selon l'invention peut être un polymère.

Selon un mode de réalisation particulier, l'invention concerne l'utilisation d'un catalyseur M-NHC, dans la mise en œuvre d'un procédé de préparation sélective d'oxalates ou d'oxamides, à partir de monoxyde de carbone, d'un oxydant, et d'un alcool ou d'une amine respectivement. Selon un mode de réalisation particulier, l'invention concerne l'utilisation d'un catalyseur M-NHC, dans la mise en œuvre d'un procédé de préparation sélective d'oxalates, à partir de monoxyde de carbone, d'un oxydant et d'un alcool.

Selon un mode de réalisation particulier, l'invention concerne l'utilisation d'un catalyseur M-NHC, dans la mise en œuvre d'un procédé de préparation sélective d'oxamides, à partir de monoxyde de carbone, d'un oxydant, et d'une amine.

Avantageusement, l'invention concerne l'utilisation d'un catalyseur au palladium Pd-NHC.

Selon un mode de réalisation particulier, l'invention concerne l'utilisation d'un catalyseur Pd-NHC, dans la mise en œuvre d'un procédé de préparation sélective d'oxalates ou d'oxamides, à partir de monoxyde de carbone, d'un oxydant et d'un alcool ou d'une amine respectivement. Selon un mode de réalisation particulier, l'invention concerne l'utilisation

d’un catalyseur Pd-NHC, dans la mise en œuvre d’un procédé de préparation sélective d’oxalates, à partir de monoxyde de carbone, d’un oxydant et d’un alcool.

Selon un mode de réalisation particulier, l’invention concerne l’utilisation d’un catalyseur Pd-NHC, dans la mise en œuvre d’un procédé de préparation sélective d’oxamides, à partir de monoxyde de carbone, d’un oxydant et d’une amine.

Avantageusement, l’invention concerne l’utilisation d’un catalyseur au platine Pt-NHC.

Selon un mode de réalisation particulier, l’invention concerne l’utilisation d’un catalyseur Pt-NHC, dans la mise en œuvre d’un procédé de préparation sélective d’oxalates ou d’oxamides, à partir de monoxyde de carbone, d’un oxydant et d’un alcool ou d’une amine respectivement. Selon un mode de réalisation particulier, l’invention concerne l’utilisation d’un catalyseur Pt-NHC, dans la mise en œuvre d’un procédé de préparation sélective d’oxalates, à partir de monoxyde de carbone, d’un oxydant et d’un alcool.

Selon un mode de réalisation particulier, l’invention concerne l’utilisation d’un catalyseur Pt-NHC, dans la mise en œuvre d’un procédé de préparation sélective d’oxamides, à partir de monoxyde de carbone, d’un oxydant et d’une amine.

Selon un mode de réalisation particulier l’invention concerne l’utilisation telle que définie ci-dessus dans laquelle l’oxydant est choisi parmi : l’oxygène moléculaire ($O_2$), l’air, une dione en particulier la 1,4-benzoquinone, la 1,4-dichloro-2-butène et $CuCl_2$.

Au sens de la présente invention l’air est défini comme un oxydant. L’air est une composition de gaz comprenant en fraction molaire environ 78 % de diazote ($N_2$), 21 % de dioxygène $O_2$ et environ moins de 1% d’autres gaz dont le dioxyde de carbone ($CO_2$), du méthane ($CH_4$) et des gaz rares dont l’argon, l’hélium, le néon, le krypton et le xénon. Le diazote étant un gaz inerte, on comprend que la réactivité oxydante de l’air est gouvernée par celle du dioxygène. Le dioxygène est appelé aussi oxygène moléculaire ou oxygène dans la présente invention. Avantageusement, l’invention concerne l’utilisation telle que définie ci-dessus dans laquelle l’oxygène moléculaire ou l’air est utilisé comme oxydant.

Selon un mode de réalisation particulier, l’invention concerne l’utilisation d’un catalyseur M-NHC, dans la mise en œuvre d’un procédé de préparation sélective d’oxalates ou d’oxamides, à partir de monoxyde de carbone, d’oxygène moléculaire ou d’air et d’un alcool ou d’une amine respectivement.

Selon un mode de réalisation particulier, l’invention concerne l’utilisation d’un catalyseur M-NHC, dans la mise en œuvre d’un procédé de préparation sélective d’oxalates, à partir de monoxyde de carbone, d’oxygène moléculaire ou d’air et d’un alcool.

Selon un mode de réalisation particulier, l’invention concerne l’utilisation d’un catalyseur M-NHC, dans la mise en œuvre d’un procédé de préparation sélective d’oxamides, à partir de monoxyde de carbone, d’oxygène moléculaire ou d’air et d’une amine.

Avantageusement, l’invention concerne l’utilisation telle que définie ci-dessus d’un catalyseur Pd-NHC dans laquelle l’oxygène moléculaire ou l’air est utilisé comme oxydant.

Selon un mode de réalisation particulier, l’invention concerne l’utilisation d’un catalyseur Pd-NHC, dans la mise en œuvre d’un procédé de préparation sélective d’oxalates ou d’oxamides, à partir de monoxyde de carbone, d’oxygène moléculaire ou d’air et d’un alcool ou d’une amine respectivement.

Selon un mode de réalisation particulier, l’invention concerne l’utilisation d’un catalyseur Pd-NHC dans la mise en œuvre d’un procédé de préparation sélective d’oxalates à partir de monoxyde de carbone, d’oxygène moléculaire ou d’air et d’un alcool.

Selon un mode de réalisation particulier, l’invention concerne l’utilisation d’un catalyseur Pd-NHC dans la mise en œuvre d’un procédé de préparation sélective d’oxamides, à partir de monoxyde de carbone, d’oxygène moléculaire ou d’air et d’une amine.

Avantageusement, l’invention concerne l’utilisation telle que définie ci-dessus d’un catalyseur Pt-NHC dans laquelle l’oxygène moléculaire ou l’air est utilisé comme oxydant.

Selon un mode de réalisation particulier, l’invention concerne l’utilisation d’un catalyseur Pt-NHC, dans la mise en œuvre d’un procédé de préparation sélective d’oxalates ou d’oxamides, à partir de monoxyde de carbone, d’oxygène moléculaire ou d’air et d’un alcool ou d’une amine respectivement.

Selon un mode de réalisation particulier, l’invention concerne l’utilisation d’un catalyseur Pt-NHC dans la mise en œuvre d’un procédé de préparation sélective d’oxalates à partir de monoxyde de carbone, d’oxygène moléculaire ou d’air et d’un alcool.

Selon un mode de réalisation particulier, l’invention concerne l’utilisation d’un catalyseur Pt-NHC dans la mise en œuvre d’un procédé de préparation sélective d’oxamides, à partir de monoxyde de carbone, d’oxygène moléculaire ou d’air et d’une amine.

Avantageusement, l’invention concerne l’utilisation telle que définie ci-dessus d’un catalyseur Pd-NHC, en présence d’un promoteur.

Selon un mode de réalisation particulier, l’invention concerne l’utilisation d’un catalyseur Pd-NHC dans la mise en œuvre d’un procédé de préparation sélective d’oxalates ou d’oxamides, à partir de monoxyde de carbone, d’un oxydant

et d'un alcool ou d'une amine respectivement, en présence d'un promoteur.

Selon un mode de réalisation particulier, l'invention concerne l'utilisation d'un catalyseur Pd-NHC dans la mise en œuvre d'un procédé de préparation sélective d'oxalates, à partir de monoxyde de carbone, d'un oxydant et d'un alcool, en présence d'un promoteur.

Selon un mode de réalisation particulier, l'invention concerne l'utilisation d'un catalyseur Pd-NHC dans la mise en œuvre d'un procédé de préparation sélective d'oxamides, à partir de monoxyde de carbone, d'un oxydant et d'une amine, en présence d'un promoteur.

Avantageusement, l'invention concerne l'utilisation telle que définie ci-dessus d'un catalyseur Pd-NHC, dans laquelle l'oxygène moléculaire ou l'air est utilisé comme oxydant, en présence d'un promoteur.

Selon un mode de réalisation particulier, l'invention concerne l'utilisation d'un catalyseur Pd-NHC dans la mise en œuvre d'un procédé de préparation sélective d'oxalates ou d'oxamides, à partir de monoxyde de carbone, d'oxygène moléculaire ou d'air et d'un alcool ou d'une amine respectivement, en présence d'un promoteur.

Selon un mode de réalisation particulier, l'invention concerne l'utilisation d'un catalyseur Pd-NHC dans la mise en œuvre d'un procédé de préparation sélective d'oxalates, à partir de monoxyde de carbone, d'oxygène moléculaire ou d'air et d'un alcool, en présence d'un promoteur.

Selon un mode de réalisation particulier, l'invention concerne l'utilisation d'un catalyseur Pd-NHC dans la mise en œuvre d'un procédé de préparation sélective d'oxamides, à partir de monoxyde de carbone, d'oxygène moléculaire ou d'air et d'une amine, en présence d'un promoteur.

Avantageusement le catalyseur pour l'utilisation selon l'invention peut être choisi de façon à ne comprendre aucun ligand phosphoré.

Selon un mode de réalisation particulier, l'invention concerne l'utilisation d'un catalyseur M-NHC ne présentant pas de ligands phosphorés, en particulier de groupements phosphine.

Au contraire des complexes M-Phosphines dans lesquels les ligands phosphines P-donneurs s'oxydent conduisant à la formation de produits de dégradation lors de la catalyse, la stabilité des catalyseurs M-NHC permet une réutilisation des catalyseurs et une stabilité du catalyseur dans un milieu réactionnel oxydant.

*Catalyseur supporté*

Avantageusement il est possible de lier le catalyseur M-NHC à un support.

Selon un mode de réalisation avantageux, l'invention concerne l'utilisation telle que définie ci-dessus d'un catalyseur supporté M-NHC dans la mise en œuvre d'un procédé de préparation sélective d'oxalates ou d'oxamides respectivement à partir de monoxyde de carbone, d'un oxydant et d'un alcool ou d'une amine respectivement.

Selon un mode de réalisation particulier, l'invention concerne l'utilisation telle que définie ci-dessus d'un catalyseur supporté M-NHC dans la mise en œuvre d'un procédé de préparation sélective d'oxalates à partir de monoxyde de carbone, d'un oxydant et d'un alcool.

Selon un mode de réalisation particulier, l'invention concerne l'utilisation telle que définie ci-dessus d'un catalyseur supporté M-NHC dans la mise en œuvre d'un procédé de préparation sélective d'oxamides à partir de monoxyde de carbone, d'un oxydant et d'une amine.

Par « catalyseur supporté M-NHC », on entend que le catalyseur M-NHC est lié à un support. L'association entre le complexe organométallique et le support peut être réalisé de différentes façons.

L'association entre le catalyseur et le support peut être par liaison chimique, incluant les liaisons covalentes, les liaisons ioniques et/ou les interactions intermoléculaires telles que les liaisons hydrogène. De préférence, le catalyseur est lié de façon covalente au support. Selon un mode de réalisation particulier, le catalyseur est lié audit support par au moins un de ses ligands, permettant ainsi l'accessibilité au centre métallique.

Selon un mode de réalisation particulier, le catalyseur est lié audit support à travers le groupement carbène *N*-hétérocyclique.

Dans un mode de réalisation, le support est un oxyde en particulier de la silice, un polymère, un matériau carboné tel que les nanotubes de carbone et l'oxyde de graphène, ou des nanoparticules magnétiques, de préférence un polymère ou de la silice.

Dans un mode de réalisation, le support est sous forme de billes.

A titre d'exemples non limitatifs le support est constitué de billes de polystyrène ou de billes de silice, par exemple sous forme de gel de silice.

L'utilisation d'un catalyseur supporté a pour avantage de faciliter la séparation du catalyseur avec le milieu réactionnel, permettant aisément de récupérer et de réutiliser le catalyseur. L'utilisation d'un catalyseur supporté a aussi pour avantage de permettre de fixer dans le réacteur le catalyseur dans une enceinte telle qu'une cartouche, lorsqu'on opère sous flux continu et ainsi d'obtenir des produits en sorti du réacteur exempt de catalyseur.

Catalyseur M-NHC

Avantageusement, à titre non limitatif, l'utilisation selon l'invention telle que définie ci-dessus peut être mise en œuvre avec des catalyseurs M-NHC de formules spécifiques, définies ci-dessous.

*Catalyseur M-(carbène N-hétérocyclique) comprenant un centre métallique M lié à un ligand NHC.*

Selon un mode de réalisation particulier, l'invention concerne l'utilisation telle que définie ci-dessus, dans laquelle le catalyseur répond à la Formule I :

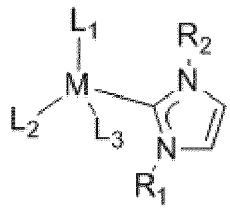

Formule I

dans laquelle :

M représente Pd ou Pt,
$R_1$ et $R_2$ représentent indépendamment l'un de l'autre un groupement choisi parmi :

- alkyle en $C_1$ à $C_{10}$, linéaire ou ramifié,
- cycloalkyle en $C_3$ à $C_{10}$,
- aryle en $C_6$ à $C_{20}$ ou hétéroaryle en $C_3$ à $C_{20}$, et
- alkyl-aryle en $C_7$ à $C_{20}$ ou alkyl-hétéroaryle en $C_4$ à $C_{20}$,

$L_1$ représente un atome d'halogène choisi parmi Cl, Br et I, et

- soit $L_2$ et $L_3$ sont liés et représentent ensemble un ligand bidentate,
- soit $L_2$ représente un atome d'halogène choisi parmi Cl, Br et I, et $L_3$ représente un ligand monodentate,

ledit composé de Formule I pouvant être lié à un support, notamment un polymère ou de la silice, par au moins un des groupements $R_1$ ou $R_2$.

Selon un mode de réalisation particulier, l'invention concerne l'utilisation telle que définie ci-dessus, ledit composé de Formule I étant lié à un support, notamment un polymère ou de la silice, par au moins un des groupements $R_1$ ou $R_2$.

Selon un mode de réalisation particulier, l'invention concerne l'utilisation telle que définie ci-dessus, dans laquelle le catalyseur répond à la Formule I , dans laquelle :
$L_1$ représente un atome d'iode, et

- soit $L_2$ et $L_3$ sont liés et représentent ensemble un ligand bidentate,
- soit $L_2$ représente un atome d'halogène choisi parmi Cl, Br et I et $L_3$ représente un ligand monodentate.

Les Inventeurs ont découvert de façon surprenante que l'utilisation d'un catalyseur portant un ligand iodé présente l'avantage de s'affranchir de la présence d'un promoteur, notamment d'un promoteur iodé tel que l'iodure de tétrabutylammonium ($Bu_4NI$).

Au sens de la présente invention, on entend par «alkyle en $C_1$ à $C_{10}$, linéaire ou ramifié» une chaîne carbonée acyclique, saturée, linéaire ou ramifiée, comprenant 1 à 10 atomes de carbone. Il s'agit des groupements méthyle, éthyle, propyle, butyle, pentyle, hexyle, heptyle, octyle, nonyle et décyle. La définition de propyle, butyle, pentyle, hexyle, heptyle, octyle, nonyle et décyle inclut tous les isomères possibles. Par exemple, le terme butyle comprend *n*-butyle, *iso*-butyle, *sec*-butyle et *ter*-butyle. Un ou plusieurs atomes d'hydrogène peuvent être remplacés dans la chaîne alkyle.

On entend par « cycloalkyle en $C_3$ à $C_{10}$ » : un groupe cyclopropyle en $C_3$, un groupe cyclobutyle en $C_4$, un groupe cyclopentyle en $C_5$, un groupe cyclohexyle en $C_6$, un groupe cycloheptyle en $C_7$, un groupe cyclooctyle en $C_8$, un groupe cyclononyle en $C_9$, ou un groupe cyclodécyle en $C_{10}$, et les cycles de cycloalcanes fusionnés tels que l'adamantyle.

Le terme «aryle en $C_6$ à $C_{20}$ » désigne un groupe aromatique comprenant 6 à 20 atomes de carbone au sein du cycle aromatique, notamment de 6 à 12 atomes de carbone, en particulier comprenant 6, 7, 8, 9, 10, 11, 12, 13, 14, 15,16,

17, 18, 19 ou 20 atomes de carbone. Les groupes aryles selon la présente invention peuvent également être substitués, notamment par un ou plusieurs substituants choisis parmi un groupe alkyle linéaire ou ramifié en $C_1$ à $C_{10}$.

Phényle, toluyle, anisyle et naphtyle *o*-tolyle, *m*-tolyle, *p*-tolyle, *o*-xylyle, *m*-xylyle, *p*-xylyle, sont des exemples de groupes aryles.

Le terme *« hétéroaryle »* désigne un groupe aryle tel que défini ci-dessus, comprenant des atomes autres que les atomes de carbone, en particulier N, O ou S au sein du cycle aromatique. Pyridyle, imidazoyle, furfuryle ou furanyle sont des exemples de groupes hétéroaryles selon la présente invention.

Le terme «alkyl-aryle en $C_7$ à $C_{20}$ » désigne une chaine linéaire alkyle de formule $-(CH_2)_m-$, m variant de 1 à 14, liée à un groupement aryle tel que défini ci-dessus, le groupement alkyl-aryle étant constitué de 7 à 20 atomes de carbones.

Le terme «alkyl-hétéroaryle en $C_4$ à $C_{20}$ » désigne une chaine linéaire alkyle de formule-$(CH_2)_m-$, m variant de 1 à 16, liée à un groupement hétéroaryle tel que défini ci-dessus, le groupement alkyl-hétéroaryle étant constitué de 4 à 20 atomes de carbones.

Avantageusement, les groupements $R_1$ et $R_2$ peuvent être choisis indépendamment l'une de l'autre parmi les groupements suivants : méthyle, *iso*-propyle, *tert*-butyle, cyclohexyle, adamantyle, mésityl (2,4,6-triméthylphényle) et diisopropylphényle.

Dans un mode de réalisation les groupements $R_1$ et $R_2$ sont identiques. Dans un autre mode de réalisation les groupements $R_1$ et $R_2$ sont différents.

On entend par « ligand bidentate » un groupement moléculaire, chargé ou neutre, formant deux liaisons avec le centre métallique M, notamment à travers deux atomes du groupement moléculaire.

On entend par « ligand monodentate » un groupement moléculaire, chargé ou neutre, formant une liaison avec le centre métallique M, notamment à travers un atome du groupement moléculaire.

Dans un mode de réalisation, les ligands $L_1$, $L_2$ et $L_3$ ne contiennent pas de Phosphore. Avantageusement le catalyseur ne contient pas de ligand phosphine.

Les groupements $R_1$ ou $R_2$ peuvent permettre de lier le catalyseur à un support afin de former un catalyseur supporté.

Dans un mode de réalisation particulier, l'un au moins des groupements $R_1$ et $R_2$ du catalyseur est lié à un support, de préférence par liaison covalente. Le catalyseur utilisé est un catalyseur supporté.

Dans un mode de réalisation particulier, le support est un polymère, notamment du polystyrène (PS).

Dans un mode de réalisation particulier, le support est de la silice, notamment un gel de silice.

Dans un mode de réalisation particulier, les groupements $R_1$ et $R_2$ du catalyseur ne sont pas liés à un support. Le catalyseur utilisé est un catalyseur homogène.

*Catalyseur palladium-(carbène N-hétérocyclique) comprenant un Pd lié à un ligand NHC.*

Selon un mode de réalisation particulier, l'invention concerne l'utilisation telle que définie ci-dessus, dans laquelle le catalyseur répond à la Formule II :

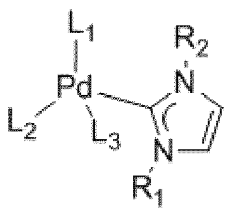

Formule II

dans laquelle :

$R_1$ et $R_2$ représentent indépendamment l'un de l'autre un groupement choisi parmi :

- alkyle en $C_1$ à $C_{10}$, linéaire ou ramifié,
- cycloalkyle en $C_3$ à $C_{10}$,
- aryle en $C_6$ à $C_{20}$ ou hétéroaryle en $C_3$ à $C_{20}$, et
- alkyl-aryle en $C_7$ à $C_{20}$ ou alkyl-hétéroaryle en $C_4$ à $C_{20}$,

$L_1$ représente un atome d'halogène choisi parmi Cl, Br et I, et

- soit $L_2$ et $L_3$ sont liés et représentent ensemble un ligand bidentate,
- soit $L_2$ représente un atome d'halogène choisi parmi Cl, Br et I, et $L_3$ représente un ligand monodentate,

ledit composé de Formule II pouvant être lié à un support, notamment un polymère ou de la silice, par au moins un des groupements $R_1$ ou $R_2$.

Selon un mode de réalisation particulier, l'invention concerne l'utilisation telle que définie ci-dessus, ledit composé de Formule II étant lié à un support, notamment un polymère ou de la silice, par au moins un des groupements $R_1$ ou $R_2$.

Selon un mode de réalisation particulier, l'invention concerne l'utilisation telle que définie ci-dessus, dans laquelle le catalyseur répond à la Formule II , dans laquelle :
$L_1$ représente un atome d'iode, et

- soit $L_2$ et $L_3$ sont liés et représentent ensemble un ligand bidentate,
- soit $L_2$ représente un atome d'halogène choisi parmi Cl, Br et I et $L_3$ représente un ligand monodentate,

Selon un mode de réalisation particulier, l'invention concerne l'utilisation telle que définie ci-dessus, dans laquelle le catalyseur répond à la Formule II , dans laquelle :

$L_1$ et $L_2$ représentent chacun un atome d'iode,
et $L_3$ représente un ligand monodentate,

Selon un mode de réalisation particulier, l'invention concerne l'utilisation telle que définie ci-dessus, dans laquelle le ligand bidentate est choisi parmi : l'acétylacétonate (acac), l'allyle, le cinnamyle et l'acétate, le ligand bidentate étant notamment l'acétylacétonate.

Selon un mode de réalisation particulier, l'invention concerne l'utilisation telle que définie ci-dessus, dans laquelle le ligand monodentate est choisi parmi : la pyridine, la 3-chloropyridine, l'acétonitrile, la triéthylamine, ou un ligand à base de phosphine, en particulier la triphénylphosphine. De préférence, le ligand monodentate est notamment le 3-chloropyridine.

Selon un mode de réalisation particulier, l'invention concerne l'utilisation telle que définie ci-dessus, dans laquelle le ligand monodentate est choisi parmi : la pyridine, la 3-chloropyridine, l'acétonitrile, la triéthylamine. De préférence le ligand monodentate est le 3-chloropyridine.

Selon un mode de réalisation particulier, l'invention concerne l'utilisation telle que définie ci-dessus, dans laquelle le catalyseur répond à la Formule II :

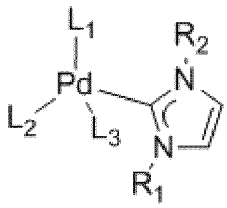

Formule II

dans laquelle :

$R_1$ et $R_2$ représentent indépendamment l'un de l'autre un groupement choisi parmi :

- alkyle en $C_1$ à $C_{10}$, linéaire ou ramifié,
- cycloalkyle en $C_3$ à $C_{10}$,
- aryle en $C_6$ à $C_{20}$ ou hétéroaryle en $C_3$ à $C_{20}$,
- alkyl-aryle en $C_7$ à $C_{20}$ ou alkyle-hétéroaryle en $C_4$ à $C_{20}$,

$L_1$ représente un atome d'halogène choisi parmi Cl, Br et I, et

- soit $L_2$ et $L_3$ représentent un ligand bidentate, en particulier choisi parmi l'acétylacétonate (acac), l'allyle, le cinnamyle et l'acétate, de préférence l'acétylacétonate,
- soit $L_2$ représente un atome d'halogène choisi parmi Cl, Br et I,

et $L_3$ représente un ligand monodentate, en particulier choisi parmi : la pyridine, la 3-chloropyridine, l'acétonitrile, la triéthylamine, ou un ligand à base de phosphine, en particulier la triphénylphosphine, de préférence la 3-chloropyridine,
ledit composé de Formule II pouvant être lié à un support, notamment un polymère ou de la silice, par au moins un des groupements $R_1$ ou $R_2$.

Selon un mode de réalisation particulier, l'invention concerne l'utilisation telle que définie ci-dessus, dans laquelle le catalyseur répond à la Formule II :

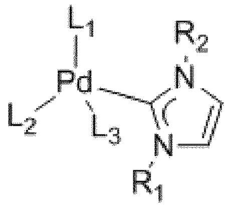

Formule II

dans laquelle :

$R_1$ et $R_2$ représentent indépendamment l'un de l'autre un groupement choisi parmi :

- alkyle en $C_1$ à $C_{10}$, linéaire ou ramifié,
- cycloalkyle en $C_3$ à $C_{10}$,
- aryle en $C_6$ à $C_{20}$ ou hétéroaryle en $C_3$ à $C_{20}$,
- alkyl-aryle en $C_7$ à $C_{20}$ ou alkyle-hétéroaryle en $C_4$ à $C_{20}$,

$L_1$ représente un atome d'iode, et

- soit $L_2$ et $L_3$ représentent un ligand bidentate, en particulier choisi parmi l'acétylacétonate (acac), l'allyle, le cinnamyle et l'acétate, de préférence l'acétylacétonate,
- soit $L_2$ représente un atome d'halogène choisi parmi Cl, Br et I et $L_3$ représente un ligand monodentate, en particulier choisi parmi : la pyridine, la 3-chloropyridine, l'acétonitrile, la triéthylamine, ou un ligand à base de phosphine, en particulier la triphénylphosphine, de préférence la 3-chloropyridine,

ledit composé de Formule II pouvant être lié à un support, notamment un polymère ou de la silice, par au moins un des groupements $R_1$ ou $R_2$.

Selon un mode de réalisation particulier, l'invention concerne l'utilisation telle que définie ci-dessus, dans laquelle le catalyseur répond à la Formule II-4 :

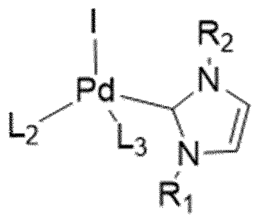

Formule II-4

dans laquelle :

$R_1$ et $R_2$ représentent indépendamment l'un de l'autre un groupement choisi parmi :

- alkyle en $C_1$ à $C_{10}$, linéaire ou ramifié,
- cycloalkyle en $C_3$ à $C_{10}$,
- aryle en $C_6$ à $C_{20}$ ou hétéroaryle en $C_3$ à $C_{20}$,
- alkyl-aryle en $C_7$ à $C_{20}$ ou alkyle-hétéroaryle en $C_4$ à $C_{20}$,

dans laquelle

- soit $L_2$ et $L_3$ représentent un ligand bidentate, en particulier choisi parmi l'acétylacétonate (acac), l'allyle, le cinnamyle et l'acétate, de préférence l'acétylacétonate,
- soit $L_2$ représente un atome d'halogène choisi parmi Cl, Br et I et $L_3$ représente un ligand monodentate, en particulier choisi parmi : la pyridine, la 3-chloropyridine, l'acétonitrile, la triéthylamine, ou un ligand à base de phosphine, en particulier la triphénylphosphine, de préférence la 3-chloropyridine,

ledit composé de Formule II-4 pouvant être lié à un support, notamment un polymère ou de la silice, par au moins un des groupements $R_1$ ou $R_2$.

Selon un mode de réalisation particulier, l'invention concerne l'utilisation telle que définie ci-dessus, dans laquelle le catalyseur répond à la Formule II :

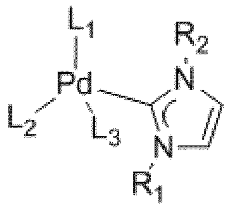

Formule II

dans laquelle :

$R_1$ et $R_2$ représentent indépendamment l'un de l'autre un groupement choisi parmi :

- alkyle en $C_1$ à $C_{10}$, linéaire ou ramifié,
- cycloalkyle en $C_3$ à $C_{10}$,
- aryle en $C_6$ à $C_{20}$ ou hétéroaryle en $C_3$ à $C_{20}$,
- alkyl-aryle en $C_7$ à $C_{20}$ ou alkyle-hétéroaryle en $C_4$ à $C_{20}$,

$L_1$ et $L_2$ représentent chacun un atome d'iode, et
$L_3$ représente un ligand monodentate, en particulier choisi parmi : la pyridine, la 3-chloropyridine, l'acétonitrile, la triéthylamine, ou un ligand à base de phosphine, en particulier la triphénylphosphine, de préférence la 3-chloropyridine,
ledit composé de Formule II pouvant être lié à un support, notamment un polymère ou de la silice, par au moins un des groupements $R_1$ ou $R_2$.

Selon un mode de réalisation particulier, l'invention concerne l'utilisation telle que définie ci-dessus, dans laquelle le catalyseur répond à la Formule II-5 :

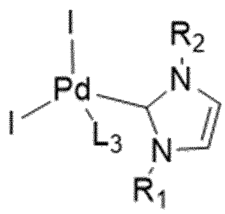

Formule II-5

dans laquelle :

$R_1$ et $R_2$ représentent indépendamment l'un de l'autre un groupement choisi parmi :

- alkyle en $C_1$ à $C_{10}$, linéaire ou ramifié,
- cycloalkyle en $C_3$ à $C_{10}$,

- aryle en $C_6$ à $C_{20}$ ou hétéroaryle en $C_3$ à $C_{20}$,
- alkyl-aryle en $C_7$ à $C_{20}$ ou alkyle-hétéroaryle en $C_4$ à $C_{20}$,

$L_3$ représente un ligand monodentate, en particulier choisi parmi : la pyridine, la 3-chloropyridine, l'acétonitrile, la triéthylamine, ou un ligand à base de phosphine, en particulier la triphénylphosphine, de préférence la 3-chloropyridine,
ledit composé de Formule II-5 pouvant être lié à un support, notamment un polymère ou de la silice, par au moins un des groupements $R_1$ ou $R_2$.

Selon un mode de réalisation particulier, l'invention concerne l'utilisation telle que définie ci-dessus, ledit composé de Formule II étant lié à un support, notamment un polymère ou de la silice, par au moins un des groupements $R_1$ ou $R_2$.
Selon un mode de réalisation particulier, l'invention concerne l'utilisation telle que définie ci-dessus, ledit composé de Formule II-4 étant lié à un support, notamment un polymère ou de la silice, par au moins un des groupements $R_1$ ou $R_2$.
Selon un mode de réalisation particulier, l'invention concerne l'utilisation telle que définie ci-dessus, ledit composé de Formule II-5 étant lié à un support, notamment un polymère ou de la silice, par au moins un des groupements $R_1$ ou $R_2$.
*Catalyseur palladium-(carbène N-hétérocyclique) comprenant un Pd lié et deux ligands NHC.* Selon un mode de réalisation particulier, l'invention concerne l'utilisation telle que définie ci-dessus, dans laquelle le catalyseur comprend deux groupements carbène N-hétérocyclique liés au palladium.
Selon un mode de réalisation particulier, l'invention concerne l'utilisation telle que définie ci-dessus, dans laquelle le catalyseur répond à la Formule III :

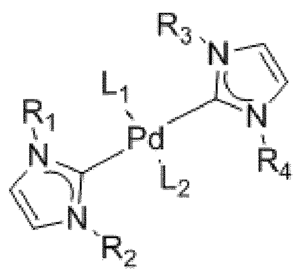

Formule III

dans laquelle :

$R_1$, $R_2$, $R_3$ et $R_4$ représentent indépendamment l'un de l'autre un groupement choisi parmi :

- alkyle en $C_1$ à $C_{10}$, linéaire ou ramifié,
- cycloalkyle en $C_3$ à $C_{10}$, et
- aryle en $C_6$ à $C_{20}$ ou hétéroaryle en $C_3$ à $C_{20}$,
- alkyl-aryle en $C_7$ à $C_{20}$ ou alkyle-hétéroaryle en $C_4$ a $C_{20}$,

soit $L_1$ et $L_2$ représentent indépendamment l'un de l'autre :

- un atome d'halogène choisi parmi Cl, Br et I, ou
- un ligand monodentate,

soit $L_1$ et $L_2$ sont liés et représentent un ligand bidentate,
ledit composé de Formule III pouvant être lié à un support, notamment un polymère ou de la silice, par au moins un des groupements $R_1$, $R_2$, $R_3$ ou $R_4$,
éventuellement l'un des groupements $R_1$ ou $R_2$ peut être relié à $R_3$ ou $R_4$ et représente ensemble un groupement bidentate comprenant deux groupements carbène N-hétérocyclique.

Selon un mode de réalisation particulier, l'invention concerne l'utilisation telle que définie ci-dessus, dans laquelle le catalyseur de Formule III comprend l'un des groupements $R_1$ ou $R_2$ relié à $R_3$ ou $R_4$ et représentent ensemble un groupement bidentate comprenant deux groupements carbène N-hétérocyclique.

Selon un mode de réalisation particulier, l'invention concerne l'utilisation telle que définie ci-dessus, dans laquelle le catalyseur répond à la Formule III dans laquelle l'un au moins des groupements $L_1$ et $L_2$ représente un atome d'iode.

Selon un mode de réalisation particulier, l'invention concerne l'utilisation telle que définie ci-dessus, dans laquelle le catalyseur répond à la Formule III-2 suivante :

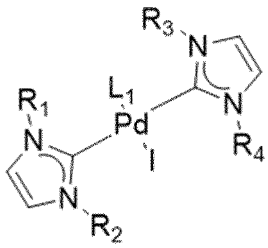

Formule III-2

dans laquelle :

$R_1$, $R_2$, $R_3$ et $R_4$ représentent indépendamment l'un de l'autre un groupement choisi parmi :

- alkyle en $C_1$ à $C_{10}$, linéaire ou ramifié,
- cycloalkyle en $C_3$ à $C_{10}$, et
- aryle en $C_6$ à $C_{20}$ ou hétéroaryle en $C_3$ à $C_{20}$,
- alkyl-aryle en $C_7$ à $C_{20}$ ou alkyle-hétéroaryle en $C_4$ a $C_{20}$,

dans laquelle $L_1$ représente :

- un atome d'halogène choisi parmi Cl, Br et I, ou
- un ligand monodentate,

ledit composé de Formule III-2 pouvant être lié à un support, notamment un polymère ou de la silice, par au moins un des groupements $R_1$, $R_2$, $R_3$ ou $R_4$,
éventuellement l'un des groupements $R_1$ ou $R_2$ peut être relié à $R_3$ ou $R_4$ et représente ensemble un groupement bidentate comprenant deux groupements carbène N-hétérocyclique.

Selon un mode de réalisation particulier, l'invention concerne l'utilisation telle que définie ci-dessus, dans laquelle le catalyseur répond à la Formule III dans laquelle les groupements $L_1$ et $L_2$ représentent chacun un atome d'iode.

Selon un mode de réalisation particulier, l'invention concerne l'utilisation telle que définie ci-dessus, dans laquelle le catalyseur répond à la Formule III-3 suivante :

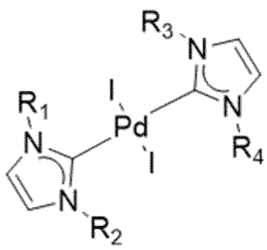

Formule III-3

dans laquelle :

$R_1$, $R_2$, $R_3$ et $R_4$ représentent indépendamment l'un de l'autre un groupement choisi parmi :

- alkyle en $C_1$ à $C_{10}$, linéaire ou ramifié,
- cycloalkyle en $C_3$ à $C_{10}$, et

- aryle en $C_6$ à $C_{20}$ ou hétéroaryle en $C_3$ à $C_{20}$,
- alkyl-aryle en $C_7$ à $C_{20}$ ou alkyle-hétéroaryle en $C_4$ a $C_{20}$,

ledit composé de Formule III-3 pouvant être lié à un support, notamment un polymère ou de la silice, par au moins un des groupements $R_1$, $R_2$, $R_3$ ou $R_4$,
éventuellement l'un des groupements $R_1$ ou $R_2$ peut être relié à $R_3$ ou $R_4$ et représente ensemble un groupement bidentate comprenant deux groupements carbène N-hétérocyclique.

Selon un mode de réalisation particulier, l'invention concerne l'utilisation telle que définie ci-dessus, ledit composé de Formule III étant lié à un support, notamment un polymère ou de la silice, par au moins un des groupements $R_1$, $R_2$, $R_3$ ou $R_4$.
Selon un mode de réalisation particulier, l'invention concerne l'utilisation telle que définie ci-dessus, ledit composé de Formule III-2 étant lié à un support, notamment un polymère ou de la silice, par au moins un des groupements $R_1$, $R_2$, $R_3$ ou $R_4$.
Selon un mode de réalisation particulier, l'invention concerne l'utilisation telle que définie ci-dessus, ledit composé de Formule III-3 étant lié à un support, notamment un polymère ou de la silice, par au moins un des groupements $R_1$, $R_2$, $R_3$ ou $R_4$.
*Dimère : Catalyseur palladium-(carbène N-hétérocyclique) comprenant deux Pd et deux ligands NHC.*
Selon un mode de réalisation particulier, l'invention concerne l'utilisation telle que définie ci-dessus, dans laquelle le catalyseur répond à la Formule IV :

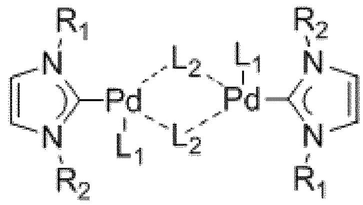

Formule IV

dans laquelle :

$R_1$ et $R_2$ représentent indépendamment l'un de l'autre un groupement choisi parmi :

- alkyle en $C_1$ à $C_{10}$, linéaire ou ramifié,
- cycloalkyle en $C_3$ à $C_{10}$,
- aryle en $C_6$ à $C_{20}$ ou hétéroaryle en $C_3$ à $C_{20}$,
- alkyl-aryle en $C_7$ à $C_{20}$ ou alkyle-hétéroaryle en $C_4$ à $C_{20}$,

$L_1$ et $L_2$ représentent un atome d'halogène choisi parmi Cl, Br et I.

On comprend dans la formule IV que les liaisons Pd---$L_2$ sont datives et que le complexe formé est un dimère de structure symétrique.
*Formules spécifiques du catalyseur palladium-(carbène N-hétérocyclique) comprenant un Pd lié à un ligand NHC.*
Selon un mode de réalisation particulier, l'invention concerne l'utilisation telle que définie ci-dessus, dans laquelle le catalyseur répond à la Formule II-1 :

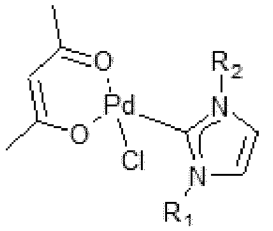

Formule II-1

dans laquelle :

$R_1$ et $R_2$ représentent indépendamment l'un de l'autre un groupement choisi parmi :

- alkyle en $C_1$ à $C_{10}$, linéaire ou ramifié,
- cycloalkyle en $C_3$ à $C_{10}$, et
- aryle en $C_6$ à $C_{20}$ ou hétéroaryle en $C_3$ à $C_{20}$,
- alkyl-aryle en $C_7$ à $C_{20}$ ou alkyle-hétéroaryle en $C_4$ à $C_{20}$,

ledit composé de Formule II-1 pouvant être lié à un support, notamment un polymère ou de la silice, par au moins un des groupements $R_1$ ou $R_2$.

Dans un mode de réalisation les groupements $R_1$ et $R_2$ du catalyseur de Formule II-1 sont identiques. Dans un autre mode de réalisation les groupements $R_1$ et $R_2$ du catalyseur de Formule II-1 sont différents.

Dans un mode de réalisation les groupements $R_1$ et $R_2$ du catalyseur de Formule II-1 ne sont pas liés à un support.

Dans un autre mode de réalisation au moins l'un des groupements $R_1$ et $R_2$ du catalyseur de Formule II-1 est lié à un support, notamment un polymère ou de la silice.

Selon un mode de réalisation particulier, l'invention concerne l'utilisation telle que définie ci-dessus, dans laquelle le catalyseur répond à la Formule II-2 :

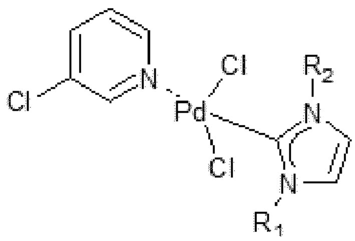

Formule II-2

**dans** laquelle :

$R_1$ et $R_2$ représentent indépendamment l'un de l'autre un groupement choisi parmi :

- alkyle en $C_1$ à $C_{10}$, linéaire ou ramifié,
- cycloalkyle en $C_3$ à $C_{10}$, et
- aryle en $C_6$ à $C_{20}$ ou hétéroaryle en $C_3$ à $C_{20}$,
- alkyl-aryle en $C_7$ à $C_{20}$ ou alkyle-hétéroaryle en $C_4$ à $C_{20}$,

ledit composé de Formule II-2 pouvant être lié à un support, notamment un polymère ou de la silice, par au moins un des groupements $R_1$ ou $R_2$.

Dans un mode de réalisation les groupements $R_1$ et $R_2$ du catalyseur de Formule II-2 sont identiques. Dans un autre mode de réalisation les groupements $R_1$ et $R_2$ du catalyseur de Formule II-2 sont différents.

Dans un mode de réalisation les groupements $R_1$ et $R_2$ du catalyseur de Formule II-2 ne sont pas liés à un support.

Dans un autre mode de réalisation au moins l'un des groupements $R_1$ et $R_2$ du catalyseur de Formule II-2 est lié à

un support, notamment un polymère ou de la silice.

Selon un mode de réalisation particulier, l'invention concerne l'utilisation telle que définie ci-dessus, dans laquelle le catalyseur répond à la Formule II-3 :

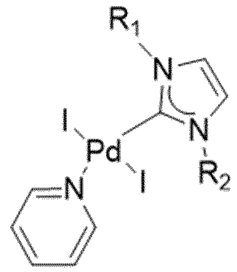

Formule II-3

dans laquelle :

$R_1$ et $R_2$ représentent indépendamment l'un de l'autre un groupement choisi parmi :

- alkyle en $C_1$ à $C_{10}$, linéaire ou ramifié,
- cycloalkyle en $C_3$ à $C_{10}$, et
- aryle en $C_6$ à $C_{20}$ ou hétéroaryle en $C_3$ à $C_{20}$,
- alkyl-aryle en $C_7$ à $C_{20}$ ou alkyle-hétéroaryle en $C_4$ à $C_{20}$,

ledit composé de Formule II-3 pouvant être lié à un support, notamment un polymère ou de la silice, par au moins un des groupements $R_1$ ou $R_2$.

Dans un mode de réalisation les groupements $R_1$ et $R_2$ du catalyseur de Formule II-3 sont identiques. Dans un autre mode de réalisation les groupements $R_1$ et $R_2$ du catalyseur de Formule II-3 sont différents.

Dans un mode de réalisation les groupements $R_1$ et $R_2$ du catalyseur de Formule II-3 ne sont pas liés à un support.

Dans un autre mode de réalisation au moins l'un des groupements $R_1$ et $R_2$ du catalyseur de Formule II-3 est lié à un support, notamment un polymère ou de la silice.

Selon un mode de réalisation particulier, l'invention concerne l'utilisation telle que définie ci-dessus, dans laquelle le catalyseur est lié à du polystyrène.

Selon un mode de réalisation particulier, l'invention concerne l'utilisation telle que définie ci-dessus, dans laquelle le catalyseur est choisi parmi :

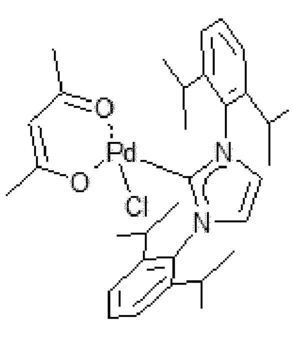

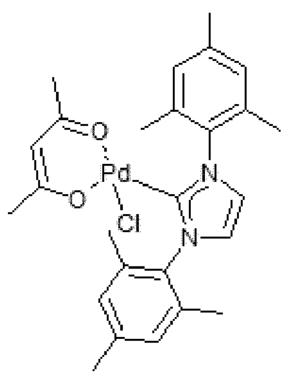

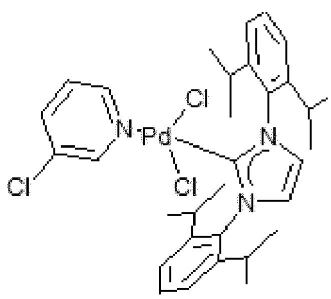

ou dans laquelle le catalyseur, lié à un support, est choisi parmi :

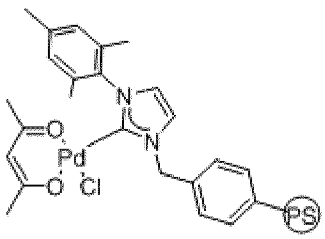

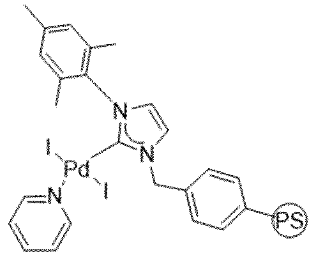

Selon un mode de réalisation particulier, l'invention concerne l'utilisation définie ci-dessus, dans laquelle le catalyseur répond à la Formule II-1 ou à la Formule II-2 ou à la Formule II-3 :

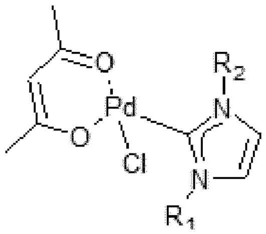

Formule II-1

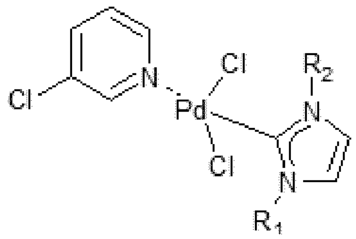

Formule II-2

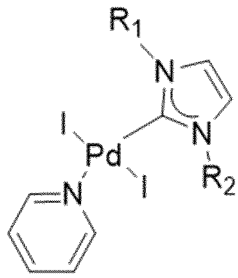

Formule II-3

**dans** laquelle :

$R_1$ et $R_2$ représentent indépendamment l'un de l'autre un groupement choisi parmi :

- alkyle en $C_1$ à $C_{10}$, linéaire ou ramifié,
- cycloalkyle en $C_3$ à $C_{10}$, et
- aryle en $C_6$ à $C_{20}$ ou hétéroaryle en $C_3$ à $C_{20}$,
- alkyl-aryle en $C_7$ à $C_{20}$ ou alkyle-hétéroaryle en $C_4$ à $C_{20}$,

ledit catalyseur pouvant être lié à un support, notamment un polymère ou de la silice, par au moins un des groupements $R_1$ ou $R_2$,
en particulier ledit catalyseur est choisi parmi :

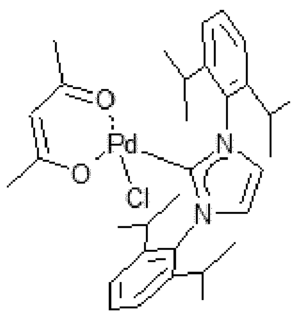

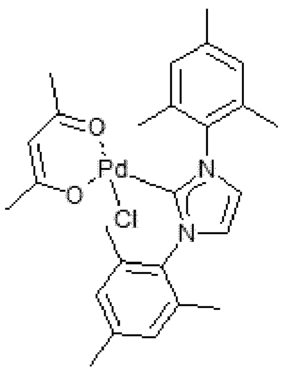

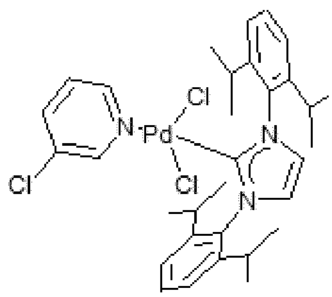

ou dans laquelle le catalyseur, lié à un support (PS) est choisi parmi :

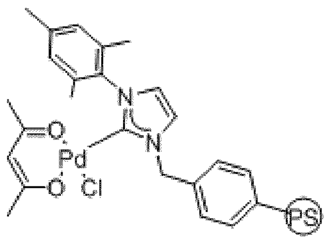

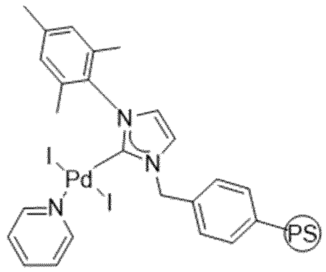

Selon un mode de réalisation particulier, l'invention concerne l'utilisation telle que définie ci-dessus, dans laquelle le catalyseur répond à la Formule III-1 :

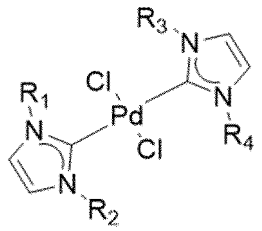

Formule III-1

dans laquelle :

$R_1$, $R_2$, $R_3$ et $R_4$ représentent indépendamment l'un de l'autre un groupement choisi parmi :

- alkyle en $C_1$ à $C_{10}$, linéaire ou ramifié,
- cycloalkyle en $C_3$ à $C_{10}$, et
- aryle en $C_6$ à $C_{20}$ ou hétéroaryle en $C_3$ à $C_{20}$,
- alkyl-aryle en $C_7$ à $C_{20}$ ou alkyle-hétéroaryle en $C_4$ a $C_{20}$,

ledit composé de Formule III-1 pouvant être lié à un support, notamment un polymère ou de la silice, par au moins un des groupements $R_1$, $R_2$, $R_3$ ou $R_4$,
éventuellement l'un des groupements $R_1$ ou $R_2$ peut être relié à $R_3$ ou $R_4$ et représente ensemble un groupement bidentate comprenant deux groupements carbène N-hétérocyclique.

Dans un mode de réalisation les groupements $R_1$, $R_2$, $R_3$ et $R_4$ du catalyseur de Formule III-1 ne sont pas liés à un support.
Selon un mode de réalisation particulier, l'invention concerne l'utilisation telle que définie ci-dessus, ledit composé de Formule III-1 étant lié à un support, notamment un polymère ou de la silice, par au moins un des groupements $R_1$, $R_2$, $R_3$ ou $R_4$.
Selon un mode de réalisation particulier, l'invention concerne l'utilisation telle que définie ci-dessus, dans lequel le catalyseur de Formule III-1, lié à un support est choisi parmi :

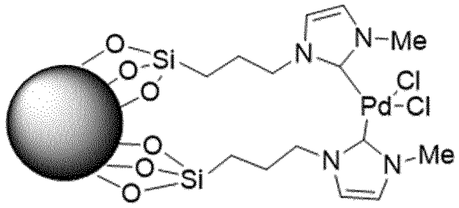

*Catalyseur platine-(carbène N-hétérocyclique) comprenant un Pt lié à un ligand NHC.*
Selon un mode de réalisation particulier, l'invention concerne l'utilisation telle que définie ci-dessus, dans laquelle le catalyseur répond à la Formule V :

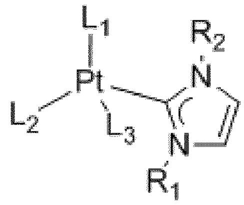

Formule V

dans laquelle :

$R_1$ et $R_2$ représentent indépendamment l'un de l'autre un groupement choisi parmi :

- alkyle en $C_1$ à $C_{10}$, linéaire ou ramifié,
- cycloalkyle en $C_3$ à $C_{10}$,
- aryle en $C_6$ à $C_{20}$ ou hétéroaryle en $C_3$ à $C_{20}$, et
- alkyl-aryle en $C_7$ à $C_{20}$ ou alkyl-hétéroaryle en $C_4$ à $C_{20}$,

$L_1$ représente un atome d'halogène choisi parmi Cl, Br et I, et

- soit $L_2$ et $L_3$ sont liés et représentent ensemble un ligand bidentate,
- soit $L_2$ représente un atome d'halogène choisi parmi Cl, Br et I, et $L_3$ représente un ligand monodentate,

ou $L_1$, $L_2$ et $L_3$ sont liés et représentent ensemble un ligand tridentate,
ledit composé de Formule V pouvant être lié à un support, notamment un polymère ou de la silice, par au moins un des groupements $R_1$ ou $R_2$.

Selon un mode de réalisation particulier, l'invention concerne l'utilisation telle que définie ci-dessus, dans laquelle le ligand bidentate est l'éthylènediamine.

Selon un mode de réalisation particulier, l'invention concerne l'utilisation telle que définie ci-dessus, dans laquelle le ligand monodentate est choisi parmi : la pyridine, la 3-chloropyridine, la cyclohexylamine, la morpholine et le diméthylsulfure, une phosphine en particulier la triphénylphosphine ($PPh_3$), l'ammoniac ($NH_3$) et le diméthylsulfoxyde (DMSO).

Selon un mode de réalisation particulier, l'invention concerne l'utilisation telle que définie ci-dessus, dans laquelle le ligand tridentate est choisi parmi la terpyridine et le diéthylènetriamine.

*Catalyseur platine-(carbène N-hétérocyclique) comprenant un Pt lié à 2 ligands NHC.*

Selon un mode de réalisation particulier, l'invention concerne l'utilisation telle que définie ci-dessus, dans laquelle le catalyseur répond à la Formule VI :

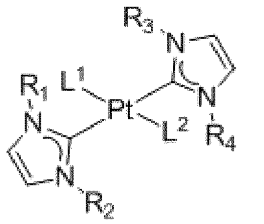

Formule VI

dans laquelle :

$R_1$, $R_2$, $R_3$ et $R_4$ représentent indépendamment l'un de l'autre un groupement choisi parmi :

- alkyle en $C_1$ à $C_{10}$, linéaire ou ramifié,
- cycloalkyle en $C_3$ à $C_{10}$, et
- aryle en $C_6$ à $C_{20}$ ou hétéroaryle en $C_3$ à $C_{20}$,
- alkyl-aryle en $C_7$ à $C_{20}$ ou alkyle-hétéroaryle en $C_4$ a $C_{20}$,

soit $L_1$ et $L_2$ représentent indépendamment l'un de l'autre :

- un atome d'halogène choisi parmi Cl, Br et I, ou
- un ligand monodentate,

soit $L_1$ et $L_2$ sont liés et représentent un ligand bidentate,
ledit composé de Formule VI pouvant être lié à un support, notamment un polymère ou de la silice, par au moins un des groupements $R_1$, $R_2$, $R_3$ ou $R_4$,
éventuellement l'un des groupements $R_1$ ou $R_2$ peut être relié à $R_3$ ou $R_4$ et représente ensemble un groupement bidentate comprenant deux groupements carbène N-hétérocyclique.

**Procédé**

**Un deuxième objet** de la présente invention est un procédé de préparation d'un composé oxalate ou d'un composé oxamide, à partir de monoxyde de carbone (CO), d'un oxydant en particulier l'oxygène moléculaire ou l'air, d'un alcool ou d'une amine respectivement, catalysé par un catalyseur M-NHC, dans lequel M représente l'atome palladium (Pd) ou l'atome platine (Pt) et NHC représente un ligand carbène N-hétérocyclique.
Avantageusement, sous des conditions particulières, la présence d'un promoteur, d'une base, d'un solvant ou d'une étape de chauffage est optionnelle.
Le bilan de réaction de la carbonylation oxydative d'un alcool ou d'une amine pour le procédé de préparation des oxalates ou des oxamides selon l'invention s'écrit comme suit :

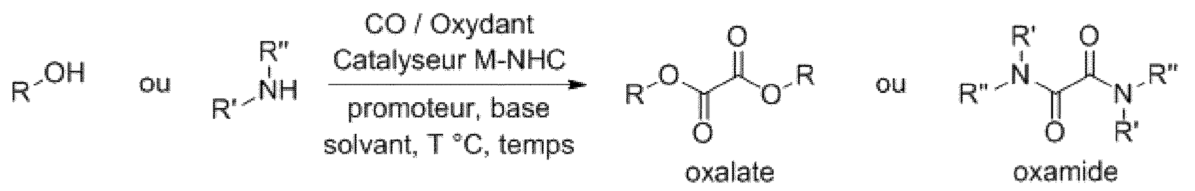


Selon un mode de réalisation particulier, l'invention concerne un procédé de préparation d'un composé oxalate ou d'un composé oxamide comprenant :

- une étape A de mise en contact d'un alcool ou d'une amine, respectivement, avec :
  - du monoxyde de carbone,
  - un oxydant,
  - un catalyseur M-NHC, dans lequel M représente Pd ou Pt et NHC représente un groupement carbène N-hétérocyclique, comprenant au moins un atome M lié à au moins un ligand carbène N-hétérocyclique, et
  - éventuellement un promoteur
  - éventuellement une base,
  - éventuellement un solvant,

  pour obtenir un milieu réactionnel,
- éventuellement une étape B de chauffage dudit milieu réactionnel,

pour obtenir le composé oxatate ou le composé oxamide.
Selon un mode de réalisation particulier, l'invention concerne un procédé tel que défini ci-dessus de préparation d'un composé oxalate comprenant :

- une étape A de mise en contact d'un alcool, respectivement, avec :
  - du monoxyde de carbone,
  - un oxydant,
  - un catalyseur M-NHC, et
  - éventuellement un promoteur,
  - éventuellement une base,
  - éventuellement un solvant,

pour obtenir un milieu réactionnel,
- éventuellement une étape B de chauffage dudit milieu réactionnel,

pour obtenir le composé oxatate.

Selon un mode de réalisation particulier, l'invention concerne un procédé tel que défini ci-dessus de préparation d'un composé oxamide comprenant :

- une étape A de mise en contact d'une amine, respectivement, avec :
  - du monoxyde de carbone,
  - un oxydant,
  - un catalyseur M-NHC, et
  - éventuellement un promoteur,
  - éventuellement une base,
  - éventuellement un solvant,

  pour obtenir un milieu réactionnel,
- éventuellement une étape B de chauffage dudit milieu réactionnel,

pour obtenir le composé oxamide.

Avantageusement, l'invention concerne un procédé de préparation d'un composé oxalate ou d'un composé oxamide, mettant en œuvre un catalyseur au palladium Pd-NHC.

Selon un mode de réalisation particulier, l'invention concerne un procédé tel que défini ci-dessus de préparation d'un composé oxalate ou d'un composé oxamide comprenant :

- une étape A de mise en contact d'un alcool ou d'une amine, respectivement, avec :
  - du monoxyde de carbone,
  - un oxydant,
  - un catalyseur Pd-NHC, et
  - éventuellement un promoteur
  - éventuellement une base,
  - éventuellement un solvant,

  pour obtenir un milieu réactionnel,
- éventuellement une étape B de chauffage dudit milieu réactionnel,

pour obtenir le composé oxatate ou le composé oxamide.

Selon un mode de réalisation particulier, l'invention concerne un procédé tel que défini ci-dessus de préparation d'un composé oxalate comprenant :

- une étape A de mise en contact d'un alcool, respectivement, avec :
  - du monoxyde de carbone,
  - un oxydant,
  - un catalyseur Pd-NHC, et
  - éventuellement un promoteur,
  - éventuellement une base,
  - éventuellement un solvant,

  pour obtenir un milieu réactionnel,
- éventuellement une étape B de chauffage dudit milieu réactionnel,

pour obtenir le composé oxatate.

Selon un mode de réalisation particulier, l'invention concerne un procédé tel que défini ci-dessus de préparation d'un composé oxamide comprenant :

- une étape A de mise en contact d'une amine, respectivement, avec :

- du monoxyde de carbone,
- un oxydant,
- un catalyseur Pd-NHC, et
- éventuellement un promoteur,
- éventuellement une base,
- éventuellement un solvant,

pour obtenir un milieu réactionnel,

- éventuellement une étape B de chauffage dudit milieu réactionnel,

pour obtenir le composé oxamide.

Avantageusement, l'invention concerne un procédé de préparation d'un composé oxalate ou d'un composé oxamide, mettant en œuvre un catalyseur au platine Pt-NHC.

Selon un mode de réalisation particulier, l'invention concerne un procédé tel que défini ci-dessus de préparation d'un composé oxalate ou d'un composé oxamide comprenant :

- une étape A de mise en contact d'un alcool ou d'une amine, respectivement, avec :
  - du monoxyde de carbone,
  - un oxydant,
  - un catalyseur Pt-NHC, et
  - éventuellement un promoteur
  - éventuellement une base,
  - éventuellement un solvant,

  pour obtenir un milieu réactionnel,
- éventuellement une étape B de chauffage dudit milieu réactionnel,

pour obtenir le composé oxatate ou le composé oxamide.

Selon un mode de réalisation particulier, l'invention concerne un procédé tel que défini ci-dessus de préparation d'un composé oxalate comprenant :

- une étape A de mise en contact d'un alcool, respectivement, avec :
  - du monoxyde de carbone,
  - un oxydant,
  - un catalyseur Pt-NHC, et
  - éventuellement un promoteur,
  - éventuellement une base,
  - éventuellement un solvant,

  pour obtenir un milieu réactionnel,
- éventuellement une étape B de chauffage dudit milieu réactionnel,

pour obtenir le composé oxatate.

Selon un mode de réalisation particulier, l'invention concerne un procédé tel que défini ci-dessus de préparation d'un composé oxamide comprenant :

- une étape A de mise en contact d'une amine, respectivement, avec :
  - du monoxyde de carbone,
  - un oxydant,
  - un catalyseur Pt-NHC, et
  - éventuellement un promoteur,
  - éventuellement une base,
  - éventuellement un solvant,

  pour obtenir un milieu réactionnel,

- éventuellement une étape B de chauffage dudit milieu réactionnel,

pour obtenir le composé oxamide.

Selon un mode de réalisation particulier l'invention concerne un procédé tel que défini ci-dessus, d'un composé oxalate ou d'un composé oxamide, dans lequel l'oxydant est choisi parmi : l'oxygène moléculaire ($O_2$), l'air, une dione en particulier la 1,4-benzoquinone, la 1,4-dichloro-2-butène et $CuCl_2$.

Avantageusement l'oxydant est l'oxygène moléculaire ou l'air.

Selon un mode de réalisation particulier, l'invention concerne un procédé tel que défini ci-dessus de préparation d'un composé oxalate ou d'un composé oxamide comprenant :

- une étape A de mise en contact d'un alcool ou d'une amine, respectivement, avec :
    - du monoxyde de carbone,
    - de l'oxygène ou de l'air,
    - un catalyseur M-NHC, et
    - un promoteur
    - éventuellement une base,
    - éventuellement un solvant,

  pour obtenir un milieu réactionnel,
- éventuellement une étape B de chauffage dudit milieu réactionnel,

pour obtenir le composé oxatate ou le composé oxamide.

Selon un mode de réalisation particulier, l'invention concerne un procédé tel que défini ci-dessus de préparation d'un composé oxalate comprenant :

- une étape A de mise en contact d'un alcool, respectivement, avec :
    - du monoxyde de carbone,
    - de l'oxygène ou de l'air,
    - un catalyseur M-NHC, et
    - un promoteur,
    - éventuellement une base,
    - éventuellement un solvant,

  pour obtenir un milieu réactionnel,
- éventuellement une étape B de chauffage dudit milieu réactionnel,

pour obtenir le composé oxatate.

Selon un mode de réalisation particulier, l'invention concerne un procédé tel que défini ci-dessus de préparation d'un composé oxamide comprenant :

- une étape A de mise en contact d'une amine, respectivement, avec :
    - du monoxyde de carbone,
    - de l'oxygène ou de l'air,
    - un catalyseur M-NHC, et
    - un promoteur,
    - éventuellement une base,
    - éventuellement un solvant,

  pour obtenir un milieu réactionnel,
- éventuellement une étape B de chauffage dudit milieu réactionnel,

pour obtenir le composé oxamide.

Avantageusement, l'invention concerne un procédé de préparation d'un composé oxalate ou d'un composé oxamide, mettant en œuvre un catalyseur au palladium Pd-NHC, en présence d'oxygène ou d'air comme oxydant.

Selon un mode de réalisation particulier, l'invention concerne un procédé tel que défini ci-dessus de préparation

d’un composé oxalate ou d’un composé oxamide comprenant :

- une étape A de mise en contact d’un alcool ou d’une amine, respectivement, avec :
  - du monoxyde de carbone,
  - de l’oxygène ou de l’air,
  - un catalyseur Pd-NHC, et
  - un promoteur
  - éventuellement une base,
  - éventuellement un solvant,

  pour obtenir un milieu réactionnel,
- éventuellement une étape B de chauffage dudit milieu réactionnel,

pour obtenir le composé oxatate ou le composé oxamide.

Selon un mode de réalisation particulier, l’invention concerne un procédé tel que défini ci-dessus de préparation d’un composé oxalate comprenant :

- une étape A de mise en contact d’un alcool, respectivement, avec :
  - du monoxyde de carbone,
  - de l’oxygène ou de l’air,
  - un catalyseur Pd-NHC, et
  - un promoteur,
  - éventuellement une base,
  - éventuellement un solvant,

  pour obtenir un milieu réactionnel,
- éventuellement une étape B de chauffage dudit milieu réactionnel,

pour obtenir le composé oxatate.

Selon un mode de réalisation particulier, l’invention concerne un procédé tel que défini ci-dessus de préparation d’un composé oxamide comprenant :

- une étape A de mise en contact d’une amine, respectivement, avec :
  - du monoxyde de carbone,
  - de l’oxygène ou de l’air,
  - un catalyseur Pd-NHC, et
  - un promoteur,
  - éventuellement une base,
  - éventuellement un solvant,

  pour obtenir un milieu réactionnel,
- éventuellement une étape B de chauffage dudit milieu réactionnel,

pour obtenir le composé oxamide.

Avantageusement, l’invention concerne un procédé de préparation d’un composé oxalate ou d’un composé oxamide, mettant en œuvre un catalyseur au platine Pt-NHC, en présence d’oxygène ou d’air comme oxydant.

Selon un mode de réalisation particulier, l’invention concerne un procédé tel que défini ci-dessus de préparation d’un composé oxalate ou d’un composé oxamide comprenant :

- une étape A de mise en contact d’un alcool ou d’une amine, respectivement, avec :
  - du monoxyde de carbone,
  - de l’oxygène ou de l’air,
  - un catalyseur Pt-NHC, et
  - un promoteur

- éventuellement une base,
- éventuellement un solvant,

pour obtenir un milieu réactionnel,
- éventuellement une étape B de chauffage dudit milieu réactionnel,

pour obtenir le composé oxatate ou le composé oxamide.

Selon un mode de réalisation particulier, l'invention concerne un procédé tel que défini ci-dessus de préparation d'un composé oxalate comprenant :

- une étape A de mise en contact d'un alcool, respectivement, avec :
  - du monoxyde de carbone,
  - de l'oxygène ou de l'air,
  - un catalyseur Pt-NHC, et
  - un promoteur,
  - éventuellement une base,
  - éventuellement un solvant,

  pour obtenir un milieu réactionnel,
- éventuellement une étape B de chauffage dudit milieu réactionnel,

pour obtenir le composé oxatate.

Selon un mode de réalisation particulier, l'invention concerne un procédé tel que défini ci-dessus de préparation d'un composé oxamide comprenant :

- une étape A de mise en contact d'une amine, respectivement, avec :
  - du monoxyde de carbone,
  - de l'oxygène ou de l'air,
  - un catalyseur Pt-NHC, et
  - un promoteur,
  - éventuellement une base,
  - éventuellement un solvant,

  pour obtenir un milieu réactionnel,
- éventuellement une étape B de chauffage dudit milieu réactionnel,

pour obtenir le composé oxamide.

On entend par « promoteur » une substance pouvant améliorer les propriétés d'un catalyseur telles que l'activité catalytique, la sélectivité, l'anti-toxicité, la stabilité, la durée de vie ou prévenir la désactivation du catalyseur.

Selon un mode de réalisation particulier, le promoteur est choisi parmi les dérivés iodés et les sels d'ammonium. Le groupe des dérivés iodés comprend $I_2$, KI, LiI, HI, NaI et l'iodure de tétrabutylammonium ($Bu_4NI$). Les dérivés iodés présentent l'avantage d'être soluble dans le milieu réactionnel et peuvent être ainsi avantageusement utilisés dans le procédé de préparation sans engendrer la formation excessive de produits solides. De préférence l'iodure de tetrabutylammonium ($Bu_4NI$) est utilisé comme promoteur, ce dernier présentant étant soluble à 25°C et ne précipitant pas durant le procédé de préparation.

Dans les réactions catalysées par Pd(II), les promoteurs sont connus pour permettre de réoxyder in-situ le Pd(0) généré en Pd(II) et ainsi assurer le cycle catalytique. Le promoteur peut aussi protéger le catalyseur au Palladium de l'eau présent dans le système et ainsi prévenir la désactivation du catalyseur, éviter l'addition d'agent déshydratant dans le système.

Selon un mode de réalisation particulier, le procédé selon l'invention est réalisé dans des conditions de carbonylation oxydative en présence d'oxygène et le promoteur agit en tant qu'oxydant supplémentaire. Le promoteur peut ainsi favoriser le procédé de carbonylation oxydative en permettant l'oxydation du M(0) en M(II) du catalyseur M-NHC lors de la réaction. En effet il est assez difficile de réoxyder directement le Pd(0) en Pd(II) par l'oxygène moléculaire, ainsi un oxydant supplémentaire est généralement utilisé.

On entend par « milieu réactionnel », l'ensemble des espèces mises en association lors d'une réaction chimique. Il comprend notamment les réactifs sous forme liquide ou gazeux, le catalyseur, et éventuellement un solvant, des additifs

ou des promoteurs.

Procédé de préparation des oxalates en présence d'un catalyseur Pd-NHC.

*Etape chauffage*

Selon un mode de réalisation particulier, l'invention concerne un procédé tel que défini ci-dessus de préparation d'un composé oxalate comprenant :

- une étape A de mise en contact d'un alcool avec :
  - du monoxyde de carbone,
  - de l'oxygène ou de l'air,
  - un promoteur,
  - un catalyseur Pd-NHC, et
  - éventuellement une base,
  - éventuellement un solvant,

  pour obtenir un milieu réactionnel,
- une étape B de chauffage dudit milieu réactionnel,

pour obtenir le composé oxalate.

Selon un mode de réalisation particulier, l'invention concerne un procédé de préparation tel que défini ci-dessus, d'un composé oxalate, dans lequel l'étape B de chauffage est effectuée à une température comprise de 25 à 200°C, notamment de 60 à 110°C, de préférence d'environ 90 °C.

L'expression « de 25 à 200°C » correspond aux gammes : de 25 à 40°C ; de 40 à 60°C ; de 60 à 80°C ; de 80 à 100°C ; de 100 à 120°C ; de 120 à 140°C ; de 140 à 160°C ; de 160 à 180°C ; de 180 à 200°C.

L'expression « de 60 à 110°C » correspond aux gammes : de 60 à 70°C ; de 70 à 80°C ; de 80 à 90°C ; de 90 à 100°C ; de 100 à 110°C.

Selon un mode de réalisation particulier, l'invention concerne un procédé de préparation selon l'invention défini ci-dessus d'un composé oxalate comprenant :

- une étape A de mise en contact d'un alcool avec :
  - du monoxyde de carbone,
  - de l'oxygène ou de l'air,
  - un promoteur,
  - un catalyseur Pd-NHC, et
  - éventuellement une base,
  - éventuellement un solvant,

  pour obtenir un milieu réactionnel,
- une étape B de chauffage dudit milieu réactionnel, pour obtenir le composé oxalate, en particulier effectuée à une température comprise de 25 à 200 °C, notamment de 60 à 110°C, de préférence d'environ 90 °C.

<u>Base *dans le milieu réactionnel*</u>

Le procédé selon l'invention de préparation des oxalates peut être réalisé avec ou sans base dans le milieu réactionnel.

*Présence d'une base*

Selon un mode de réalisation particulier, l'invention concerne un procédé tel que défini ci-dessus de préparation d'un composé oxalate comprenant :

- une étape A de mise en contact d'un alcool avec :
  - du monoxyde de carbone,

- de l'oxygène ou de l'air,
- une base,
- un promoteur,
- un catalyseur Pd-NHC, et
- éventuellement un solvant,

pour obtenir un milieu réactionnel,

- une étape B de chauffage dudit milieu réactionnel, pour obtenir le composé oxalate.

Selon un mode de réalisation particulier, l'invention concerne un procédé tel que défini ci-dessus de préparation d'un composé oxalate, dans lequel ledit milieu réactionnel comprend une base.

La présence d'une base, choisie avantageusement dans le milieu réactionnel permet une augmentation du rendement de la réaction.

Selon un mode de réalisation particulier, l'invention concerne un procédé de préparation tel que défini ci-dessus, d'un composé oxalate, dans lequel la base est choisie parmi le carbonate de potassium ($K_2CO_3$), le carbonate de sodium ($Na_2CO_3$), le tert-butylate de potassium (KOtBu), le phosphate de potassium ($K_3PO_4$) et la triéthylamine ($Et_3N$).

Selon un mode de réalisation particulier, l'invention concerne un procédé de préparation tel que défini ci-dessus, d'un composé oxalate, dans lequel la base est de la triéthylamine.

Avantageusement l'utilisation de la triéthylamine ($Et_3N$) permet aisément son évaporation lors de l'isolation du produit et sa présence n'induit pas de problèmes de recyclage du catalyseur comme les bases solides $K_2CO_3$ et $Na_2CO_3$ qui précipite à température ambiante alors que la triéthylamine reste en phase liquide.

*Absence d'une base ajoutée*

Avantageusement le procédé selon l'invention de préparation d'un composé oxalate peut être mis en œuvre sans l'utilisation d'une base.

Selon un mode de réalisation particulier, l'invention concerne un procédé tel que défini ci-dessus de préparation d'un composé oxalate comprenant :

- une étape A de mise en contact d'un alcool avec :
  - du monoxyde de carbone,
  - de l'oxygène ou de l'air,
  - un promoteur,
  - un catalyseur Pd-NHC, et
  - éventuellement un solvant,

  pour obtenir un milieu réactionnel,
- une étape B de chauffage dudit milieu réactionnel, pour obtenir le composé oxalate.

L'absence de base dans le milieu réactionnel permet de limiter les réactifs à introduire dans le procédé et de limiter la formation de produits de dégradation.

*Solvant dans le milieu réactionnel*

Le procédé selon l'invention de préparation des oxalates peut être réalisé avec ou sans solvant dans le milieu réactionnel.

*Présence d'un solvant*

Selon un mode de réalisation particulier, l'invention concerne un procédé tel que défini ci-dessus de préparation d'un composé oxalate, dans lequel ledit milieu réactionnel comprend un solvant.

Selon un mode de réalisation particulier, l'invention concerne un procédé tel que défini ci-dessus de préparation d'un composé oxalate comprenant :

- une étape A de mise en contact d'un alcool avec :
  - du monoxyde de carbone,

    - de l'oxygène ou de l'air,
    - éventuellement une base,
    - un promoteur,
    - un catalyseur Pd-NHC, et
    - un solvant,

  pour obtenir un milieu réactionnel,
- une étape B de chauffage dudit milieu réactionnel, pour obtenir le composé oxalate.

Selon un mode de réalisation particulier, l'invention concerne un procédé tel que défini ci-dessus de préparation d'un composé oxalate comprenant :

- une étape A de mise en contact d'un alcool avec :

    - du monoxyde de carbone,
    - de l'oxygène ou de l'air,
    - une base,
    - un promoteur,
    - un catalyseur Pd-NHC, et
    - un solvant,

  pour obtenir un milieu réactionnel,
- une étape B de chauffage dudit milieu réactionnel, pour obtenir le composé oxalate.

Selon un mode de réalisation particulier, l'invention concerne un procédé tel que défini ci-dessus de préparation d'un composé oxalate comprenant :

- une étape A de mise en contact d'un alcool avec :

    - du monoxyde de carbone,
    - de l'oxygène ou de l'air,
    - un promoteur,
    - un catalyseur Pd-NHC, et
    - un solvant,

  pour obtenir un milieu réactionnel,
- une étape B de chauffage dudit milieu réactionnel, pour obtenir le composé oxalate.

*Sans solvant ajouté dans le milieu réactionnel*

Selon un mode de réalisation particulier, l'invention concerne un procédé de préparation tel que défini ci-dessus, d'un composé oxalate, dans lequel le procédé est mis en œuvre en absence de solvant, l'alcool à partir desquelles l'oxalate est préparé jouant le rôle de solvant. Le procédé de préparation d'un composé oxalate selon l'invention peut être mise en œuvre sans l'utilisation de solvant. Ceci permet de limiter les étapes de préparation et la présence de produits de dégradations à traiter et de limiter les étapes de traitement du solvant. Par exemple, l'utilisation de l'alcool comme réactif et solvant peut permettre de simplifier le recyclage de l'alcool, en cas de conversion incomplète de celui-ci. Ainsi il n'y a pas besoin de séparer les solvants tel que l'acétonitrile de l'alcool.

Selon un mode de réalisation particulier, l'invention concerne un procédé tel que défini ci-dessus de préparation d'un composé oxalate comprenant :

- une étape A de mise en contact d'un alcool avec :

    - du monoxyde de carbone,
    - de l'oxygène ou de l'air,
    - éventuellement une base,
    - un promoteur, et
    - un catalyseur Pd-NHC,

pour obtenir un milieu réactionnel,
- une étape B de chauffage dudit milieu réactionnel, pour obtenir le composé oxalate.

Selon un mode de réalisation particulier, l'invention concerne un procédé tel que défini ci-dessus de préparation d'un composé oxalate comprenant :

- une étape A de mise en contact d'un alcool avec :
  - du monoxyde de carbone,
  - de l'oxygène ou de l'air,
  - une base,
  - un promoteur, et
  - un catalyseur Pd-NHC,

  pour obtenir un milieu réactionnel,
- une étape B de chauffage dudit milieu réactionnel, pour obtenir le composé oxalate.

Selon un mode de réalisation particulier, l'invention concerne un procédé tel que défini ci-dessus de préparation d'un composé oxalate comprenant :

- une étape A de mise en contact d'un alcool avec :
  - du monoxyde de carbone,
  - de l'oxygène ou de l'air,
  - un promoteur, et
  - un catalyseur Pd-NHC,

  pour obtenir un milieu réactionnel,
- une étape B de chauffage dudit milieu réactionnel, pour obtenir le composé oxalate.

*Présence d'eau*

L'eau formée in-situ ou présente dans les réactifs peut être néfaste et notamment désactiver les catalyseurs au palladium dans la carbonylation oxydative lors d'un procédé de préparation d'oxalates.
Au contraire des autres catalyseurs de l'art antérieur tels que décrits dans US3 393 136, US4 005 130, US 4005 129 et US 4005 128, où la présence d'eau est préjudiciable lors du procédé de préparation des oxalates, l'utilisation des catalyseurs palladium-(carbène N-hétérocyclique) dans des conditions selon l'invention permet de s'affranchir de l'utilisation d'agent déshydratant ou d'une étape de déshydratation des réactifs dans la préparation d'un composé oxalate.
Avantageusement, le procédé selon l'invention mettant en œuvre le catalyseur palladium-(carbène N-hétérocyclique) en présence d'oxygène ou d'air et d'un promoteur permet une préparation sélective des oxalates en présence d'eau dans le milieu réactionnel.
Selon un mode de réalisation particulier, l'invention concerne l'utilisation d'un catalyseur palladium-(carbène N-hétérocyclique) dans la mise en œuvre d'un procédé de préparation sélective d'oxalates à partir de monoxyde de carbone, d'oxygène moléculaire ou d'air, d'un promoteur et d'un alcool, ne requérant pas de conditions de préparation anhydres.

*L'alcool utilisé comme substrat*

Selon un mode de réalisation particulier, l'invention concerne un procédé de préparation tel que défini ci-dessus, d'un composé oxalate de Formule 2, dans lequel l'étape A comprend la mise en contact d'un alcool de Formule 1:

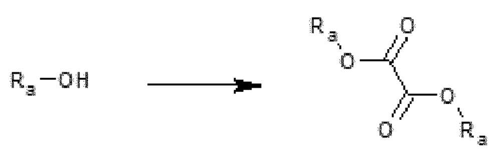

Formule 1 Formule 2

dans lesquelles $R_a$ représente un groupement choisi parmi :

- alkyle en $C_1$ à $C_{10}$, linéaire ou ramifié, et
- cycloalkyle en $C_3$ à $C_{10}$.

Selon un mode de réalisation particulier, l'invention concerne un procédé de préparation d'un composé oxalate tel que défini ci-dessus, dans lequel l'étape A comprend la mise en contact d'un alcool choisi parmi le méthanol, l'éthanol et l'isopropanol.

Les oxalates préparés avec le méthanol, l'éthanol et l'isopropanol sont respectivement le diméthyloxalate, le diéthyloxalate et le diisopropyloxalate, représentés ci-dessous :

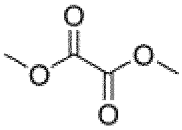

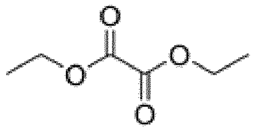

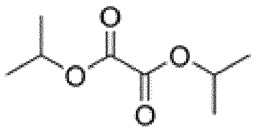

Selon un mode de réalisation particulier, l'invention concerne un procédé de préparation selon l'invention tel que défini ci-dessus, d'un composé oxalate de Formule 2, dans lequel l'étape A comprend la mise en contact d'un alcool de Formule 1 :

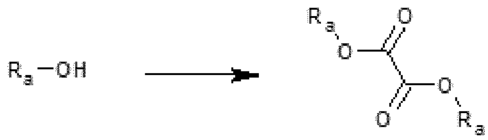

Formule 1 Formule 2

dans lesquelles $R_a$ représente un groupement choisi parmi :

- alkyle en $C_1$ à $C_{10}$, linéaire ou ramifié, et
- cycloalkyle en $C_3$ à $C_{10}$,

en particulier l'alcool est choisi parmi le méthanol, l'éthanol et l'isopropanol.

**Procédé de préparation des oxamides en présence d'un catalyseur Pd-NHC.**

Selon un mode de réalisation particulier, l'invention concerne un procédé tel que défini ci-dessus de préparation d'un composé oxamide comprenant :

- une étape A de mise en contact d'une amine avec :
  - du monoxyde de carbone,
  - de l'oxygène ou de l'air,
  - éventuellement une base,
  - éventuellement un promoteur,
  - un catalyseur Pd-NHC, et
  - un solvant,

  pour obtenir un milieu réactionnel,
- éventuellement une étape B de chauffage dudit milieu réactionnel, pour obtenir le composé oxamide.

Selon un mode de réalisation particulier, l'invention concerne un procédé tel que défini ci-dessus de préparation d'un composé oxamide comprenant :

- une étape A de mise en contact d'une amine avec :
  - du monoxyde de carbone,
  - de l'oxygène ou de l'air,
  - éventuellement une base,
  - un promoteur,

- un catalyseur Pd-NHC, et
- un solvant,

pour obtenir un milieu réactionnel,
- éventuellement une étape B de chauffage dudit milieu réactionnel,

pour obtenir le composé oxamide.

Selon un mode de réalisation particulier, l'invention concerne un procédé tel que défini ci-dessus de préparation d'un composé oxamide comprenant :

- une étape A de mise en contact d'une amine avec :
  - du monoxyde de carbone,
  - de l'oxygène ou de l'air,
  - éventuellement une base,
  - un catalyseur Pd-NHC, et
  - un solvant,

  pour obtenir un milieu réactionnel,
- éventuellement une étape B de chauffage dudit milieu réactionnel,

pour obtenir le composé oxamide.

*Base dans le milieu réactionnel*

Le procédé selon l'invention de préparation des oxamides peut être réalisé avec ou sans base dans le milieu réactionnel.

*Sans base ajouté dans le milieu réactionnel*

Selon un mode de réalisation particulier, l'invention concerne un procédé tel que défini ci-dessus de préparation d'un composé oxamide, dans lequel la base est l'amine à partir de laquelle ledit composé oxamide est préparé.

Selon un mode de réalisation particulier, l'invention concerne un procédé tel que défini ci-dessus de préparation d'un composé oxamide comprenant :

- une étape A de mise en contact d'une amine avec :
  - du monoxyde de carbone,
  - de l'oxygène ou de l'air,
  - un promoteur,
  - un catalyseur Pd-NHC, et
  - un solvant,

  pour obtenir un milieu réactionnel,
- éventuellement une étape B de chauffage dudit milieu réactionnel,

pour obtenir le composé oxamide.

*Présence d'une base ajoutée dans le milieu réactionnel*

Selon un mode de réalisation particulier, l'invention concerne un procédé tel que défini ci-dessus de préparation d'un composé oxamide comprenant :

- une étape A de mise en contact d'une amine avec :
  - du monoxyde de carbone,
  - de l'oxygène ou de l'air,
  - une base,

- un promoteur,
- un catalyseur Pd-NHC, et
- un solvant,

pour obtenir un milieu réactionnel,

- éventuellement une étape B de chauffage dudit milieu réactionnel,

pour obtenir le composé oxamide.

Selon un mode de réalisation particulier, l'invention concerne un procédé de préparation tel que défini ci-dessus, d'un composé oxamide, dans lequel la base est choisie parmi le carbonate de potassium ($K_2CO_3$), le carbonate de sodium ($Na_2CO_3$), le tert-butylate de potassium (KOtBu), le phosphate de potassium ($K_3PO_4$) et la triéthylamine ($Et_3N$).

Selon un mode de réalisation particulier, l'invention concerne un procédé tel que défini ci-dessus de préparation d'un composé oxamide, dans lequel ladite base est une base inorganique, en particulier $K_2CO_3$.

Selon un mode de réalisation particulier, l'invention concerne un procédé tel que défini ci-dessus de préparation d'un composé oxamide, dans lequel ladite base est une base organique, en particulier la triéthylamine.

Le procédé de préparation d'un composé oxamide de l'invention peut être réalisé aussi bien avec l'ajout d'une base inorganique qu'avec l'ajout d'une base organique. Il a été constaté que la base inorganique $K_2CO_3$ est aussi efficace que la base organique, la triéthylamine.

*Procédé avec une étape de chauffage*

Selon un mode de réalisation particulier, l'invention concerne un procédé tel que défini ci-dessus de préparation d'un composé oxamide comprenant :

- une étape A de mise en contact d'une amine avec :
  - du monoxyde de carbone,
  - de l'oxygène ou de l'air,
  - éventuellement une base,
  - un promoteur,
  - un catalyseur Pd-NHC, et
  - un solvant,

  pour obtenir un milieu réactionnel,
- une étape B de chauffage dudit milieu réactionnel,

pour obtenir le composé oxamide.

Selon un mode de réalisation particulier, l'invention concerne un procédé tel que défini ci-dessus de préparation d'un composé oxamide comprenant :

- une étape A de mise en contact d'une amine avec :
  - du monoxyde de carbone,
  - de l'oxygène ou de l'air,
  - une base,
  - un promoteur,
  - un catalyseur Pd-NHC, et
  - un solvant,

  pour obtenir un milieu réactionnel,
- une étape B de chauffage dudit milieu réactionnel,

pour obtenir le composé oxamide.

Selon un mode de réalisation particulier, l'invention concerne un procédé tel que défini ci-dessus de préparation d'un composé oxamide comprenant :

- une étape A de mise en contact d'une amine avec :

- du monoxyde de carbone,
- de l'oxygène ou de l'air,
- un promoteur,
- un catalyseur Pd-NHC, et
- un solvant,

pour obtenir un milieu réactionnel,

- une étape B de chauffage dudit milieu réactionnel,

pour obtenir le composé oxamide.

Selon un mode de réalisation, le procédé tel que défini ci-dessus de préparation d'un composé oxamide, comprend une étape B de chauffage à une température de 25 à 110°C.

*Procédé sans étape de chauffage*

Selon un mode de réalisation particulier, l'invention concerne un procédé tel que défini ci-dessus de préparation d'un composé oxamide, dans lequel ledit est milieu réactionnel est maintenu à une température ambiante de 20 à 25°C.

Selon un mode de réalisation particulier, l'invention concerne un procédé tel que défini ci-dessus de préparation d'un composé oxamide comprenant :

- une étape A de mise en contact d'une amine avec :
  - du monoxyde de carbone,
  - de l'oxygène ou de l'air,
  - éventuellement une base,
  - un promoteur,
  - un catalyseur Pd-NHC, et
  - un solvant,

  pour obtenir un milieu réactionnel comprenant le composé oxamide.

Selon un mode de réalisation particulier, l'invention concerne un procédé tel que défini ci-dessus de préparation d'un composé oxamide comprenant :

- une étape A de mise en contact d'une amine avec :
  - du monoxyde de carbone,
  - de l'oxygène ou de l'air,
  - une base,
  - un promoteur,
  - un catalyseur Pd-NHC, et
  - un solvant,

pour obtenir un milieu réactionnel comprenant le composé oxamide.

Selon un mode de réalisation particulier, l'invention concerne un procédé tel que défini ci-dessus de préparation d'un composé oxamide comprenant :

- une étape A de mise en contact d'une amine avec :
  - du monoxyde de carbone,
  - de l'oxygène ou de l'air,
  - un promoteur,
  - un catalyseur Pd-NHC, et
  - un solvant,

pour obtenir un milieu réactionnel comprenant le composé oxamide.

Le procédé de préparation des oxamides selon l'invention par carbonylation oxidative peut avantageusement être mis en œuvre à température ambiante de 20 à 25°C.

En effet il a été observé que le procédé de préparation des oxamides selon l'invention en présence d'une base et de solvant permettait d'obtenir les oxamides dans le milieu réactionnel sans étape de chauffage.

En conséquence une étape de chauffage du milieu réactionnel est facultative, ce qui représente un avantage industriel en termes de coût et de sécurité.

Selon un mode de réalisation particulier, l'invention concerne un procédé tel que défini ci-dessus de préparation d'un composé oxamide comprenant :

- une étape A de mise en contact d'une amine avec :
    - du monoxyde de carbone,
    - de l'oxygène ou de l'air,
    - un promoteur,
    - un catalyseur de type Pd-NHC, et
    - un solvant,
    - éventuellement une base, en particulier choisi parmi $K_2CO_3$ ou $Et_3N$,

pour obtenir un milieu réactionnel comprenant l'oxamide.

*L'amine utilisée comme substrat*

Selon un mode de réalisation particulier, l'invention concerne un procédé de préparation tel que défini ci-dessus, d'un composé oxamide de Formule 4 dans lequel l'étape A comprend la mise en contact d'une amine de Formule 3 :

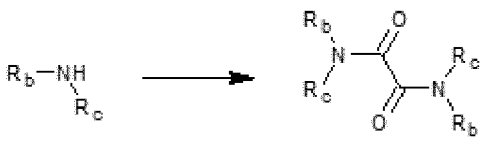

Formule 3 Formule 4

dans lesquelles $R_b$ et $R_c$ représentent indépendamment l'un de l'autre :

- un atome d'hydrogène,
- un groupement alkyle en $C_1$ à $C_{20}$, linéaire ou ramifié,
- un groupement alcényle en $C_2$ à $C_{20}$, linéaire ou ramifié
- un groupement hétéroalkyle en $C_1$ à $C_{20}$, linéaire ou ramifié, l'hétéroatome étant notamment O ou N,

au moins un des groupements $R_b$ ou $R_c$ étant différent de l'hydrogène,
$R_b$ et $R_c$ pouvant former un cycle.

Le terme « hétéroalkyle, linéaire ou ramifié » désigne un groupe alkyle, linéaire ou ramifié, tel que défini ci-dessus, comprenant des atomes autres que les atomes de carbone, en particulier N, O ou S au sein de la chaine alkyle.

Le terme « alcényle, linéaire ou ramifié » désigne un groupement alkyle, linéaire ou ramifié, tel que défini ci-dessus présentant une double liaison C=C.

Selon un mode de réalisation particulier, l'invention concerne un procédé de préparation tel que défini ci-dessus, d'un composé oxamide, dans lequel l'étape A comprend la mise en contact d'une amine choisie parmi : la diéthylamine, la pipéridine, la pyrrolidine et la morpholine.

Les oxamides préparés avec la diéthylamine, la pipéridine, la pyrrolidine et la morpholine sont respectivement représentées ci-dessous:

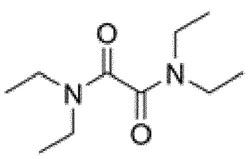 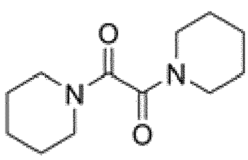 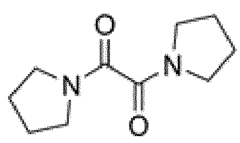 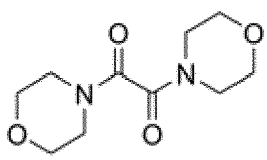

Selon un mode de réalisation particulier, l'invention concerne un procédé de préparation tel que défini ci-dessus, d'un composé oxamide de Formule 4, dans laquelle les groupements $R_b$ et $R_c$ sont liés et forment un cycle.

Selon un mode de réalisation particulier, l'invention concerne un procédé de préparation tel que défini ci-dessus, d'un composé oxamide de Formule 4, dans lequel l'étape A comprend la mise en contact d'une amine de Formule 3 :

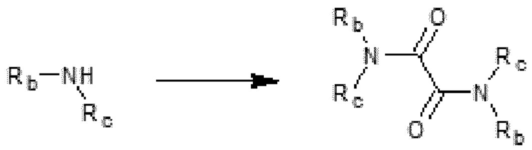

Formule 3 Formule 4

dans lesquelles $R_b$ et $R_c$ représentent indépendamment l'un de l'autre :

- un atome d'hydrogène,
- un groupement alkyle en $C_1$ à $C_{20}$, linéaire ou ramifié,
- un groupement alcényle en $C_2$ à $C_{20}$, linéaire ou ramifié
- un groupement hétéroalkyle en $C_1$ à $C_{20}$, linéaire ou ramifié, l'hétéroatome étant notamment O ou N,

au moins un des groupements $R_b$ ou $R_c$ étant différent de l'hydrogène,
$R_b$ et $R_c$ pouvant former un cycle,
en particulier l'amine est choisi parmi : la diéthylamine, la pipéridine, la morpholine, la pyrrolidine.

**Caractéristiques communes du procédé de préparation des oxalates et des oxamides.**

Catalyseurs

Selon un mode de réalisation particulier, l'invention concerne un procédé de préparation tel que défini ci-dessus, dans lequel le catalyseur répond à la Formule II-B :

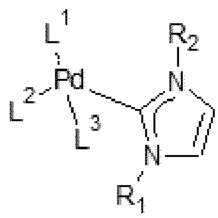

Formule II-B

dans laquelle :

$R_1$ et $R_2$ représentent indépendamment l'un de l'autre un groupement choisi parmi :

- alkyle en $C_1$ à $C_{10}$, linéaire ou ramifié,
- cycloalkyle en $C_3$ à $C_{10}$, et
- aryle en $C_6$ à $C_{20}$ ou hétéroaryle en $C_3$ à $C_{20}$,
- alkyl-aryle en $C_7$ à $C_{20}$ ou alkyle-hétéroaryle en $C_4$ à $C_{20}$,

$L^1$ représente un atome d'halogène choisi parmi Cl, Br et I, et

- soit $L^2$ et $L^3$ représentent un ligand bidentate,
- soit $L^2$ représente un atome d'halogène choisi parmi Cl, Br et I, et $L^3$ représente un ligand monodentate,

ledit composé de Formule II-B pouvant être lié à un support, notamment un polymère ou de la silice, par au moins un des groupements $R_1$ ou $R_2$.

Selon un mode de réalisation particulier, l'invention concerne le procédé de préparation tel que défini ci-dessus, dans laquelle le catalyseur répond à la Formule II-B , dans laquelle :

$L_1$ représente un atome d'iode, et

- soit $L_2$ et $L_3$ sont liés et représentent ensemble un ligand bidentate,
- soit $L_2$ représente un atome d'halogène choisi parmi Cl, Br et I et $L_3$ représente un ligand monodentate,

Selon un mode de réalisation particulier, l'invention concerne le procédé de préparation tel que défini ci-dessus, dans laquelle le catalyseur répond à la Formule II-4B :

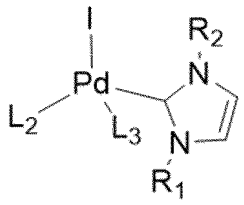

Formule II-4B

dans laquelle :

$R_1$ et $R_2$ représentent indépendamment l'un de l'autre un groupement choisi parmi :

- alkyle en $C_1$ à $C_{10}$, linéaire ou ramifié,
- cycloalkyle en $C_3$ à $C_{10}$,
- aryle en $C_6$ à $C_{20}$ ou hétéroaryle en $C_3$ à $C_{20}$,
- alkyl-aryle en $C_7$ à $C_{20}$ ou alkyle-hétéroaryle en $C_4$ à $C_{20}$,

dans laquelle

- soit $L_2$ et $L_3$ représentent un ligand bidentate, en particulier choisi parmi l'acétylacétonate (acac), l'allyle, le cinnamyle et l'acétate, de préférence l'acétylacétonate,
- soit $L_2$ représente un atome d'halogène choisi parmi Cl, Br et I et $L_3$ représente un ligand monodentate, en particulier choisi parmi : la pyridine, la 3-chloropyridine, l'acétonitrile, la triéthylamine, ou un ligand à base de phosphine, en particulier la triphénylphosphine, de préférence la 3-chloropyridine,

ledit composé de Formule II-4B pouvant être lié à un support, notamment un polymère ou de la silice, par au moins un des groupements $R_1$ ou $R_2$.

Selon un mode de réalisation particulier, l'invention concerne le procédé de préparation tel que défini ci-dessus, dans laquelle le catalyseur répond à la Formule II-B , dans laquelle :
$L_1$ et $L_2$ représentent chacun un atome d'iode, et $L_3$ représente un ligand monodentate.

Selon un mode de réalisation particulier, l'invention concerne le procédé de préparation tel que défini ci-dessus, dans laquelle le catalyseur répond à la Formule II-5B :

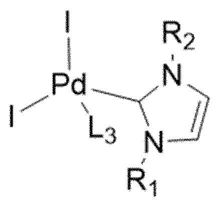

Formule II-5B

dans laquelle :

$R_1$ et $R_2$ représentent indépendamment l'un de l'autre un groupement choisi parmi :

- alkyle en $C_1$ à $C_{10}$, linéaire ou ramifié,
- cycloalkyle en $C_3$ à $C_{10}$,

- aryle en $C_6$, à $C_{20}$ ou hétéroaryle en $C_3$ à $C_{20}$,
- alkyl-aryle en $C_7$ à $C_{20}$ ou alkyle-hétéroaryle en $C_4$ à $C_{20}$,

$L_3$ représente un ligand monodentate, en particulier choisi parmi : la pyridine, la 3-chloropyridine, l'acétonitrile, la triéthylamine, ou un ligand à base de phosphine, en particulier la triphénylphosphine, de préférence la 3-chloropyridine,
ledit composé de Formule II-5B pouvant être lié à un support, notamment un polymère ou de la silice, par au moins un des groupements $R_1$ ou $R_2$.

Selon un mode de réalisation particulier, l'invention concerne un procédé de préparation tel que défini ci-dessus, dans laquelle le ligand bidentate est choisi parmi : l'acétylacétonate (acac), l'allyle, le cinnamyle et l'acétate, le ligand bidentate étant notamment l'acétylacétonate.

Selon un mode de réalisation particulier, l'invention concerne un procédé de préparation tel que défini ci-dessus, dans laquelle le ligand monodentate est choisi parmi : la pyridine, la 3-chloropyridine, l'acétonitrile, la triéthylamine, ou un ligand à base de phosphine, en particulier la triphénylphosphine. De préférence le ligand monodentate est notamment le 3-chloropyridine.

Selon un mode de réalisation particulier, l'invention concerne un procédé de préparation tel que défini ci-dessus, dans laquelle le ligand monodentate est choisi parmi : la pyridine, la 3-chloropyridine, l'acétonitrile, la triéthylamine, de préférence le ligand monodentate étant notamment le 3-chloropyridine.

Selon un mode de réalisation particulier, l'invention concerne le procédé de préparation tel que défini ci-dessus, ledit composé de Formule II-B n'étant pas lié à un support.

Selon un mode de réalisation particulier, l'invention concerne le procédé de préparation tel que défini ci-dessus, ledit composé de Formule II-B étant lié à un support, notamment un polymère ou de la silice, par au moins un des groupements $R_1$ ou $R_2$.

Selon un mode de réalisation particulier, l'invention concerne le procédé de préparation tel que défini ci-dessus, ledit composé de Formule II-4B étant lié à un support, notamment un polymère ou de la silice, par au moins un des groupements $R_1$ ou $R_2$.

Selon un mode de réalisation particulier, l'invention concerne le procédé de préparation tel que défini ci-dessus, ledit composé de Formule II-5B étant lié à un support, notamment un polymère ou de la silice, par au moins un des groupements $R_1$ ou $R_2$.

Selon un mode de réalisation particulier, l'invention concerne un procédé de préparation tel que défini ci-dessus, dans lequel le catalyseur répond à la Formule II-1B :

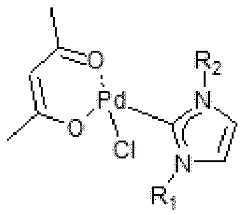

Formule II-1B

dans laquelle :

$R_1$ et $R_2$ représentent indépendamment l'un de l'autre un groupement choisi parmi :

- alkyle en $C_1$ à $C_{10}$, linéaire ou ramifié,
- cycloalkyle en $C_3$ à $C_{10}$, et
- aryle en $C_6$ à $C_{20}$ ou hétéroaryle en $C_3$ à $C_{20}$,
- alkyl-aryle en $C_7$ à $C_{20}$ ou alkyl-hétéroaryle en $C_4$ à $C_{20}$

ledit composé de Formule II-1B pouvant être lié à un support, notamment un polymère ou de la silice, par au moins un des groupements $R_1$ ou $R_2$.

Selon un mode de réalisation particulier, l'invention concerne un procédé de préparation tel que défini ci-dessus, dans lequel le catalyseur répond à la Formule II-2B :

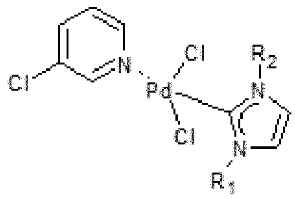

Formule II-2B

dans laquelle :

$R_1$ et $R_2$ représentent indépendamment l'un de l'autre un groupement choisi parmi :

- alkyle en $C_1$ à $C_{10}$, linéaire ou ramifié,
- cycloalkyle en $C_3$ à $C_{10}$, et
- aryle en $C_6$ à $C_{20}$ ou hétéroaryle en $C_3$ à $C_{20}$,
- alkyl-aryle en $C_7$ à $C_{20}$ ou alkyl-hétéroaryle en $C_4$ à $C_{20}$,

ledit composé de Formule II-2B pouvant être lié à un support, notamment un polymère ou de la silice, par au moins un des groupements $R_1$ ou $R_2$.

Selon un mode de réalisation particulier, l'invention concerne un procédé de préparation tel que défini ci-dessus, dans lequel le catalyseur répond à la Formule II-3B :

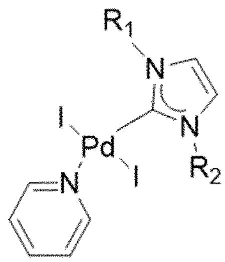

Formule II-3B

dans laquelle :

$R_1$ et $R_2$ représentent indépendamment l'un de l'autre un groupement choisi parmi :

- alkyle en $C_1$ à $C_{10}$, linéaire ou ramifié,
- cycloalkyle en $C_3$ à $C_{10}$, et
- aryle en $C_6$ à $C_{20}$ ou hétéroaryle en $C_3$ à $C_{20}$,
- alkyl-aryle en $C_7$ à $C_{20}$ ou alkyl-hétéroaryle en $C_4$ à $C_{20}$,

ledit composé de Formule II-3B pouvant être lié à un support, notamment un polymère ou de la silice, par au moins un des groupements $R_1$ ou $R_2$.

Selon un mode de réalisation particulier, l'invention concerne le procédé de préparation tel que défini ci-dessus, ledit composé de Formule II-3B étant lié à un support, notamment un polymère ou de la silice, par au moins un des groupements $R_1$ ou $R_2$.

Selon un mode de réalisation particulier, l'invention concerne un procédé de préparation tel que défini ci-dessus, dans lequel le support est du polystyrène ou un gel de silice, ledit catalyseur étant lié à audit polystyrène ou audit gel de silice.

Selon un mode de réalisation particulier, l'invention concerne un procédé de préparation tel que défini ci-dessus, dans lequel le catalyseur est choisi parmi :

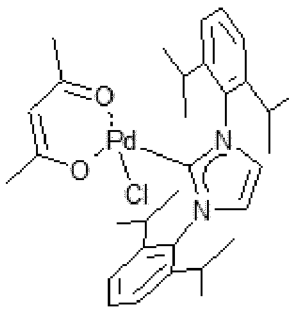

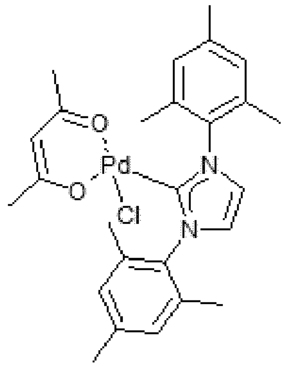

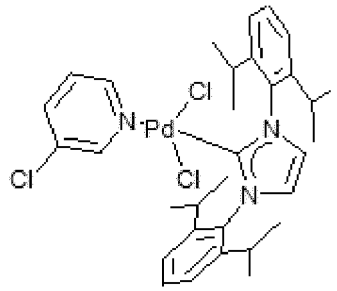

ou dans laquelle le catalyseur, lié à un support est choisi parmi :

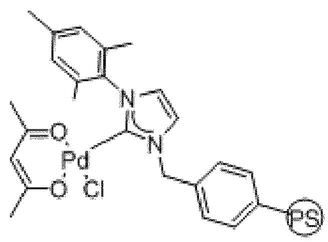

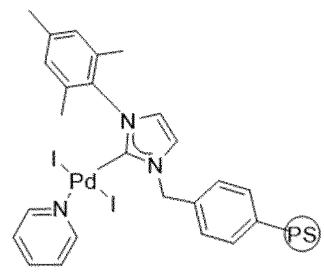

Selon un mode de réalisation particulier, l'invention concerne un procédé de préparation tel que défini ci-dessus, dans lequel le catalyseur répond à la Formule III-B :

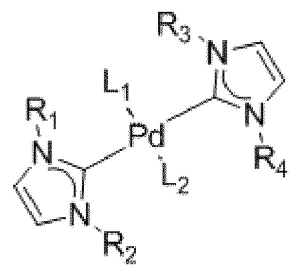

Formule III-B

dans laquelle :

$R_1$, $R_2$, $R_3$ et $R_4$ représentent indépendamment l'un de l'autre un groupement choisi parmi :

- alkyle en $C_1$ à $C_{10}$, linéaire ou ramifié,
- cycloalkyle en $C_3$ à $C_{10}$, et
- aryle en $C_6$ à $C_{20}$ ou hétéroaryle en $C_3$ à $C_{20}$,
- alkyl-aryle en $C_7$ à $C_{20}$ ou alkyle-hétéroaryle en $C_4$ a $C_{20}$,

soit $L_1$ et $L_2$ représentent indépendamment l'un de l'autre :

- un atome d'halogène choisi parmi Cl, Br et I, ou
- un ligand monodentate,

soit $L_1$ et $L_2$ sont liés et représentent un ligand bidentate,
ledit composé de Formule III-B pouvant être lié à un support, notamment un polymère, par au moins un des groupements $R_1$, $R_2$, $R_3$ ou $R_4$,
éventuellement l'un des groupements $R_1$ ou $R_2$ peut être relié à $R_3$ ou $R_4$ et représente ensemble un groupement

bidentate comprenant deux groupements carbène N-hétérocyclique.

Selon un mode de réalisation particulier, l'invention concerne le procédé de préparation tel que défini ci-dessus, ledit composé de Formule III-B étant lié à un support, notamment un polymère ou de la silice, par au moins un des groupements $R_1$, $R_2$, $R_3$ ou $R_4$.
Selon un mode de réalisation particulier, l'invention concerne l'utilisation telle que définie ci-dessus, dans laquelle le catalyseur répond à la Formule III-B dans laquelle l'un au moins des groupements $L_1$ et $L_2$ représente un atome d'iode.
Selon un mode de réalisation particulier, l'invention concerne le procédé de préparation tel que défini ci-dessus, dans lequel le catalyseur répond à la Formule III-1B :

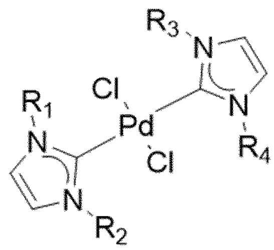

Formule III-1B

dans laquelle :

$R_1$, $R_2$, $R_3$ et $R_4$ représentent indépendamment l'un de l'autre un groupement choisi parmi :

- alkyle en $C_1$ à $C_{10}$, linéaire ou ramifié,
- cycloalkyle en $C_3$ à $C_{10}$, et
- aryle en $C_6$ à $C_{20}$ ou hétéroaryle en $C_3$ à $C_{20}$,
- alkyl-aryle en $C_7$ à $C_{20}$ ou alkyle-hétéroaryle en $C_4$ a $C_{20}$,

ledit composé de Formule III-1B pouvant être lié à un support, notamment un polymère ou de la silice, par au moins un des groupements $R_1$, $R_2$, $R_3$ ou $R_4$,
éventuellement l'un des groupements $R_1$ ou $R_2$ peut être relié à $R_3$ ou $R_4$ et représente ensemble un groupement bidentate comprenant deux groupements carbène N-hétérocyclique.

Selon un mode de réalisation particulier, l'invention concerne un procédé de préparation tel que défini ci-dessus, dans lequel le catalyseur de Formule III-1B lié à un support est choisi parmi :

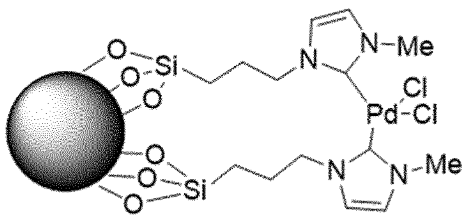

Selon un mode de réalisation particulier, l'invention concerne le procédé de préparation tel que défini ci-dessus, dans lequel le catalyseur répond à la Formule III-2B :

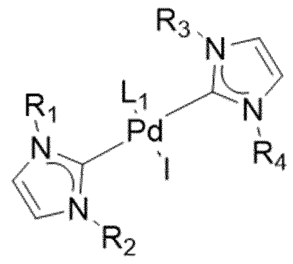

Formule III-2B

dans laquelle :

$R_1$, $R_2$, $R_3$ et $R_4$ représentent indépendamment l'un de l'autre un groupement choisi parmi :

- alkyle en $C_1$ à $C_{10}$, linéaire ou ramifié,
- cycloalkyle en $C_3$ à $C_{10}$, et
- aryle en $C_6$ à $C_{20}$ ou hétéroaryle en $C_3$ à $C_{20}$,
- alkyl-aryle en $C_7$ à $C_{20}$ ou alkyle-hétéroaryle en $C_4$ a $C_{20}$,

dans laquelle $L_1$ représente :

- un atome d'halogène choisi parmi Cl, Br et I, ou
- un ligand monodentate,

ledit composé de Formule III-2B pouvant être lié à un support, notamment un polymère ou de la silice, par au moins un des groupements $R_1$, $R_2$, $R_3$ ou $R_4$,
éventuellement l'un des groupements $R_1$ ou $R_2$ peut être relié à $R_3$ ou $R_4$ et représente ensemble un groupement bidentate comprenant deux groupements carbène N-hétérocyclique.

Selon un mode de réalisation particulier, l'invention concerne le procédé de préparation tel que défini ci-dessus, dans lequel le catalyseur répond à la Formule III-3B :

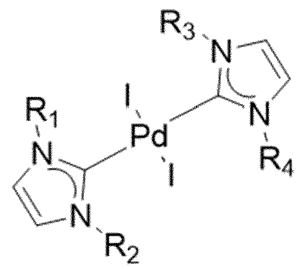

Formule III-3B

dans laquelle :

$R_1$, $R_2$, $R_3$ et $R_4$ représentent indépendamment l'un de l'autre un groupement choisi parmi :

- alkyle en $C_1$ à $C_{10}$, linéaire ou ramifié,
- cycloalkyle en $C_3$ à $C_{10}$, et
- aryle en $C_6$, à $C_{20}$ ou hétéroaryle en $C_3$ à $C_{20}$,
- alkyl-aryle en $C_7$ à $C_{20}$ ou alkyle-hétéroaryle en $C_4$ a $C_{20}$,

ledit composé de Formule III-3B pouvant être lié à un support, notamment un polymère ou de la silice, par au moins un des groupements $R_1$, $R_2$, $R_3$ ou $R_4$,
éventuellement l'un des groupements $R_1$ ou $R_2$ peut être relié à $R_3$ ou $R_4$ et représente ensemble un groupement bidentate comprenant deux groupements carbène N-hétérocyclique.

Selon un mode de réalisation particulier, l'invention concerne un procédé de préparation tel que défini ci-dessus, dans lequel le catalyseur répond à la Formule IV-B :

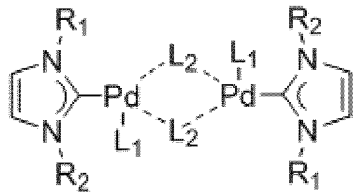

Formule IV-B

dans laquelle :

$R_1$ et $R_2$ représentent indépendamment l'un de l'autre un groupement choisi parmi :

- alkyle en $C_1$ à $C_{10}$, linéaire ou ramifié,
- cycloalkyle en $C_3$ à $C_{10}$,
- aryle en $C_6$ à $C_{20}$ ou hétéroaryle en $C_3$ à $C_{20}$,
- alkyl-aryle en $C_7$ à $C_{20}$ ou alkyle-hétéroaryle en $C_4$ à $C_{20}$,

$L^1$ et $L^2$ représentent un atome d'halogène choisi parmi Cl, Br et I.

*Autres paramètres*

Selon un mode de réalisation particulier, l'invention concerne un procédé de préparation tel que défini ci-dessus, dans lequel le promoteur est choisi parmi : l'iodure de tétrabutylammonium ($Bu_4NI$), le iodure de sodium (NaI) et le iodure de potassium (KI),
le promoteur étant notamment le iodure de tétrabutylammonium.

Selon un mode de réalisation particulier, l'invention concerne un procédé de préparation tel que défini ci-dessus, dans lequel le solvant est choisi parmi : l'acétonitrile, le toluène, l'1,4-dioxane, le tétrahydrofurane, l'éthanol, le méthanol et l'acétate d'éthyle,
le solvant étant notamment l'acétonitrile ou le tétrahydrofurane.

Selon un mode de réalisation particulier, l'invention concerne un procédé de préparation tel que défini ci-dessus, dans lequel l'oxydant est de l'oxygène ou de l'air, l'oxygène étant utilisé à raison de 0,5 à 2,5 MPa (5 à 25 bars).

Avantageusement, l'oxygène est utilisé à raison de 1,5 MPa pour le procédé de préparation des oxalates.

Avantageusement, l'oxygène est utilisé à raison de 1,0 MPa pour le procédé de préparation des oxamides.

L'expression MPa correspond à $10^6$ Pascal et est équivalente à 10 bars.

L'expression « de 0,5 à 2,5 MPa » correspond aux gammes : de 0,5 à 1,0 MPa ; de 1,0 à 1,5 MPa ; de 1,5 à 2,0 MPa ; de 2,0 à 2,5 MPa.

Selon un mode de réalisation particulier, l'invention concerne un procédé de préparation tel que défini ci-dessus, dans lequel le monoxyde de carbone est utilisé à raison de 1,0 à 10,0 MPa (10 à 100 bars), notamment à 6,5 MPa (65 bars).

L'expression « de 1,0 à 10,0 MPa » correspond aux gammes : de 1,0 à 1,5 MPa ; de 1,5 à 2,0 MPa ; de 2,0 à 2,5 MPa ; de 2,5 à 3,0 MPa ; de 3,0 à 3,5 MPa ; de 3,5 à 4,0 MPa ; de 4,0 à 4,5 MPa ; de 4,5 à 5,0 MPa ; de 5,0 à 5,5 MPa ; de 5,5 à 6,0 MPa ; de 6,0 à 6,5 MPa ; de 6,5 à 7,0 MPa ; de 7,0 à 7,5 MPa ; de 7,5 à 8,0 MPa ; de 8,0 à 8,5 MPa ; de 8,5 à 9,0 MPa ; de 9,0 à 9,5 MPa ; de 9,5 à 10 MPa.

Selon un mode de réalisation particulier, l'invention concerne un procédé de préparation tel que défini ci-dessus, dans lequel le ratio de pression monoxyde de carbone / oxygène utilisé est compris de 3 à 10, notamment d'environ 4.

L'expression « de 3 à 10 » correspond aux gammes : de 3 à 4 ; de 4 à 5 ; de 5 à 6 ; de 6 à 7 ; de 7 à 8 ; de 8 à 9 ; de 9 à 10.

Avantageusement, le ratio de pression $CO/O_2$ est d'environ 4 pour le procédé de préparation des oxalates.

Avantageusement, le ratio de pression $CO/O_2$ est d'environ 6,5 pour le procédé de préparation des oxamides.

Selon un mode de réalisation particulier, l'invention concerne un procédé de préparation tel que défini ci-dessus, dans lequel le catalyseur est utilisé à raison de 0,001 à 10%molaire par rapport à l'alcool ou l'amine.

L'expression « de 0,001 à 10 %» correspond aux gammes : de 0,001 à 0,005%; de 0,005 à 0,01%; de 0,01 à 0,05%; de 0,05 à 0,1%; de 0,1 à 0,15 %; de 0,15 à 0,2 %; de 0,2 à 0,5%; de 0,5 à 1 %; de 1 à 2%; de 2 à 3%; de 3 à 4%; de 4 à 5%; de 5 à 6%; de 6 à 7%; de 7 à 8%; de 8 à 9%; de 9 à 10%.

Selon un mode de réalisation particulier, l'invention concerne un procédé de préparation tel que défini ci-dessus, dans lequel le promoteur est utilisé à raison de 2 à 100 équivalents molaires par rapport au catalyseur.

Avantageusement, le promoteur est utilisé à raison de 5 équivalents molaires par rapport au catalyseur pour le procédé de préparation des oxalates.

Avantageusement, le promoteur est utilisé à raison de 62,5 équivalents molaires par rapport au catalyseur pour le procédé de préparation des oxamides.

Selon un mode de réalisation particulier, l'invention concerne un procédé de préparation tel que défini ci-dessus, dans lequel la base est utilisée à raison de 2 à 150 équivalents molaires par rapport au catalyseur.

Avantageusement, la base est utilisée à raison de 5 équivalents molaires par rapport au catalyseur pour le procédé de préparation des oxalates.

Avantageusement, le base est utilisée à raison de 125 équivalents molaires par rapport au catalyseur pour le procédé de préparation des oxamides.

Selon un mode de réalisation particulier, l'invention concerne un procédé de préparation tel que défini ci-dessus, d'un composé oxalate ou d'un composé oxamide comprenant :

- une étape A de mise en contact d'un alcool ou d'une amine, respectivement, avec :

  - du monoxyde de carbone, en particulier utilisé à raison de 1,0 à 10,0 MPa, notamment à 6,5 MPa,
  - un oxydant, en particulier de l'oxygène ou de l'air, de préférence de l'oxygène utilisé à raison de 0,5 à 2,5 MPa,
  - un catalyseur M-NHC, dans lequel M représente Pd ou Pt et NHC représente un groupement carbène *N*-hétérocyclique, comprenant au moins un atome M lié à au moins un ligand carbène *N*-hétérocyclique, et
  - éventuellement un promoteur, en particulier choisi parmi : l'iodure de tétrabutylammonium ($Bu_4NI$), l'iodure de sodium (NaI) et l'iodure de potassium (KI), de préférence l'iodure de tétrabutylammonium,
  - éventuellement une base, en particulier choisi parmi le carbonate de potassium ($K_2CO_3$), le carbonate de sodium ($Na_2CO_3$), le *tert*-butylate de potassium (KOtBu), le phosphate de potassium ($K_3PO_4$) et la triéthylamine ($Et_3N$),
  - éventuellement un solvant, en particulier l'acétonitrile, le toluène, l'1,4-dioxane, le tétrahydrofurane, l'éthanol, le méthanol et l'acétate d'éthyle, de préférence l'acétonitrile et le tétrahydrofurane,

  pour obtenir un milieu réactionnel,
- éventuellement une étape B de chauffage dudit milieu réactionnel,

pour obtenir le composé oxalate, ou le composé oxamide.

Selon un mode de réalisation particulier, l'invention concerne un procédé de préparation tel que défini ci-dessus, dans lequel le procédé comprend en outre, après l'étape B de chauffage, une étape C de filtration du milieu réactionnel pour obtenir un catalyseur récupéré, et un filtrat dépourvu de catalyseur,
ledit catalyseur étant un catalyseur supporté tel que défini ci-dessus.

Selon un mode de réalisation particulier, l'invention concerne un procédé de préparation tel que défini ci-dessus, dans lequel le catalyseur utilisé dans l'étape A de mise en contact, est un catalyseur récupéré à l'issue d'une étape C de filtration.

Avantageusement, le catalyseur récupéré après le procédé de l'invention n'est pas dégradé et est stable, et il peut être réutilisé dans un autre processus de réaction catalytique. Ainsi il est possible de réitérer le procédé selon l'invention avec le même catalyseur récupéré. Selon un mode de réalisation particulier, l'invention concerne un procédé de préparation tel que défini ci-dessus, d'un composé oxalate ou d'un composé oxamide, dans lequel le catalyseur est stable en fin de réaction et peut être réutilisé dans un autre procédé de réaction catalytique.

Selon un mode de réalisation particulier, l'invention concerne un procédé de préparation tel que défini ci-dessus, dans lequel les étapes A, B et C sont répétées au moins 5 fois, sans perte substantielle de l'activité catalytique du catalyseur.

Selon un mode de réalisation particulier, l'invention concerne un procédé de préparation tel que défini ci-dessus, dans lequel le procédé est effectué en flux continu, le catalyseur étant un catalyseur supporté tel que défini ci-dessus.

Selon un mode de réalisation particulier, l'invention concerne un procédé de préparation d'oxalates ou d'oxamides tel que défini ci-dessus, réalisé en flux continu,

dans lequel le catalyseur est un catalyseur supporté introduit dans une colonne ou une cartouche,
ou dans lequel le catalyseur est en suspension dans le mélange réactionnel.

À titre d'exemple non limitatif, le procédé en flux continu est réalisé dans un réacteur de type :

- un réacteur à cuve agitée en continu (CSTR),
- un réacteur à écoulement ou un réacteur tubulaire,
- un réacteur à lit fixe ou garni.

À titre non limitatif, le procédé selon l'invention peut être mis en oeuvre dans un appareil de chimie en flux, par exemple dans les réacteurs commerciaux tels que « H-Cube Pro® » ou « Phoenix® » de la société ThalesNano INC. (7 Zahony Street, Graphisoft Park, Building D, H-1031 Budapest, Hongrie) ou tels que les réacteurs « E-Series » ou « R-Series flow chemistry systems » de la société Vapourtec Ltd (Unit 21/Park Farm Business Centre/Fornham Pk, Bury Saint Edmunds IP28 6TS, Royaume-Uni).

Avantageusement le procédé en flux continu est réalisé à une température de 25°C à 200°C. Avantageusement, le procédé en flux continu est réalisé à une pression de 0,1 MPa à 4 MPa. L'expression de « 0,1 à 4 MPa » correspond aux gammes suivantes : de 0,1 à 0,5 MPa; de 0,5 à 1,0 MPa; de 1,0 à 1,5 MPa; de 1,5 à 2,0 MPa; de 2,0 à 2,5 MPa; de 2,5 à 3,0 MPa; de 3,0 à 3,5 MPa; de 3,5 à 4,0MPa.

Selon un mode de réalisation particulier, le procédé en flux continu est réalisé dans un réacteur dans lequel les gaz représentent 10 à 90% du volume du réacteur.

L'expression de « 10 à 90% » correspond aux gammes suivantes : de 10 à 20%; de 20 à 30%; de 30 à 40%; de 40 à 50%; de 50 à 60%; de 60 à 70%; de 70 à 80%; de 80 à 90%.

Selon un mode de réalisation particulier, le procédé en flux continu est réalisé par des moyens permettant un temps de contact entre les réactifs de 1 seconde à 2 heures, en particulier de 1 seconde à 2 minutes.

L'expression de « 1 seconde à 2 heures » correspond aux gammes : de 1 à 15 secondes; de 15 à 30 secondes; de 30 secondes à 1 minute; de 1 à 2 minutes; de 2 à 15 minutes; de 15 à 30 minutes; de 30 minutes à 1 heure; de 1 à 2 heures.

Selon un mode de réalisation particulier, le procédé en flux continu comprend des moyens permettant d'introduire dans le réacteur le flux de CO en contact avec le substrat (l'alcool ou l'amine) et le flux d'oxygène ou d'air de façon individuelle ou en mélange.

Selon un mode de réalisation particulier, l'invention concerne un procédé de préparation tel que défini ci-dessus, dans lequel le produit oxalate, ou le produit oxamide, est isolé en absence de produits carbonate ou de produits urée respectivement, le ratio oxalate/carbonate étant supérieur à 98%, le ratio oxamide/urée étant supérieur à 98%.

Selon un mode de réalisation particulier, l'invention concerne un procédé de préparation tel que défini ci-dessus, dans lequel le produit oxalate, est isolé en absence de produits carbonate, le ratio oxalate/carbonate étant supérieur à 98%.

Selon un mode de réalisation particulier, l'invention concerne un procédé de préparation tel que défini ci-dessus, dans lequel le produit oxamide, est isolé en absence de produits urée, le ratio oxamide/urée étant supérieur à 98%.

À titre d'exemples non limitatifs, le produit oxalate ou le produit oxamide peuvent être isolés par distillation ou par extraction et recristallisation.

Selon un mode de réalisation particulier, l'invention concerne un procédé de préparation tel que défini ci-dessus, d'un composé oxalate ou d'un composé oxamide, dans lequel le rendement obtenu est supérieur à 100 mmol du composé oxalate ou du composé oxamide par mmol de Pd.

Selon un mode de réalisation particulier, l'invention concerne un procédé de préparation tel que défini ci-dessus, d'un composé oxalate, dans lequel le rendement obtenu est supérieur à 100 mmol du composé oxalate par mmol de Pd.

Selon un mode de réalisation particulier, l'invention concerne un procédé de préparation tel que défini ci-dessus, d'un composé oxamide, dans lequel le rendement obtenu est supérieur à 200 mmol du composé oxamide par mmol de Pd.

Avantageusement, le procédé de préparation des oxamides selon l'invention permet d'atteindre des rendements de plus de 90%, notamment de l'ordre de 97%.

Les rendements du procédé de préparation d'oxalates ou d'oxamides selon l'invention peuvent être décrits en termes de « Nombre de Cycles Catalytique (NCC) »

Notamment l'activité catalytique dans des conditions de température, pression et concentrations des solutés et de temps donnés du procédé de préparation d'oxalates ou d'oxamides selon l'invention peut être décrite en termes de NCC.

Le « Nombre de Cycles Catalytique (NCC) » est défini comme le rapport entre le nombre de moles de produit formé ($n_{prod}$) et le nombre de moles de métal (Pd ou Pt) du catalyseur ($n_{cat}$) :

$$NCC = \frac{n_{prod}}{n_{cat}}$$

À la différence du « Turnover Number (TON) » qui représente le maximum de cycles catalytiques qu'un catalyseur peut atteindre avant sa dégradation totale et irréversible, le Nombre de Cycles Catalytique représente un

nombre total de cycles catalytiques réalisés par le catalyseur dans des conditions données de réaction. En fin de réaction, le catalyseur utilisé ne serait pas forcément dégradé et pourrait donc être réutilisé. Ainsi le NCC n'est pas une mesure de la durée de vie d'un catalyseur, mais permet de mesurer la productivité du catalyseur sous des conditions données de la réaction catalysée.

Selon un mode de réalisation particulier, l'invention concerne un procédé de préparation tel que défini ci-dessus, d'un composé oxalate, dans lequel le NCC obtenu est supérieur à 100. Selon un mode de réalisation particulier, l'invention concerne un procédé de préparation tel que défini ci-dessus, d'un composé oxamide, dans lequel le NCC est supérieur à 200.

Selon un mode de réalisation particulier, l'invention concerne un procédé de préparation tel que défini ci-dessus, d'un composé oxalate ou d'un composé oxamide, dans lequel la sélectivité vers le produit oxalate, ou le produit oxamide, est supérieure à 70 %, en particulier d'environ 80%.

Selon un mode de réalisation particulier, l'invention concerne un procédé de préparation tel que défini ci-dessus, d'un composé oxalate dans lequel la sélectivité vers le produit oxalate, est supérieure à 75%, en particulier d'environ 80%.

Selon un mode de réalisation particulier, l'invention concerne un procédé de préparation tel que défini ci-dessus, d'un composé oxamide, dans lequel la sélectivité vers le produit oxamide, est supérieure à 95%, en particulier d'environ 98%.

Les exemples suivants illustrent l'invention, sans en limiter la portée.

**EXEMPLES**

Exemple 1 : Les catalyseurs

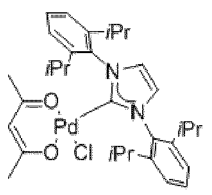

[(IPr)Pd(acac)Cl]

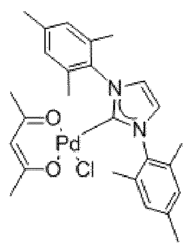

[(IMes)Pd(acac)Cl]

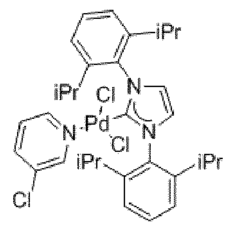

PEPPSI™-IPr

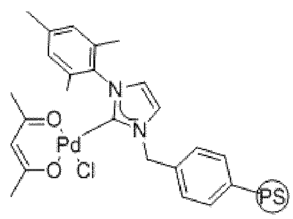

[(PS-IMes)Pd(acac)Cl]

Le catalyseur PEPPSI™-IPr peut être commercialement obtenu auprès de Sigma Aldrich.

Les catalyseurs [(IPr)Pd(acac)Cl], [(IMes)Pd(acac)Cl] ont été préparés selon la littérature décrite dans N. Marion et al. (Adv. Synth. Catal. 2007, 349, 2380-2384).

Le support polymérique chargé d'1-(mesityl)imidazolium (PS-IMes-HCl), a été préparé selon la littérature décrite dans D.-H. Lee, et al. (Org. Lett. 2008, 10, 1609-1612).

La quantité d'imidazolium sur le support polymérique chargé d'imidazolium (PS-IMes-HCl) a été déterminée en évaluant la teneur en azote N par analyse élémentaire (N 0.89%, soit une quantité en catalyseur de: 0.32 mmol/g).

Le support (PS-IMes-HCl) est utilisé pour la préparation du catalyseur [(PS-IMes)Pd(acac)Cl] décrite dans l'exemple 2.

Exemple 2 - *Préparation du catalyseur [(PS-IMes)Pd(acac)Cl]*

Dans un ballon équipé d'un barreau magnétique et d'un condenseur, du $Pd(acac)_2$ (456 mg, 1,5 mmol), un support polymérique chargé d'imidazolium (PS-IMes-HCl) (2 g, 0,32 mmol/g) et du 1,4-dioxane (30 ml) ont été introduits et le mélange réactionnel formé a été chauffé à 100°C pendant 16 h. Ensuite, le mélange réactionnel a été refroidi à température ambiante, filtré et le support polymère a été lavé vigoureusement avec de l'eau distillée (5 x 10 ml), du méthanol (5 x 10 ml) et séché sous pression réduite pour obtenir [(PS-IMes) Pd(acac)Cl] (2,1 g).

La quantité de Pd chargée sur le support polymère a été déterminée en utilisant l'analyse ICP-AES. Le complexe palladium-N-hétérocyclique supporté par polymère (50 mg) a été traité avec un mélange (25 ml) d'acide chlorhydrique et d'acide nitrique (1:1, v/v) à température ambiante pendant 30 min. La solution orangée formée est filtrée et lavée à l'eau distillée. Le filtrat et la solution de lavage ont été combinés pour déterminer la quantité de Pd par spectrométrie d'émission atomique à plasma à couplage inductif (ICP-AES). La quantité de Pd calculée a été évaluée à 0,29 mmol/g de support.

Exemple 3 : Procédure représentative pour les tests comparatifs avec des catalyseurs Pd-phosphine

Essai A :

Dans un mode opératoire général, un autoclave Parr de 450 ml équipé d'un barreau d'agitation a été chargé d'acétylacétonate de palladium (II) (91,3 mg, 0,3 mmol), de triphénylphosphine (236,1 mg, 0,9 mmol), d'iodure de tétrabutylammonium (0,5 g, 1,5 mmol), triéthylamine (0,2 ml, 1,5 mmol), acétonitrile (100 ml) et éthanol absolu (50 ml). Le réacteur a été scellé, le mélange a été purgé trois fois à l'azote (5 bars), puis deux fois à l'oxygène (5 bars). L'autoclave est ensuite pressurisé avec 15 bars d'oxygène puis 65 bars de monoxyde de carbone pour obtenir une pression totale à 80 bars ; et le milieu réactionnel a été agité à 90°C pendant 14 h. L'autoclave a ensuite été laissé refroidir à température ambiante avant d'être dépressurisé lentement et purgé trois fois avec de l'azote (5 bars). Le contenu du réacteur a été transféré dans un ballon à fond rond et l'excès d'éthanol et de solvant a été éliminé par un évaporateur rotatif et l'oxalate de diéthyle DEO (9,3 g) a été récupéré par distillation sous vide (point d'ébullition = 120 °C / 30-15 mbar).

Pour les réactions avec les alcools, les résultats sont indiqués en termes de masse de produit oxalate obtenus et décrits en nombre de cycle catalytique NCC tel que défini ci-dessus.

Essai B

L'essai B est réalisé dans les mêmes conditions opératoires que l'essai A mais en l'absence d'iodure de tétrabutylammonium. Des traces d'oxalate de diéthyle ont été observées par analyse quantitative par chromatographie en phase gazeuse.

Essai C

L'essai C est réalisé dans les mêmes conditions opératoires que l'essai A mais en l'absence de triéthylamine. Des traces d'oxalate de diéthyle ont été observées par analyse quantitative par chromatographie en phase gazeuse.

Le tableau 1 reporte les conditions opératoires et les résultats (masse obtenue de l'oxalate et NCC) des essais réalisés avec un catalyseur palladium-phosphine.

Tableau 1 : Conditions opératoires des essais réalisés avec un catalyseur palladium-phosphine et les résultats de rendement (masse obtenue de l'oxalate et NCC)

| Ex | n (EtOH), mmol | [Pd]cat, mol% | *n*$Bu_4NI$, mol% | base, mol% | solvant, ml | $O_2$/CO, bar | T, °C | Durée, h | m (DEO) g | NCC |
|---|---|---|---|---|---|---|---|---|---|---|
| A | 856 | $Pd(acac)_2$ : $PPh_3$ = 1:3 0.04 | 0.18 | $NEt_3$, 0.18 | $CH_3CN$, 100 | 15/65 | 90 | 14 | 9,3 | 212 |
| B | 856 | $Pd(acac)_2$ : $PPh_3$ = 1:3 0.04 | - | $NEt_3$, 0.18 | $CH_3CN$, 100 | 15/65 | 90 | 14 | traces | - |
| C | 856 | $Pd(acac)_2$ : $PPh_3$ = 1:3 0.04 | 0.18 | - | $CH_3CN$, 100 | 15/65 | 90 | 14 | traces | - |

Exemple 4 : Procédure représentative pour la carbonylation oxydative catalytique d'alcools aliphatiques en oxalates à l'aide de catalyseurs homogènes au palladium

Dans une procédure générale, un autoclave Parr de 450 ml équipé d'un barreau d'agitation a été chargé de complexe homogène Pd-NHC (0,3 mmol), d'iodure de tétrabutylammonium (0,5 g, 1,5 mmol), de triéthylamine (0,2 ml, 1,5 mmol), d'acétonitrile (100 ml) et de l'alcool aliphatique (50 ml). Le réacteur a été scellé, le mélange a été purgé trois fois à l'azote (5 bars), puis deux fois à l'oxygène (5 bars). L'autoclave a été ensuite pressurisé avec 15 bars d'oxygène puis 65 bars de monoxyde de carbone pour amener à une pression totale à 80 bars; et le mélange réactionnel a été agité à 90 °C pendant 14 h. L'autoclave a ensuite été laissé refroidir à température ambiante avant d'être dépressurisé lentement et purgé trois fois avec de l'azote (5 bars). Le contenu du réacteur a été transféré dans un ballon à fond rond et l'excès d'alcool aliphatique et de solvant a été éliminé par un évaporateur rotatif et l'oxalate de dialkyle (DEO) a été récupéré par distillation sous vide (point d'ébullition = 120 °C / 50-15 mbar). Le rendement en oxalate de dialkyle rapporté a été calculé sur le rendement isolé en masse et un Nombre de Cycle Catalytique NCC a été calculé comme défini ci-dessus.

Le tableau 2 reporte les conditions opératoires et les résultats (masse obtenue de l'oxalate et NCC) des essais

réalisés avec un catalyseur Pd-NHC homogène selon l'invention.

Tableau 2 : Conditions opératoires des essais réalisés avec un catalyseur Pd-NHC homogène et les résultats de rendement (masse obtenue de l'oxalate) et NCC.

| Ex | n (EtOH), mmol | [Pd]cat, mol% | $nBu_4NI$, mol% | base, mol% | solvant, ml | $O_2$/CO, bar | T, °C | Durée, h | m (DEO) g | NCC |
|---|---|---|---|---|---|---|---|---|---|---|
| 1 | 856 | 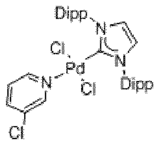 0.04 | 0.18 | $NEt_3$, 0.18 | $CH_3CN$, 100 | 15/65 | 90 | 14 | 8 | 182 |
| 2 | 856 | 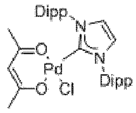 0.04 | 0.18 | $NEt_3$, 0.18 | $CH_3CN$, 100 | 15/65 | 90 | 14 | 10 | 228 |
| 3 | 856 | 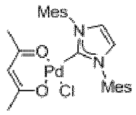 0.04 | 0.18 | $NEt_3$, 0.18 | $CH_3CN$, 100 | 15/65 | 90 | 14 | 9,5 | 216 |

Exemple 5 : Influence du promoteur et de la base sur la réaction de carbonylation oxydative d'alcool

Essai A1- présence d'eau : L'essai A1 est réalisé dans les mêmes conditions opératoires que l'essai 1 dans l'exemple 4 mais en présence d'eau (1 mol%). Le rendement réactionnel est équivalent à l'essai 1 dans l'exemple 4.

Essai A2- sans base $Et_3N$ :

Essai A3- sans $nBu_4NI$ :

Le tableau 3 reporte les conditions opératoires et les résultats (masse obtenue de l'oxalate et NCC) des essais réalisés.

Tableau 3 reporte les conditions opératoires des essais réalisés avec Pd-NHC et les résultats de rendement (masse obtenue de l'oxalate) et NCC.

| # | n (EtOH), mmol | [Pd]cat, mol% | $nBu_4NI$, mol% | $Et_3N$, mol% | $CH_3CN$, ml | $O_2$/CO, Bar | T, °C | Durée, h | m (DEO), g | NCC |
|---|---|---|---|---|---|---|---|---|---|---|
| 1 | 856 | 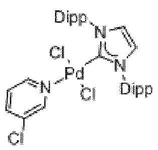 0.04 | 0.18 | 0.18 | 100 | 15/65 | 90 | 14 | 8 | 182 |
| A1 | 856 | 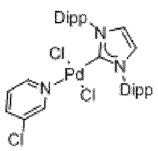 0.04 | 0.18 | 0.18 | 100 et $H_2O$ (1 mol%) | 15/65 | 90 | 14 | 7.8 | 178 |

(suite)

| # | n (EtOH), mmol | [Pd]cat, mol% | $n$Bu$_4$NI, mol% | Et$_3$N, mol% | CH$_3$CN, ml | O$_2$/CO, Bar | T, °C | Durée, h | m (DEO), g | NCC |
|---|---|---|---|---|---|---|---|---|---|---|
| A2 | 856 | 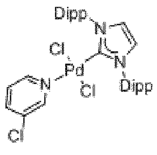 0.04 | 0.18 | - | 100 | 15/65 | 90 | 14 | <1.0 | - |
| A3 | 856 | 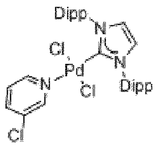 0.04 | - | 0.18 | 100 | 15/65 | 90 | 14 | <0.1 | - |

Exemple 6 : Procédure représentative pour la carbonylation oxydative catalytique d'alcools aliphatiques en oxalates à l'aide d'un catalyseur Pd-NHC hétérogène selon l'invention

Dans une procédure générale, un autoclave Parr de 450 ml équipé d'un barreau d'agitation a été chargé de complexe hétérogène Pd-NHC (1,2 g, 0,29 mmol Pd/g, 0,35 mmol Pd), de l'iodure de tétrabutylammonium (0,5 g, 1,5 mmol), de la triéthylamine (0,2 ml, 1,5 mmol), l'acétonitrile (100 ml) et l'alcool aliphatique (50 ml). Le réacteur a été scellé, le mélange réactionnel a été purgé trois fois à l'azote (5 bars), puis deux fois à l'oxygène (5 bars). L'autoclave est ensuite pressurisé avec 15 bars d'oxygène puis 65 bars de monoxyde de carbone pour amener à une pression totale à 80 bars ; et le mélange réactionnel a été agité à 90 °C pendant 14 h. L'autoclave a ensuite été laissé refroidir à température ambiante avant d'être dépressurisé lentement et purgé trois fois avec de l'azote (5 bars). Le contenu du réacteur a été filtré pour récupérer le catalyseur. Le filtrat a été transféré dans un ballon à fond rond et l'excès d'alcool aliphatique et de solvant ont été éliminés par un évaporateur rotatif et l'oxalate de dialkyle a été récupéré par distillation sous vide (point d'ébullition = 120 °C / 50-15 mbar).

Le rendement en oxalate de dialkyle rapporté a été calculé sur le rendement isolé en masse et un Nombre de Cycle Catalytique NCC a été calculé tel que défini ci-dessus.

Le tableau 4 reporte les conditions opératoires et les résultats (masse obtenue de l'oxalate et NCC) d'un essai réalisé avec un catalyseur Pd-NHC hétérogène selon l'invention.

Tableau 4 : Conditions opératoires de l'essai avec le catalyseur [(PS-IMes)Pd(acac)Cl] et les résultats de rendement (masse obtenue de l'oxalate) et NCC.

| # | n (EtOH), mmol | [Pd]cat, mol%, | *n*$Bu_4NI$, mol% | base, mol % | solvant, ml | $O_2/CO$, bar | T, °C | Durée, h | m (DEO) g | NCC |
|---|---|---|---|---|---|---|---|---|---|---|
| 4 | 856 | 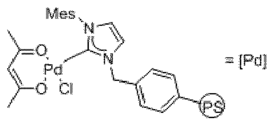 = [Pd] 0,04 | 0.18 | $NEt_3$, 0.18 | $CH_3CN$, 100 | 15/65 | 90 | 14 | 8 | 182 |

Exemple 7 : Procédure représentative pour l'étude de recyclabilité du complexe [(PS-IMes)Pd(acac)Cl]

La réaction a été effectuée comme mentionné dans l'exemple 6 ci-dessus dans une procédure expérimentale typique. Cependant, une fois la réaction terminée, ventilée et purgée à l'azote, le catalyseur a été filtré et lavé vigoureusement avec de l'eau distillée (5 × 10 ml) et du méthanol (5 × 10 ml) pour éliminer toute trace de produit ou de réactifs présents. Le catalyseur filtré a ensuite été séché sous pression réduite avant le recyclage suivant. Le catalyseur séché a ensuite été utilisé pour une expérience de recyclabilité du catalyseur, et il a été observé que le catalyseur récupéré pouvait être réutilisé au moins jusqu'à cinq cycles consécutifs donnant un rendement bon à appréciable du produit souhaité.

L'étude de recyclabilité démontre une application possible du catalyseur hétérogène dans un réacteur à flux continu.

Le tableau 5 reporte les conditions et les résultats des essais de recyclabilité du complexe [(PS-IMes) Pd(acac) Cl].

Tableau 5 : Conditions des essais de recyclabilité du complexe [(PS-IMes)Pd(acac)Cl] et les résultats de rendement.

| exp | Cycle N | n[Pd], mmol | Conditions de carbonylation | m (DEO), g | NCC | NCC total |
|---|---|---|---|---|---|---|
| C1 | 1 | 0.3 | 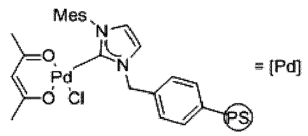 856 mmol (50 mL) EtOH; 1.5 mmol $NEt_3$; 1.5 mmol $n$$Bu_4NI$ ; $CH_3CN$ 100 mL ; $CO/O_2$ = 65/15, 90 °C, 14h | 8 | 182 | 1096 |
| C2 | 2 | 0.24 | | 6 | 171 | |
| C3 | 3 | 0.198 | | 6 | 207 | |
| C4 | 4 | 0.15 | | 6 | 274 | |
| C5 | 5 | 0.1125 | | 4.3 | 262 | |

Les résultats de ces expériences de recyclage manuel démontrent que le catalyseur Pd-NHC est stable. En effet le catalyseur récupéré n'est pas ou peu sensible à l'eau et à l'oxygène. De plus il n'y a pas de perte d'efficacité en termes de NCC pendant les recyclages du catalyseur dans le cadre de plusieurs réactions catalytiques successives.

En passant en flux, il n'est plus nécessaire d'effectuer de recyclage manuel, prévenant une perte de catalyseur durant les lavages, ainsi l'efficacité devrait être conservée au cours de la réaction puisque les conditions réactionnelles seront stables.

**Oxamide**

Exemple 8 : Procédure représentative pour la carbonylation oxydative catalytique d'amines en oxamides

Dans un autoclave Parr de 450 ml équipé d'un barreau d'agitation, ont été introduits du PEPPSI™-IPr, de l'iodure de tétrabutylammonium, une base, un solvant et de pipéridine. Le réacteur a été scellé, le mélange a été purgé trois fois à l'azote (5 bars), puis deux fois à l'oxygène (5 bars). L'autoclave a été ensuite pressurisé avec 10 bars d'oxygène puis 65 bars de monoxyde de carbone pour obtenir à une pression totale à 75 bars ; et le milieu réactionnel a été agité à température ambiante pendant 18 h. Ensuite, la pression a été relâchée avec précaution et l'autoclave a été purgé trois fois avec de l'azote (5 bars). Le contenu du réacteur a été transféré dans un ballon à fond rond et les substances volatiles ont été éliminées sous pression réduite. Ensuite, le résidu a été extrait dans du toluène, filtré sur du gel de silice (2-3 cm) et la solution a été évaporée à sec pour donner l'1, 1'-oxalyl dipipéridine sous la forme d'une poudre blanc cassé.

Le rendement en oxamide est indiqué en masse de produit isolé et un Nombre de Cycle Catalytique NCC a été calculé tel que défini ci-dessus.

Exemple 9: Influence du solvant sur la carbonylation oxydative catalytique d'amine en oxamide.

Essai M1-THF

Dans un autoclave Parr de 450 ml équipé d'un barreau d'agitation, ont été introduits du catalyseur PEPPSI™-IPr (62,5 mg, 0,092 mmol), de l'iodure de tétrabutylammonium (2,12 g, 5,75 mmol), du carbonate de potassium (1,59 g, 11,5 mmol), du THF (200 ml) et de la pipéridine (22,7 ml, 230 mmol). Le réacteur a été scellé, le mélange a été purgé trois fois à l'azote (5 bars), puis deux fois à l'oxygène (5 bars). L'autoclave a été ensuite pressurisé avec 10 bars d'oxygène puis 65 bars de monoxyde de carbone pour obtenir une pression totale à 75 bars ; et le milieu réactionnel a été agité à température

ambiante pendant 18 h. Après ce temps, la pression a été relâchée avec précaution et l'autoclave a été purgé trois fois avec de l'azote (5 bars). Le contenu du réacteur a été transféré dans un ballon à fond rond et les substances volatiles ont été éliminées sous pression réduite. Ensuite, le résidu a été extrait dans du toluène, filtré sur du gel de silice (2-3 cm) et la solution a été évaporée à sec pour donner l'1, 1'-oxalyl dipipéridine sous la forme d'une poudre blanc cassé (9,5 g ; 42 mmol).

Essai M2 -Acétonitrile

L'essai M2 a été réalisé dans les mêmes conditions que l'essai M1, excepté que 200 mL d'acétonitrile ont été utilisés à la place du THF.

Résultat : 9,5 g d'oxamide 2 ont été isolés.

Essai M3- sans solvant

L'essai M3 a été réalisé dans les mêmes conditions que l'essai M1, en l'absence de solvant. Résultat : 4,2 g d'oxamide 2 ont été isolés.

Le tableau 6 reporte les conditions sur la nature et la présence du solvant et les résultats des essais de carbonylation oxydative catalytique d'amines en oxamides avec des catalyseurs Pd-NHC homogènes selon l'invention.

Tableau 6 : Conditions opératoires avec différents solvants des essais avec la pipéridine avec des catalyseurs Pd-NHC et les résultats de rendement.

| Ex | n (pipéridine), mmol | [Pd]cat, mol% | *n*$Bu_4NI$, mol% | base, mol% | solvant, ml | $O_2/CO$, bar | T, °C | Durée, h | Rendement (oxamide) |
|---|---|---|---|---|---|---|---|---|---|
| M1 | 230 | 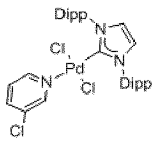 0.04 | 2.5 | $K_2CO_3$ 5 | THF, 200 | 10/65 | 25 | 18 | 9.5 g NCC = 460 |
| M2 | 230 | 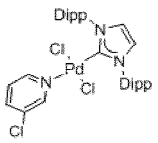 0.04 | 2.5 | $K_2CO_3$ 5 | $CH_3CN$, 200 | 10/65 | 25 | 18 | 9.5 g NCC = 460 |
| M3 | 230 | 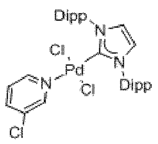 0.04 | 2.5 | $K_2CO_3$ 5 | - | 10/65 | 25 | 18 | 4.2 g NCC = 203 |

La réaction s'opère aussi bien en présence de THF que de $CH_3CN$ avec la même efficacité comme le démontre les essais M1 et M2.

La réaction s'opère en l'absence de solvant comme le démontre l'essai M3.

Exemple 10 : Influence de la base sur la carbonylation oxydative catalytique d'amine en oxamide.

Essai M4

L'essai M4 a été réalisé dans les mêmes conditions que l'essai M1, excepté que la base utilisée est la triéthylamine (5 mol %).

Essai M5

L'essai M5 a été réalisé dans les mêmes conditions que l'essai M1, sans base ajoutée.

Le tableau 7 reporte les conditions sur la nature et la présence de la base ajoutée et les résultats des essais de

carbonylation oxydative catalytique d'amines en oxamides avec des catalyseurs Pd-NHC homogènes selon l'invention.

Tableau 7 : Conditions opératoires avec différentes bases des essais avec la pipéridine avec des catalyseurs Pd-NHC et les résultats de rendement.

| Ex | n (pipéridine), mmol | [Pd]cat, mol% | $n$Bu$_4$NI, mol% | base, mol% | solvant, ml | $O_2$/CO, bar | T, °C | Durée, h | Rendement (oxamide) |
|---|---|---|---|---|---|---|---|---|---|
| M1 | 230 | 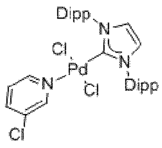 0.04 | 2.5 | $K_2CO_3$ 5 | THF, 200 | 10/65 | 25 | 18 | 9.5 g NCC = 460 |
| M4 | 230 | 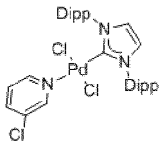 0.04 | 2.5 | $NEt_3$ 5 | THF, 200 | 10/65 | 25 | 18 | 9.3 g NCC = 450 |
| M5 | 230 | 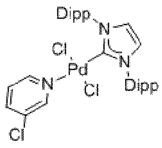 0.04 | 2.5 | - | THF, 200 | 10/65 | 25 | 18 | 17.0 g NCC = 823 |

Les bases organiques et inorganiques ont des effets comparables sur la réaction comme le démontre les résultats de M1 et M4.

La réaction est plus efficace en l'absence d'une base ajoutée dans le mélange réactionnel comme le démontre l'essai M5.

Exemple 11 : Utilisation catalyseur [(PS-IMes)Pd(acac)Cl] pour la carbonylation oxydative catalytique d'amines en oxamides à l'aide de catalyseur hétérogène Pd-NHC :

Essai M6

Dans un autoclave Parr de 450 ml équipé d'un barreau d'agitation, ont été introduit du catalyseur [(PS-Imes) Pd(acac)Cl] (317 mg, 0,29 mmol Pd/g, 0,092 mmol Pd), de l'iodure de tétrabutylammonium (2,12 g, 5,75 mmol), du THF (200 ml) et de la pipéridine (22,7 ml, 230 mmol). Le réacteur a été scellé, le mélange a été purgé trois fois à l'azote (5 bars), puis deux fois à l'oxygène (5 bars). L'autoclave a ensuite été pressurisé avec 10 bars d'oxygène puis 65 bars de monoxyde de carbone pour obtenir une pression totale à 75 bars ; et le milieu réactionnel a été agité à température ambiante pendant 18 h. Puis, la pression a été relâchée avec précaution et l'autoclave a été purgé trois fois avec de l'azote (5 bars). Le contenu du réacteur a été filtré pour récupérer le catalyseur. Le filtrat a été transféré dans un ballon à fond rond et les volatils ont été éliminés sous pression réduite. Ensuite, le résidu a été extrait dans du toluène, filtré sur du gel de silice (2-3 cm) et la solution a été évaporée à sec pour donner l'1, 1'-oxalyl dipipéridine sous la forme d'une poudre blanc cassé.

Le tableau 8 reporte les conditions opératoires d'un essai avec un catalyseur homogène et d'un essai avec un catalyseur supporté de carbonylation oxydative catalytique d'amines, la pipéridine, en oxamide, l'1, 1'-oxalyl dipipéridine, et les résultats de rendement.

Tableau 8 : Conditions opératoires avec un catalyseur homogène et avec un catalyseur supporté de carbonylation oxydative avec la pipéridine et les résultats de rendement.

| Ex | n (pipéridine), mmol | [Pd]cat, mol% | $nBu_4NI$, mol% | base, mol% | solvant, ml | $O_2/CO$, bar | T, °C | Durée, h | Rendement (oxamide) |
|---|---|---|---|---|---|---|---|---|---|
| M5 | 230 | 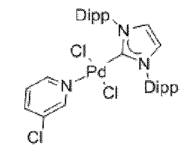 0.04 | 2.5 | - | THF, 200 | 10/65 | 25 | 18 | 17.0 g NCC = 823 66% |
| M6 | 230 | 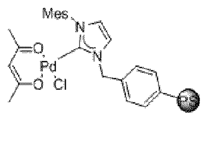 0.04 | 2.5 | - | THF, 200 | 10/65 | 25 | 18 | 18.0 g NCC = 872 70% |

Exemple 12 : Influence de l'introduction de CO et $O_2$

Essai M7

L'essai M7 a été réalisé dans les mêmes conditions que l'essai M5, excepté qu'après 18 heures d'agitation à température ambiante, le réacteur a été purgé et pressurisé une deuxième fois avec 10 bars d'oxygène et 65 bars de CO ; et le milieu réactionnel a été agité à température ambiante pendant 18 heures supplémentaires.

Le tableau 9 reporte les conditions opératoires.

Tableau 9 : Conditions opératoires sur l'influence des conditions de pression et de durée.

| Ex | n (pipéridine), mmol | [Pd]cat, mol% | $nBu_4NI$, mol% | base, mol% | solvant, ml | $O_2/CO$, bar | T, °C | Durée, H | Rendement (oxamide) |
|---|---|---|---|---|---|---|---|---|---|
| M5 | 230 | 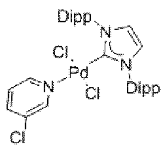 0.04 | 2.5 | - | THF, 200 | 10/65 | 25 | 18 | 17.0g NCC = 823 66% |
| M7 | 230 | 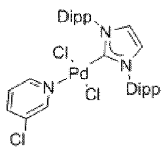 0.04 | 2.5 | - | THF, 200 | 10/65 for 18h + 10/65 for 18h | 25 | 36 | 25.0 g NCC = 1211 97% |

L'essai M7 montre que la re-pressurisation du réacteur avec du CO et du $O_2$ additionnel au cours de la réaction permet d'atteindre un rendement total.

Exemple 13 : Procédure représentative pour la carbonylation oxydative catalytique d'alcools aliphatiques en oxalates à l'aide de catalyseurs Pt-NHC.

Dans une procédure générale, un autoclave Parr de 450 ml équipé d'un barreau d'agitation est chargé de complexe homogène Pt-NHC (0,6 mmol), d'iodure de tétrabutylammonium (1,1 g, 3,0 mmol), de triéthylamine (0,4 ml, 3,0 mmol), d'acétonitrile (100 ml) et de l'alcool aliphatique (50 ml). Le réacteur est scellé, le mélange est purgé trois fois à l'azote (5 bars), puis deux fois à l'oxygène (5 bars). L'autoclave est ensuite pressurisé avec 15 bars d'oxygène puis 65 bars de monoxyde de carbone pour amener à une pression totale à 80 bars; et le mélange réactionnel est agité à 90 °C pendant

14 h. L'autoclave est ensuite laissé refroidir à température ambiante avant d'être dépressurisé lentement et purgé trois fois avec de l'azote (5 bars). Le contenu du réacteur est transféré dans un ballon à fond rond et l'excès d'alcool aliphatique et de solvant est éliminé par un évaporateur rotatif et l'oxalate de dialkyle (DEO) est récupéré par distillation sous vide (point d'ébullition = 120 °C / 50-15 mbar). Le rendement en oxalate de dialkyle rapporté est calculé sur le rendement isolé en masse et un Nombre de Cycle Catalytique NCC a été calculé tel que défini ci-dessus.

Exemple 14 : Procédure représentative pour la carbonylation oxydative catalytique d'amines en oxamides à l'aide de catalyseurs Pt-NHC :

Dans un autoclave Parr de 450 ml équipé d'un barreau d'agitation, sont introduits du catalyseur Pt-NHC (1,0 mmol), de l'iodure de tétrabutylammonium (2,12 g, 5,75 mmol), du THF (200 ml) et de la pipéridine (22,7 ml, 230 mmol). Le réacteur est scellé, le mélange est purgé trois fois à l'azote (5 bars), puis deux fois à l'oxygène (5 bars). L'autoclave est ensuite pressurisé avec 10 bars d'oxygène puis 65 bars de monoxyde de carbone pour obtenir une pression totale à 75 bars ; et le milieu réactionnel est agité à 90 °C pendant 14 h. L'autoclave est ensuite laissé refroidir à température ambiante avant d'être dépressurisé lentement et purgé trois fois avec de l'azote (5 bars). Le contenu du réacteur a été transféré dans un ballon à fond rond et les substances volatiles ont été éliminées sous pression réduite. Ensuite, le résidu a été extrait dans du toluène, filtré sur du gel de silice (2-3 cm) et la solution a été évaporée à sec pour donner l'1,1'-oxalyl dipipéridine.

Exemple 15 : Synthèse d'un catalyseur Pd-NHC supporté sur gel de silice ($PdCl_2$NHC/Si) de formule suivante :

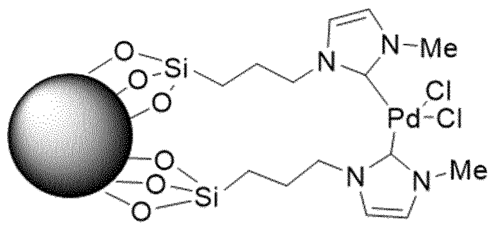

Le catalyseur a été préparé selon les étapes suivantes :

A) Synthèse du chlorure de 1-méthyl-3-(triméthoxysilylpropyl)-imidazolium selon le schéma réactionnel suivant :

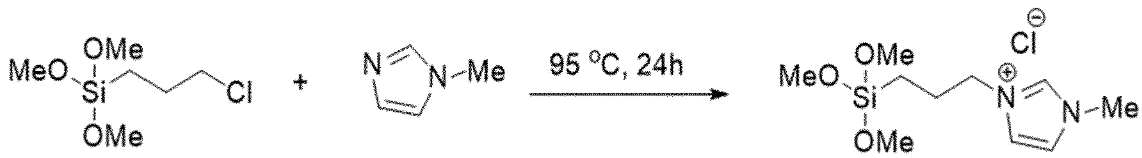


Dans un ballon équipé d'un barreau magnétique et d'un condenseur, Le mélange de N-méthyl imidazole (fraîchement distillé) (10,3160 g, 0,1258 mol) et de 3-chloropropyl triméthoxysilane (25 g, 0,1258 mol) a été porté à reflux à 95 °C pendant 24 heures. Après refroidissement à température ambiante, le mélange réactionnel a été lavé avec de l'éther diéthylique et séché sous vide pour obtenir le produit désiré.

B) Immobilisation du chlorure de 1-méthyl-3-(triméthoxysilylpropyl)-imidazolium à la surface du gel de silice selon le schéma réactionnel suivant :

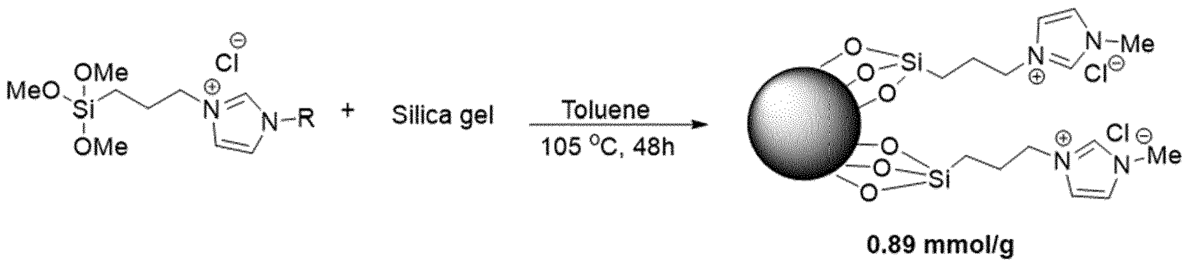


À une solution de chlorure de 1-méthyl-3-(triméthoxysilylpropyl)-imidazolium (0,70 g, 2,2 mmol) dans le toluene le gel de silice a été ajouté. Le mélange a été agité à 105 °C pendant 12 heures. Après refroidissement, le mélange réactionnel a été filtré et lavé avec du $CH_2Cl_2$ (3*10 mL), et séché à 60 °C sous vide pour donner le liquide ionique supporté sur silice (2.39 g). L'analyse élémentaire a montré la présence de 0,89 mmol de ligand sur 1,0 g de support.

C) Préparation d'un complexe NHC-Pd supporté sur du gel de silice selon le schéma réactionnel suivant

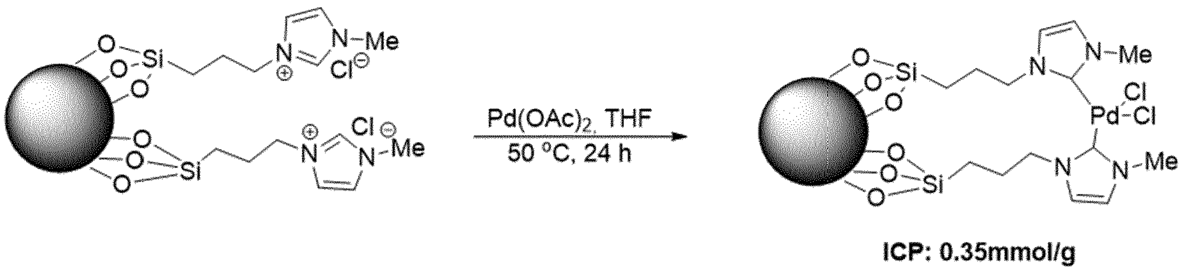


A une solution de ligand supporté sur silice (1,0 g, 0,89 mmol) dans du THF (5 mL), on a ajouté $Pd(OAc)_2$ (101 mg, 0,45 mmol). Le mélange a été agité pendant 4 h à 60 °C puis 30 min supplémentaires à 100 °C. Le complexe NHC-Pd supporté sur silice a été filtré sur un fritté et lavé à l'eau puis avec du $CH_2Cl_2$ (3 x10 mL). Une fois lavé, le catalyseur est séché. L'analyse ICP a montré la présence de 0.35 mmol de Pd sur 1g de support.

Avec une quantité de 0.35 mmol de Pd sur 1g de support, on observe une meilleure complexation du palladium sur ce ligand supporté par rapport au catalyseur [(PS-IMes)Pd(acac)Cl] de l'exemple 2 qui comprend une quantité de 0.29 mmol/g.

Exemple 16 : Etude de recyclabilité du catalyseur $PdCl_2NHC/Si$

Protocole

Dans une procédure générale, un autoclave Parr de 450 ml équipé d'un barreau d'agitation a été chargé de complexe hétérogène Pd-NHC (1,0 g, 0,35 mmol Pd/g), de l'iodure de tétrabutylammonium (0,5 g, 1,5 mmol), de la triéthylamine (0,2 ml, 1,5 mmol), l'acétonitrile (100 ml) et l'alcool aliphatique (50 ml). Le réacteur a été scellé, le mélange réactionnel a été purgé trois fois à l'azote (5 bars), puis deux fois à l'oxygène (5 bars). L'autoclave a été ensuite pressurisé avec 15 bars d'oxygène puis 65 bars de monoxyde de carbone pour amener à une pression totale à 80 bars, et le mélange réactionnel a été agité à 90 °C pendant 14 h. L'autoclave a ensuite été laissé refroidir à température ambiante avant d'être dépressurisé lentement et purgé trois fois avec de l'azote (5 bars). Le contenu du réacteur a été filtré pour récupérer le catalyseur. Le filtrat a été transféré dans un ballon à fond rond et l'excès d'alcool aliphatique et de solvant ont été éliminés par un évaporateur rotatif et l'oxalate de dialkyle a été récupéré par distillation sous vide (point d'ébullition = 120 °C / 50-15 mbar). Le rendement en oxalate de dialkyle rapporté a été calculé sur le rendement isolé en masse et un Nombre de Cycle Catalytique NCC a été calculé tel que défini ci-dessus.

Le tableau 10 reporte les conditions et les résultats des essais de recyclabilité avec le catalyseur $PdCl_2NHC/Si$.

Tableau 10 : Conditions des essais de recyclabilité avec le catalyseur $PdCl_2$NHC/Si et les résultats de rendement et de NCC.

| Exp | Cycle | n[Pd], mmol | Conditions de carbonylation | m(DEO), 8 | NCC | NCC total |
|---|---|---|---|---|---|---|
| A | 1 | 0.35 | 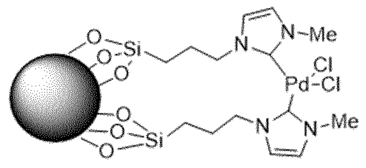 856 mmol (50 mL) EtOH ; 1.5 mmol $Net_3$; 1.5 mmol $n$$Bu_4$NI ; $CH_3CN$ 100 mL ;$CO/O_2$ = 65/15, 90 °C, 14h | 11 | 215 | 561 |
| B | 2 | 0.3 | | 8 | 182 | |
| C | 3 | 0.25 | | 6 | 164 | |

Exemple 17 : Préparation du catalyseur palladium avec un groupement NHC supporté avec des ligands iodés, $PdI_2$NHC/PS de formule suivante :

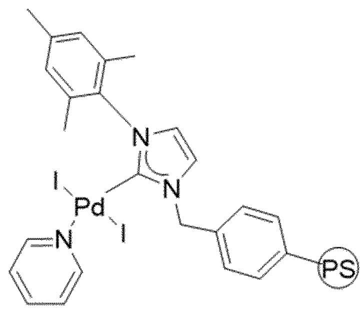

Dans un ballon équipé d'un barreau magnétique et d'un condenseur, du $PdCl_2$ (266 mg, 1,5 mmol), un support polymérique chargé d'imidazolium (PS-IMes-HCl) (1 g, 1,6 mmol/g), d'iodure de potassium (1,2 g, 7,5 mmol), carbonate de potassium (1,03 g, 7,5 mmol) et du pyridine (7 ml) ont été introduits et le mélange réactionnel formé a été chauffé à 80°C pendant 16 h. Ensuite, le mélange réactionnel a été refroidi à température ambiante, filtré et le support polymère a été lavé vigoureusement avec de l'eau distillée (5 x 10 ml), du méthanol (5 x 10 ml) et séché sous pression réduite pour obtenir (1,6 g) du produit désiré.

La quantité de Pd chargée sur le support polymère a été déterminée en utilisant l'analyse ICP-AES. Le complexe palladium-*N*-hétérocyclique supporté par polymère (50 mg) a été traité avec un mélange (25 ml) d'acide chlorhydrique et d'acide nitrique (1:1, v/v) à température ambiante pendant 30 min. La solution orangée formée a été filtrée et lavée à l'eau distillée. Le filtrat et la solution de lavage ont été combinés pour déterminer la quantité de Pd par spectrométrie d'émission atomique à plasma à couplage inductif (ICP-AES). La quantité de Pd calculé a été évaluée à 0,9 mmol/g de support.

Exemple 18 : Procédure de carbonylation sans sel d'iode ajouté

Les Inventeurs ont réussi à s'affranchir de l'ajout de sel d'iode. En effet, l'iode est directement incorporé au catalyseur tel que $PdI_2$NHC/PS préparé dans l'exemple 17 et permet de maintenir une ré oxydation du catalyseur efficace tout en évitant d'avoir à gérer l'ajout de sel d'iode additionnel.

Protocole

Un autoclave Parr de 450 ml équipé d'un barreau d'agitation a été chargé de complexe hétérogène Pd-NHC (1,0 g, 0,35 mmol Pd/g), de la triéthylamine (0,2 ml, 1,5 mmol), l'acétonitrile (100 ml) et d'éthanol (50 ml). Le réacteur a été scellé, le mélange réactionnel a été purgé trois fois à l'azote (5 bars), puis deux fois à l'oxygène (5 bars). L'autoclave a été ensuite pressurisé avec 15 bars d'oxygène puis 65 bars de monoxyde de carbone pour amener à une pression totale à 80 bars, et le mélange réactionnel a été agité à 90 °C pendant 14 h. L'autoclave a été refroidi à température ambiante avant d'être dépressurisé lentement et purgé trois fois avec de l'azote (5 bars). Le contenu du réacteur a été filtré pour récupérer le catalyseur. Le filtrat a été transféré dans un ballon à fond rond et l'éthanol résiduel ainsi que l'acetonitrile ont été éliminé grâce à l'utilisation d'un évaporateur rotatif. L'oxalate de dialkyle purifié a été récupéré par distillation sous vide (point

d'ébullition = 120 °C / 50-15 mbar). Le rendement en oxalate de dialkyle rapporté a été calculé sur le rendement isolé en masse et un Nombre de Cycle Catalytique NCC a été calculé tel que défini ci-dessus.

Le tableau 11 reporte les conditions opératoires et les résultats obtenus

Tableau 11 : Conditions opératoires de carbonylation avec le catalyseur $PdI_2NHC/PS$ sans ajour d'un sel d'iode comme promoteur et les résultats de rendement et de NCC.

| Réaction | Cat | Ethanol | MeCN | T, (°C) | CO (Bar) | $O_2$ (Bar) | Temps, (h) | Quantité (g) | NCC |
|---|---|---|---|---|---|---|---|---|---|
| 1 | $PdI_2NHC/PS$ 0,9 mmol (1g) | 50 mL | 100 mL | 90 | 65 | 15 | 16 | 8,5 g | 64 |

Exemple 19 : Procédure représentative pour la carbonylation oxydative catalytique d'amines en oxamides sans sel d'iode ajouté

Un autoclave Parr de 450 ml équipé d'un barreau d'agitation a été chargé de complexe hétérogène $PdI_2NHC/PS$ préparé selon l'exemple 17 (1,0 g, 0,9 mmol Pd/g), du THF (200 ml) et de la pipéridine (22,7 ml, 230 mmol). Le réacteur a été scellé, le mélange a été purgé trois fois à l'azote (5 bars), puis deux fois à l'oxygène (5 bars). L'autoclave a ensuite été pressurisé avec 10 bars d'oxygène puis 65 bars de monoxyde de carbone pour obtenir une pression totale à 75 bars ; et le milieu réactionnel a été agité à température ambiante pendant 18 h. Puis, la pression a été relâchée avec précaution et l'autoclave a été purgé trois fois avec de l'azote (5 bars). Le contenu du réacteur a été filtré pour récupérer le catalyseur. Le filtrat a été transféré dans un ballon à fond rond et les volatils ont été éliminés sous pression réduite. Ensuite, le résidu a été extrait dans du toluène, filtré sur du gel de silice (2-3 cm) et la solution a été évaporée à sec pour donner l'1, 1'-oxalyl dipipéridine sous la forme d'une poudre blanc cassé.

## Revendications

1. Utilisation d'un catalyseur M-NHC, dans lequel M représente Pd ou Pt et NHC représente un groupement carbène *N*-hétérocyclique, comprenant au moins un atome M lié à au moins un ligand carbène *N*-hétérocyclique, dans la mise en œuvre d'un procédé de préparation sélective d'oxalates ou d'oxamides, à partir de monoxyde de carbone, d'un oxydant, en particulier l'oxygène moléculaire ou l'air, et d'un alcool ou d'une amine respectivement, éventuellement en présence d'un promoteur.

2. Utilisation selon la revendication 1, dans laquelle le catalyseur répond à la Formule II :

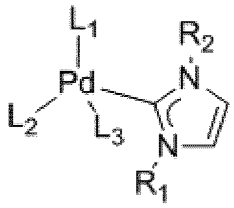

Formule II

dans laquelle :

$R_1$ et $R_2$ représentent indépendamment l'un de l'autre un groupement choisi parmi :

- alkyle en $C_1$ à $C_{10}$, linéaire ou ramifié,
- cycloalkyle en $C_3$ à $C_{10}$,
- aryle en $C_6$ à $C_{20}$ ou hétéroaryle en $C_3$ à $C_{20}$,
- alkyl-aryle en $C_7$ à $C_{20}$ ou alkyle-hétéroaryle en $C_4$ à $C_{20}$,

$L_1$ représente un atome d'halogène choisi parmi Cl, Br et I, et

- soit $L_2$ et $L_3$ représentent un ligand bidentate, en particulier choisi parmi l'acétylacétonate (acac), l'allyle, le

cinnamyle et l'acétate, de préférence l'acétylacétonate,
• soit $L_2$ représente un atome d'halogène choisi parmi Cl, Br et I,

et $L_3$ représente un ligand monodentate, en particulier choisi parmi : la pyridine, la 3-chloropyridine, l'acétonitrile, la triéthylamine, ou un ligand à base de phosphine, en particulier la triphénylphosphine, de préférence la 3-chloropyridine,
ledit composé de Formule II pouvant être lié à un support, notamment un polymère ou de la silice, par au moins un des groupements $R_1$ ou $R_2$.

**3.** Utilisation selon la revendication 2, dans laquelle le catalyseur répond à la Formule II , dans laquelle :
$L_1$ représente un atome d'iode, et

• soit $L_2$ et $L_3$ sont liés et représentent ensemble un ligand bidentate,
• soit $L_2$ représente un atome d'halogène choisi parmi Cl, Br et I et $L_3$ représente un ligand monodentate,

**4.** Utilisation selon l'une des revendications 2 ou 3, dans laquelle le catalyseur répond à la Formule II , dans laquelle :

$L_1$ et $L_2$ représentent chacun un atome d'iode
et $L_3$ représente un ligand monodentate,

**5.** Utilisation selon la revendication 1, dans laquelle le catalyseur répond à la Formule III :

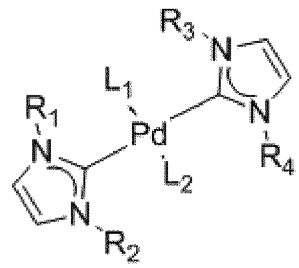

Formule III

dans laquelle :

$R_1$, $R_2$, $R_3$ et $R_4$ représentent indépendamment l'un de l'autre un groupement choisi parmi :

• alkyle en $C_1$ à $C_{10}$, linéaire ou ramifié,
• cycloalkyle en $C_3$ à $C_{10}$, et
• aryle en $C_6$ à $C_{20}$ ou hétéroaryle en $C_3$ à $C_{20}$,
• alkyl-aryle en $C_7$ à $C_{20}$ ou alkyle-hétéroaryle en $C_4$ a $C_{20}$,

soit $L_1$ et $L_2$ représentent indépendamment l'un de l'autre:

- un atome d'halogène choisi parmi Cl, Br et I, ou
- un ligand monodentate,

soit $L_1$ et $L_2$ sont liés et représentent un ligand bidentate,
ledit composé de Formule III pouvant être lié à un support, notamment un polymère ou de la silice, par au moins un des groupements $R_1$, $R_2$, $R_3$ ou $R_4$,
éventuellement l'un des groupements $R_1$ ou $R_2$ peut être relié à $R_3$ ou $R_4$ et représente ensemble un groupement bidentate comprenant deux groupements carbène N-hétérocyclique.

**6.** Utilisation selon la revendication 5, dans laquelle le catalyseur répond à la Formule III dans laquelle l'un au moins des groupements $L_1$ et $L_2$ représente un atome d'iode.

**7.** Utilisation selon l'une des revendications 5 ou 6, dans laquelle le catalyseur répond à la Formule III dans laquelle les groupements $L_1$ et $L_2$ représentent chacun un atome d'iode.

**8.** Utilisation selon la revendication 1, dans laquelle le catalyseur répond à la Formule IV :

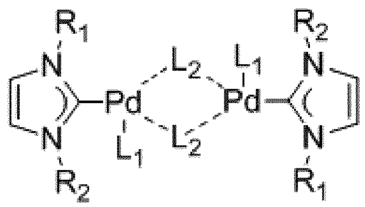

Formule IV

dans laquelle :

$R_1$ et $R_2$ représentent indépendamment l’un de l’autre un groupement choisi parmi :

- alkyle en $C_1$ à $C_{10}$, linéaire ou ramifié,
- cycloalkyle en $C_3$ à $C_{10}$,
- aryle en $C_6$ à $C_{20}$ ou hétéroaryle en $C_3$ à $C_{20}$,
- alkyl-aryle en $C_7$ à $C_{20}$ ou alkyle-hétéroaryle en $C_4$ a $C_{20}$,

$L_1$ et $L_2$ représentent un atome d’halogène choisi parmi Cl, Br et I.

**9.** Utilisation selon l’une des revendications 1 ou 2, dans laquelle le catalyseur répond à la Formule II-1 ou à la Formule II-2 ou à la Formule II-3 :

[Chem 9]

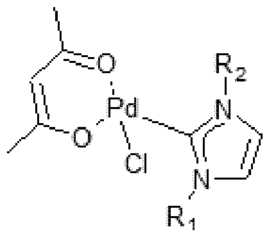

Formule II-1

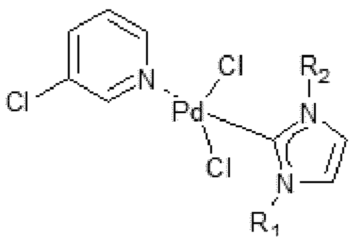

Formule II-2

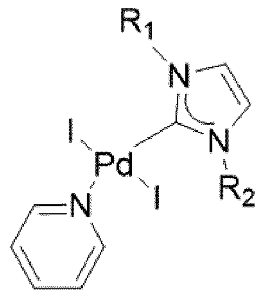

Formule II-3

dans laquelle :

$R_1$ et $R_2$ représentent indépendamment l’un de l’autre un groupement choisi parmi :

- alkyle en $C_1$ à $C_{10}$, linéaire ou ramifié,
- cycloalkyle en $C_3$ à $C_{10}$,
- aryle en $C_6$ à $C_{20}$ ou hétéroaryle en $C_3$ à $C_{20}$, et
- alkyl-aryle en $C_7$ à $C_{20}$ ou alkyle-hétéroaryle en $C_4$ à $C_{20}$,

ledit catalyseur pouvant être lié à un support, notamment un polymère ou de la silice, par au moins un des groupements $R_1$ ou $R_2$,
en particulier ledit catalyseur est choisi parmi :

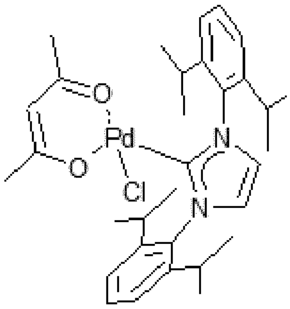
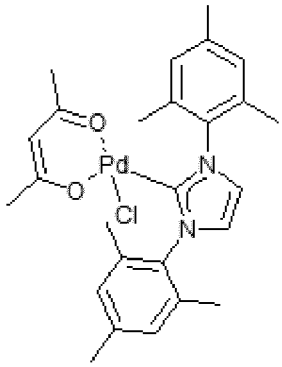
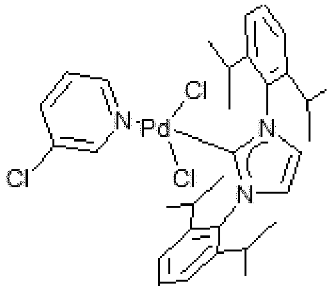

ou dans laquelle le catalyseur, lié à un support (PS) est choisi parmi:

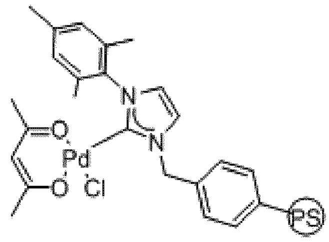
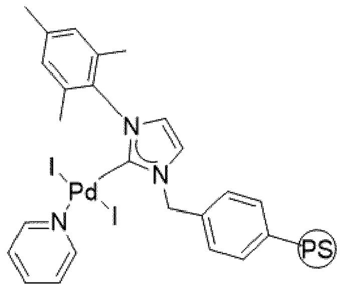

**10.** Utilisation selon l'une des revendications 1 ou 5, dans laquelle le catalyseur répond à la Formule III-1 :

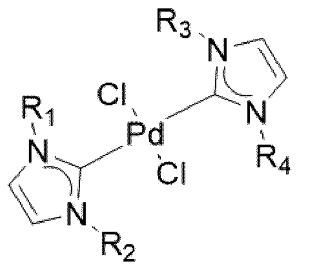

Formule III-1

dans laquelle :

$R_1$, $R_2$, $R_3$ et $R_4$ représentent indépendamment l'un de l'autre un groupement choisi parmi :

- alkyle en $C_1$ à $C_{10}$, linéaire ou ramifié,
- cycloalkyle en $C_3$ à $C_{10}$, et
- aryle en $C_6$ à $C_{20}$ ou hétéroaryle en $C_3$ à $C_{20}$,
- alkyl-aryle en $C_7$ à $C_{20}$ ou alkyle-hétéroaryle en $C_4$ a $C_{20}$,

ledit composé de Formule III-1 pouvant être lié à un support, notamment un polymère ou de la silice, par au moins un des groupements $R_1$, $R_2$, $R_3$ ou $R_4$,
éventuellement l'un des groupements $R_1$ ou $R_2$ peut être relié à $R_3$ ou $R_4$ et représente ensemble un groupement bidentate comprenant deux groupements carbène *N*-hétérocyclique,
en particulier ledit catalyseur de Formule III-1, lié à un support est choisi parmi :

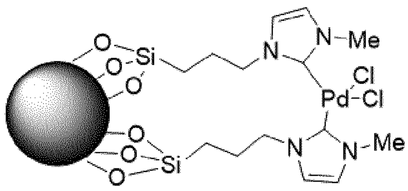

.

**11.** Utilisation selon la revendication 1 d'un catalyseur Pt-NHC, dans la mise en oeuvre d'un procédé de préparation sélective d'oxalates ou d'oxamides, à partir de monoxyde de carbone, d'un oxydant, en particulier l'oxygène moléculaire ou l'air, et d'un alcool ou d'une amine respectivement.

**12.** Procédé de préparation d'un composé oxalate ou d'un composé oxamide comprenant :

- une étape A de mise en contact d'un alcool ou d'une amine, respectivement, avec :
  - du monoxyde de carbone, en particulier utilisé à raison de 1,0 à 10,0 MPa, notamment à 6,5 MPa,
  - un oxydant, en particulier de l'oxygène ou de l'air, de préférence de l'oxygène utilisé à raison de 0,5 à 2,5 MPa,
  - un catalyseur M-NHC, dans lequel M représente Pd ou Pt et NHC représente un groupement carbène *N*-hétérocyclique, comprenant au moins un atome M lié à au moins un ligand carbène *N*-hétérocyclique, et
  - éventuellement un promoteur, en particulier choisi parmi : l'iodure de tétrabutylammonium ($Bu_4NI$), l'iodure de sodium (NaI) et l'iodure de potassium (KI), de préférence l'iodure de tétrabutylammonium,
  - éventuellement une base, en particulier choisi parmi le carbonate de potassium ($K_2CO_3$), le carbonate de sodium ($Na_2CO_3$), le *tert*-butylate de potassium (KotBu), le phosphate de potassium ($K_3PO_4$) et la triéthylamine ($Et_3N$),
  - éventuellement un solvant, en particulier l'acétonitrile, le toluène, l'1,4-dioxane, le tétrahydrofurane, l'éthanol, le méthanol et l'acétate d'éthyle, de préférence l'acétonitrile et le tétrahydrofurane,

pour obtenir un milieu réactionnel,
- éventuellement une étape B de chauffage dudit milieu réactionnel,

pour obtenir le composé oxalate, ou le composé oxamide, en particulier comprenant :

- une étape A de mise en contact d'un alcool ou d'une amine, respectivement, avec :
  - du monoxyde de carbone,
  - de l'oxygène ou de l'air,
  - un catalyseur Pd-NHC,
  - éventuellement un promoteur,
  - éventuellement une base,
  - éventuellement un solvant,

  pour obtenir un milieu réactionnel,

- éventuellement une étape B de chauffage dudit milieu réactionnel,

pour obtenir le composé oxalate, ou le composé oxamide.

**13.** Procédé de préparation selon la revendication 12 d'un composé oxalate comprenant :

- une étape A de mise en contact d'un alcool avec :
  - du monoxyde de carbone,
  - de l'oxygène ou de l'air,
  - un catalyseur Pd-NHC, et
  - éventuellement un promoteur,
  - éventuellement une base,
  - éventuellement un solvant,

pour obtenir un milieu réactionnel,
• une étape B de chauffage dudit milieu réactionnel, pour obtenir le composé oxalate, en particulier effectuée à une température comprise de 25 à 200 °C, notamment de 60 à 110°C, de préférence d'environ 90 °C.

**14.** Procédé de préparation selon la revendication 12 ou 13, d'un composé oxalate de Formule 2, dans lequel l'étape A comprend la mise en contact d'un alcool de Formule 1 :

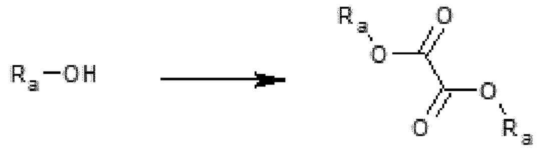

Formule 1 Formule 2

dans lesquelles $R_a$ représente un groupement choisi parmi :

• alkyle en $C_1$ à $C_{10}$, linéaire ou ramifié, et
• cycloalkyle en $C_3$ à $C_{10}$,

en particulier l'alcool est choisi parmi le méthanol, l'éthanol et l'isopropanol.

**15.** Procédé de préparation selon la revendication 12 d'un composé oxamide comprenant :

• une étape A de mise en contact d'une amine avec :

- du monoxyde de carbone,
- de l'oxygène ou de l'air,
- éventuellement un promoteur,
- un catalyseur de type Pd-NHC,
- un solvant, et
- éventuellement une base, en particulier choisi parmi $K_2CO_3$ ou $Et_3N$,

pour obtenir un milieu réactionnel comprenant l'oxamide, en particulier
pour la préparation d'un composé oxamide de Formule 4, dans lequel l'étape A comprend la mise en contact d'une amine de Formule 3 :

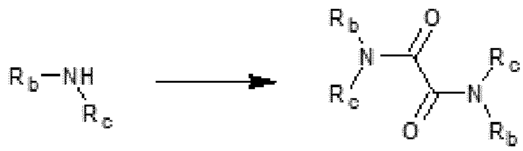

Formule 3 Formule 4

dans lesquelles $R_b$ et $R_c$ représentent indépendamment l'un de l'autre :

• un atome d'hydrogène,
• un groupement alkyle en $C_1$ à $C_{20}$, linéaire ou ramifié,
• un groupement alcényle en $C_2$ à $C_{20}$, linéaire ou ramifié,
• un groupement hétéroalkyle en $C_1$ à $C_{20}$, linéaire ou ramifié, l'hétéroatome étant notamment O ou N,

au moins un des groupements $R_b$ ou $R_c$ étant différent de l'hydrogène,
$R_b$ et $R_c$ pouvant former un cycle,
en particulier l'amine est choisi parmi: la diéthylamine, la pipéridine, la pyrrolidine et la morpholine.

## Patentansprüche

1. Verwendung eines M-NHC-Katalysators, wobei M für Pd oder Pt steht und NHC für eine *N*-heterocyclische Carbengruppe steht, umfassend mindestens ein M-Atom, das an mindestens einen *N*-heterocyclischen Carbenliganden gebunden ist, bei der Durchführung eines Verfahrens zur selektiven Herstellung von Oxalaten oder Oxamiden, ausgehend von Kohlenmonoxid, einem Oxidationsmittel, insbesondere molekularem Sauerstoff oder Luft, und einem Alkohol bzw. einem Amin, gegebenenfalls in Gegenwart eines Promotors.

2. Verwendung nach Anspruch 1, wobei der Katalysator die Formel II aufweist:

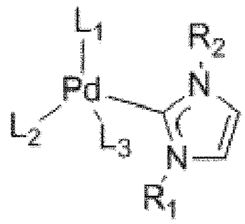

Formel II

wobei:

$R_1$ und $R_2$ unabhängig voneinander für eine Gruppe stehen, ausgewählt aus:

- linearem oder verzweigtem $C_1$- bis $C_{10}$-Alkyl,
- $C_3$- bis $C_{10}$-Cycloalkyl,
- $C_6$- bis $C_{20}$-Aryl oder $C_3$- bis $C_{20}$-Heteroaryl,
- $C_7$- bis $C_{20}$-Alkylaryl oder $C_4$- bis $C_{20}$-Alkylheteroaryl,

$L_1$ für ein Halogenatom steht, ausgewählt aus Cl, Br und I, und

- $L_2$ und $L_3$ entweder für einen zweizähnigen Liganden stehen, insbesondere ausgewählt aus Acetylacetonat (acac), Allyl, Cinnamyl und Acetat, vorzugsweise Acetylacetonat,
- oder $L_2$ für ein Halogenatom steht, ausgewählt aus Cl, Br und I,

und $L_3$ für einen einzähnigen Liganden steht, insbesondere ausgewählt aus: Pyridin, 3-Chlorpyridin, Acetonitril, Triethylamin, oder einen Liganden auf Phosphinbasis, insbesondere Triphenylphosphin, vorzugsweise 3-Chlorpyridin,
wobei die Verbindung der Formel II an einen Träger, insbesondere ein Polymer oder Siliciumdioxid, durch mindestens eine der Gruppen $R_1$ oder $R_2$ gebunden sein kann.

3. Verwendung nach Anspruch 2, wobei der Katalysator die Formel II aufweist, wobei:
$L_1$ für ein Iodatom steht, und

- $L_2$ und $L_3$ entweder aneinander gebunden sind und zusammen für einen zweizähnigen Liganden stehen,
- oder $L_2$ für ein Halogenatom steht, ausgewählt aus Cl, Br und I, und $L_3$ für einen einzähnigen Liganden steht.

4. Verwendung nach einem der Ansprüche 2 oder 3, wobei der Katalysator die Formel II aufweist, wobei:

$L_1$ und $L_2$ jeweils für ein Iodatom stehen,
und $L_3$ für einen einzähnigen Liganden steht.

5. Verwendung nach Anspruch 1, wobei der Katalysator die Formel III aufweist:

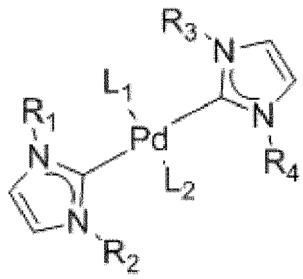

Formel III

wobei:

$R_1$, $R_2$, $R_3$ und $R_4$ unabhängig voneinander für eine Gruppe stehen, ausgewählt aus:

- einem linearen oder verzweigten $C_1$- bis $C_{10}$-Alkyl,
- $C_3$- bis $C_{10}$-Cycloalkyl, und
- $C_6$- bis $C_{20}$-Aryl oder $C_3$- bis $C_{20}$-Heteroaryl,
- $C_7$- bis $C_{20}$-Alkylaryl oder $C_4$- bis $C_{20}$-Alkylheteroaryl,

$L_1$ und $L_2$ unabhängig voneinander entweder für:

- ein Halogenatom, ausgewählt aus Cl, Br und I, oder
- einen einzähnigen Liganden stehen,

oder $L_1$ und $L_2$ aneinander gebunden sind und für einen zweizähnigen Liganden stehen,
wobei die Verbindung der Formel III an einen Träger, insbesondere ein Polymer oder Siliciumdioxid, durch mindestens eine der Gruppen $R_1$, $R_2$, $R_3$ oder $R_4$ gebunden sein kann,
gegebenenfalls eine der Gruppen $R_1$ oder $R_2$ mit $R_3$ oder $R_4$ verbunden sein kann und zusammen für eine zweizähnige Gruppe stehen, umfassend zwei *N*-heterocyclische Carbengruppen.

**6.** Verwendung nach Anspruch 5, wobei der Katalysator die Formel III aufweist, wobei mindestens eine der Gruppen $L_1$ und $L_2$ für ein Iodatom steht.

**7.** Verwendung nach einem der Ansprüche 5 oder 6, wobei der Katalysator die Formel III aufweist, wobei die Gruppen $L_1$ und $L_2$ jeweils für ein Iodatom stehen.

**8.** Verwendung nach Anspruch 1, wobei der Katalysator die Formel IV aufweist:

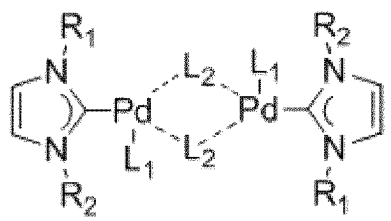

Formel IV

wobei:

$R_1$ und $R_2$ unabhängig voneinander für eine Gruppe stehen, ausgewählt aus:

- einem linearen oder verzweigten $C_1$- bis $C_{10}$-Alkyl,
- $C_3$- bis $C_{10}$-Cycloalkyl,
- $C_6$- bis $C_{20}$-Aryl oder $C_3$- bis $C_{20}$-Heteroaryl,
- $C_7$- bis $C_{20}$-Alkylaryl oder $C_4$- bis $C_{20}$-Alkylheteroaryl,

$L_1$ und $L_2$ für ein Halogenatom stehen, ausgewählt aus Cl, Br und I.

**9.** Verwendung nach einem der Ansprüche 1 oder 2, wobei der Katalysator die Formel II-1 oder die Formel II-2 oder die Formel II-3 aufweist:

[Chem 9]

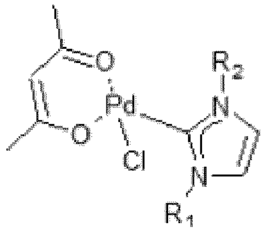

Formel II-1

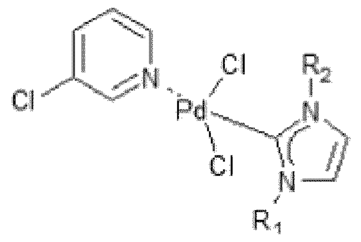

Formel II-2

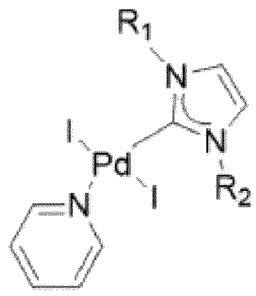

Formel II-3

wobei:

$R_1$ und $R_2$ unabhängig voneinander für eine Gruppe stehen, ausgewählt aus:

- einem linearen oder verzweigten $C_1$- bis $C_{10}$-Alkyl,
- $C_3$- bis $C_{10}$-Cycloalkyl,
- $C_6$- bis $C_{20}$-Aryl oder $C_3$- bis $C_{20}$-Heteroaryl, und
- $C_7$- bis $C_{20}$-Alkylaryl oder $C_4$- bis $C_{20}$-Alkylheteroaryl,

wobei der Katalysator an einen Träger, insbesondere ein Polymer oder Siliciumdioxid, durch mindestens eine der Gruppen $R_1$ oder $R_2$ gebunden sein kann,
wobei der Katalysator ausgewählt ist aus:

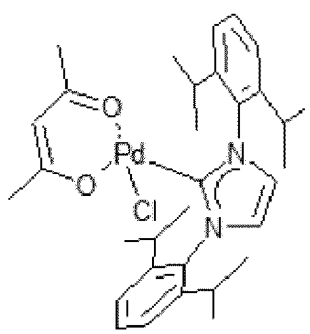

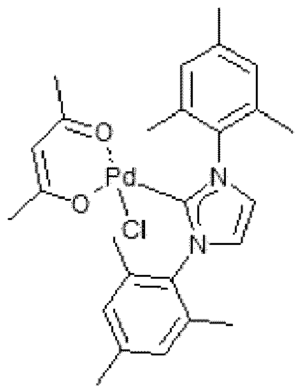

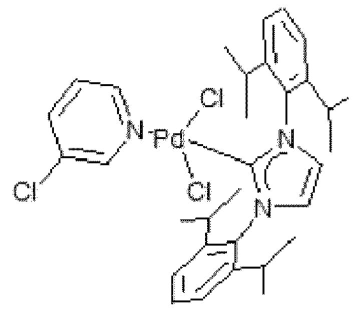

oder wobei der Katalysator, der an einen Träger (PS) gebunden ist, ausgewählt ist aus:

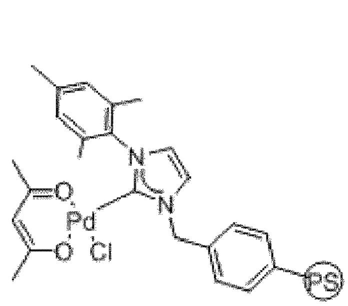

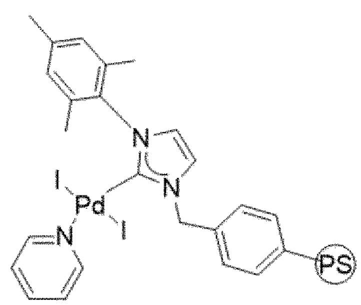

**10.** Verwendung nach einem der Ansprüche 1 oder 5, wobei der Katalysator die Formel III-1 aufweist:

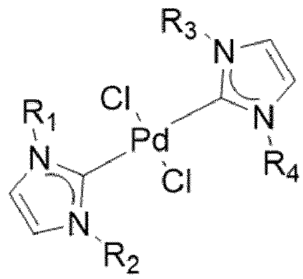

Formel III-1

wobei:

$R_1$, $R_2$, $R_3$ und $R_4$ unabhängig voneinander für eine Gruppe stehen, ausgewählt aus:

- einem linearen oder verzweigten $C_1$- bis $C_{10}$-Alkyl,
- $C_3$- bis $C_{10}$-Cycloalkyl, und
- $C_6$- bis $C_{20}$-Aryl oder $C_3$- bis $C_{20}$-Heteroaryl,
- $C_7$- bis $C_{20}$-Alkylaryl oder $C_4$- bis $C_{20}$-Alkylheteroaryl, wobei die Verbindung der Formel III-1 an einen Träger, insbesondere ein Polymer oder Siliciumdioxid, durch mindestens eine der Gruppen $R_1$, $R_2$, $R_3$ oder $R_4$ gebunden sein kann,

wobei gegebenenfalls eine der Gruppen $R_1$ oder $R_2$ mit $R_3$ oder $R_4$ verbunden sein kann und zusammen für eine zweizähnige Gruppe stehen, umfassend zwei *N*-heterocyclische Carbengruppen,
wobei der Katalysator der Formel III-1, der an einen Träger gebunden ist, ausgewählt ist, aus:

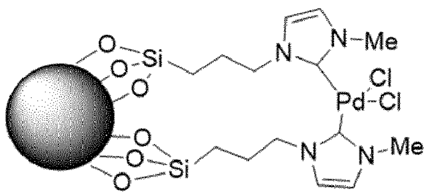

**11.** Verwendung nach Anspruch 1 eines Pt-NHC-Katalysators bei der Durchführung eines Verfahrens zur selektiven Herstellung von Oxalaten oder Oxamiden aus Kohlenmonoxid, einem Oxidationsmittel, insbesondere molekularem Sauerstoff oder Luft, und einem Alkohol bzw. einem Amin.

**12.** Verfahren zur Herstellung einer Oxalatverbindung oder einer Oxamidverbindung, umfassend:

- einen Schritt A zum Inkontaktbringen eines Alkohols bzw. eines Amins mit:

  - Kohlenmonoxid, das insbesondere in einer Menge von 1,0 bis 10,0 MPa, insbesondere 6,5 MPa, verwendet wird,
  - einem Oxidationsmittel, insbesondere Sauerstoff oder Luft, vorzugsweise Sauerstoff, der in einer Menge von 0,5 bis 2,5 MPa verwendet wird,
  - einem M-NHC-Katalysator, wobei M für Pd oder Pt steht und NHC für eine *N*-heterocyclische Carbengruppe steht, umfassend mindestens ein M-Atom, das an mindestens einen *N*-heterocyclischen Carbenliganden gebunden ist, und
  - gegebenenfalls einem Promotor, insbesondere ausgewählt aus: Tetrabutylammoniumiodid ($Bu_4NI$), Natriumiodid (NaI) und Kaliumiodid (KI), vorzugsweise Tetrabutylammoniumiodid,
  - gegebenenfalls einer Base, insbesondere ausgewählt aus Kaliumcarbonat ($K_2CO_3$), Natriumcarbonat ($Na_2CO_3$), Kalium-*tert*-butylat (Ko*t*Bu), Kaliumphosphat ($K_3PO_4$) und Triethylamin ($Et_3N$),
  - gegebenenfalls einem Lösungsmittel, insbesondere Acetonitril, Toluol, 1,4-Dioxan, Tetrahydrofuran, Ethanol, Methanol und Ethylacetat, vorzugsweise Acetonitril und Tetrahydrofuran,

um ein Reaktionsmedium zu erhalten,
• gegebenenfalls einen Schritt B zum Erhitzen des Reaktionsmediums,
um die Oxalatverbindung oder die Oxamidverbindung zu erhalten, insbesondere umfassend:

- einen Schritt A zum Inkontaktbringen eines Alkohols bzw. eines Amins mit:

  - Kohlenmonoxid,
  - Sauerstoff oder Luft,
  - einem Pd-NHC-Katalysator,
  - gegebenenfalls einem Promoter,
  - gegebenenfalls einer Base,
  - gegebenenfalls einem Lösungsmittel,

  um ein Reaktionsmedium zu erhalten,
- gegebenenfalls einen Schritt B zum Erhitzen des Reaktionsmediums,

um die Oxalatverbindung oder die Oxamidverbindung zu erhalten.

**13.** Verfahren zur Herstellung einer Oxalatverbindung nach Anspruch 12, umfassend:

- einen Schritt A zum Inkontaktbringen eines Alkohols mit:

  - Kohlenmonoxid,
  - Sauerstoff oder Luft,
  - einem Pd-NHC-Katalysator, und
  - gegebenenfalls einem Promoter,
  - gegebenenfalls einer Base,
  - gegebenenfalls einem Lösungsmittel,

um ein Reaktionsmedium zu erhalten,
• einen Schritt B zum Erhitzen des Reaktionsmediums, um die Oxalatverbindung zu erhalten, der insbesondere bei einer Temperatur zwischen 25 bis 200 °C, insbesondere zwischen 60 und 110 °C, vorzugsweise etwa 90 °C durchgeführt wird.

**14.** Verfahren nach Anspruch 12 oder 13 zur Herstellung einer Oxalatverbindung der Formel 2, wobei Schritt A das Inkontaktbringen eines Alkohols der Formel 1 umfasst:

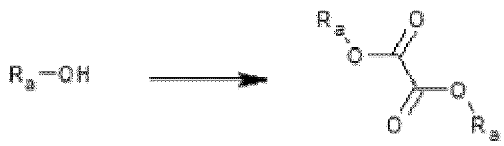

Formel 1 Formel 2

wobei $R_a$ für eine Gruppe steht, ausgewählt aus:

- einem linearen oder verzweigten $C_1$- bis $C_{10}$-Alkyl und
- $C_3$- bis $C_{10}$-Cycloalkyl,

wobei der Alkohol insbesondere ausgewählt ist aus Methanol, Ethanol und Isopropanol.

**15.** Verfahren nach Anspruch 12 zur Herstellung einer Oxamidverbindung, umfassend:

- einen Schritt A zum Inkontaktbringen eines Amins mit:

  - Kohlenmonoxid,
  - Sauerstoff oder Luft,
  - gegebenenfalls einem Promoter,

- einem Katalysator vom Typ Pd-NHC,
- einem Lösungsmittel, und
- gegebenenfalls einer Base, insbesondere ausgewählt aus $K_2CO_3$ oder $Et_3N$,

um ein Reaktionsmedium zu erhalten, das Oxamid umfasst, insbesondere
zur Herstellung einer Oxamidverbindung der Formel 4, wobei Schritt A das Inkontaktbringen eines Amins der Formel 3 umfasst:

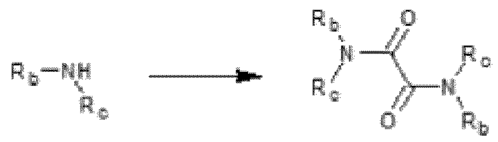

Formel 3 Formel 4

wobei $R_b$ und $R_c$ unabhängig voneinander entweder für:

- ein Wasserstoffatom,
- eine lineare oder verzweigte $C_1$- bis $C_{20}$-Alkylgruppe,
- eine lineare oder verzweigte $C_2$- bis $C_{20}$-Alkenylgruppe,
- eine lineare oder verzweigte $C_1$- bis $C_{20}$-Heteroalkylgruppe, wobei das Heteroatom insbesondere O oder N ist,

stehen,
wobei mindestens eine der Gruppen $R_b$ oder $R_c$ verschieden von Wasserstoff ist,
$R_b$ und $R_c$ einen Ring bilden können,
wobei das Amin insbesondere ausgewählt ist aus: Diethylamin, Piperidin, Pyrrolidin und Morpholin.

**Claims**

**1.** Use of an M-NHC catalyst, in which M represents Pd or Pt and NHC represents an *N-heterocyclic* carbene group, comprising at least one M atom linked to at least one *N-heterocyclic* carbene ligand, in the implementation of a method for selectively preparing oxalates or oxamides, from carbon monoxide, an oxidant, in particular molecular oxygen or air, and an alcohol or an amine respectively, optionally in the presence of a promoter.

**2.** Use according to claim 1, in which the catalyst corresponds to Formula II :

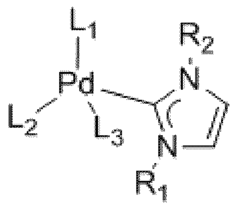

Formula II

in which :

$R_1$ and $R_2$ independently of one another represent a group chosen from :

- $C_1$ to $C_{10}$ linear or branched alkyl,
- $C_3$ to $C_{10}$ cycloalkyl,
- $C_6$ to $C_{20}$ aryl or $C_3$ to $C_{20}$ heteroaryl, and
- $C_7$ to $C_{20}$ alkyl-aryl or $C_4$ to $C_{20}$ alkyl-heteroaryl,

$L_1$ represents a halogen atom chosen from Cl, Br and I, and

- either $L_2$ and $L_3$ represent a bidentate ligand, in particular chosen from acetylacetonate (acac), allyl, cinnamyl and acetate, preferably acetylacetonate,

• or $L_2$ represents a halogen atom chosen from Cl, Br and I,

and $L_3$ represents a monodentate ligand, in particular selected from: pyridine, 3-chloropyridine, acetonitrile, triethylamine, or a phosphine-based ligand, in particular triphenylphosphine, preferably 3-chloropyridine, said compound of Formula II may be bonded to a support, in particular a polymer or silica, by at least one of the groups $R_1$ or $R_2$.

**3.** Use according to claim 2, in which the catalyst corresponds to Formula II , in which :
$L_1$ represents an iodine atom, and

• either $L_2$ and $L_3$ are linked and together represent a bidentate ligand,
• or $L_2$ represents a halogen atom chosen from Cl, Br and I and $L_3$ represents a monodentate ligand.

**4.** Use according to one of claims 2 or 3, in which the catalyst corresponds to Formula II , in which :

$L_1$ and $L_2$ each represent an iodine atom
and $L_3$ represents a monodentate ligand.

**5.** Use according to claim 1, in which the catalyst corresponds to Formula III :

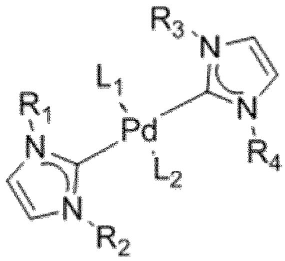

Formula III

in which :

$R_1$, $R_2$, $R_3$ and $R_4$ independently of one another represent a group selected from :

• $C_1$ to $C_{10}$ linear or branched alkyl
• $C_3$ to $C_{10}$ cycloalkyl
• $C_6$ to $C_{20}$ aryl or $C_3$ to $C_{20}$ heteroaryl, and
• $C_7$ to $C_{20}$ alkyl-aryl or $C_4$ to $C_{20}$ alkyl-heteroaryl,

either $L_1$ and $L_2$ represent each other independently:

- a halogen atom selected from Cl, Br and I, or
- a monodentate ligand,

or $L_1$ and $L_2$ are linked and represent a bidentate ligand,
said compound of Formula II can be bonded to a support in particular a polymer or silica, by at least one of the groups $R_1$, $R_2$, $R_3$ or $R_4$,
optionally one of the groups $R_1$ or $R_2$ can be linked to $R_3$ or $R_4$ and together represent a bidentate group comprising two *N-heterocyclic* carbene groups.

**6.** Use according to claim 5, in which the catalyst corresponds to Formula III in which at least one of the groups $L_1$ and $L_2$ represents an iodine atom.

**7.** Use according to one of claims 5 or 6, in which the catalyst corresponds to Formula III in which the groups $L_1$ and $L_2$ each represent an iodine atom.

**8.** Use according to claim 1, in which the catalyst corresponds to Formula IV :

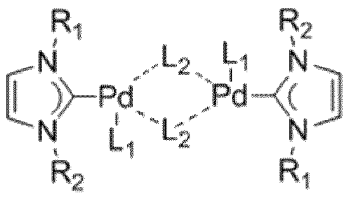

Formula IV

in which :

$R_1$ and $R_2$ independently of one another represent a group chosen from :

- $C_1$ to $C_{10}$ linear or branched alkyl,
- $C_3$ to $C_{10}$ cycloalkyl,
- $C_6$ to $C_{20}$ aryl or $C_3$ to $C_{20}$ heteroaryl, and
- $C_7$ to $C_{20}$ alkyl-aryl or $C_4$ to $C_{20}$ alkyl-heteroaryl,

$L_1$ and $L_2$ represent a halogen atom chosen from Cl, Br and I.

**9.** Use according to one of claims 1 or 2, in which the catalyst corresponds to Formula II-1 or Formula II-2 or Formula II-3 :

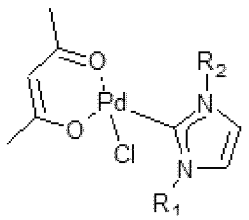

Formula II-1

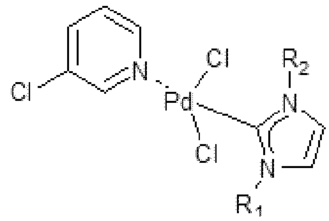

Formula II-2

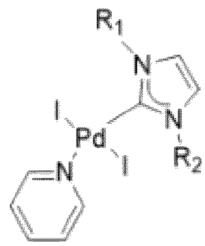

Formula II-3

in which :

$R_1$ and $R_2$ independently of one another represent a group chosen from :

- $C_1$ to $C_{10}$ linear or branched alkyl,
- $C_3$ to $C_{10}$ cycloalkyl,
- $C_6$ to $C_{20}$ aryl or $C_3$ to $C_{20}$ heteroaryl, and
- $C_7$ to $C_{20}$ alkyl-aryl or $C_4$ to $C_{20}$ alkyl-heteroaryl,

said catalyst may be bonded to a support, in particular a polymer or silica, by at least one of the groups $R_1$ or $R_2$ , in particular said catalyst is selected from :

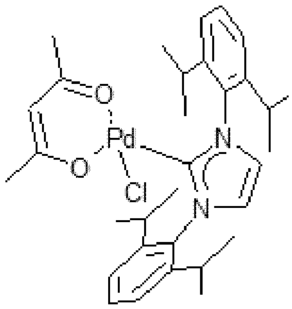 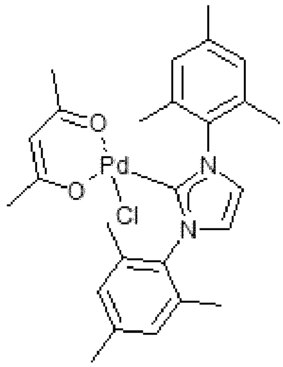 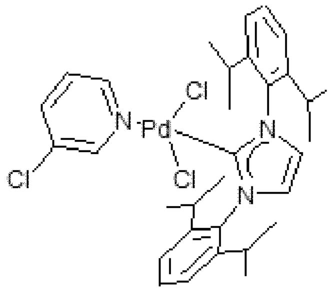

or in which the catalyst, bound to a support (PS), is chosen from:

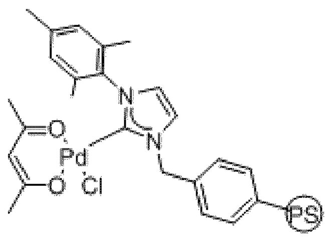 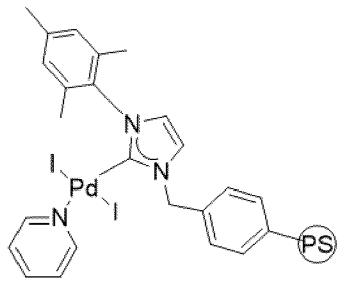

**10.** Use according to one of claims 1 or 5, wherein the catalyst corresponds to Formula III-1 :

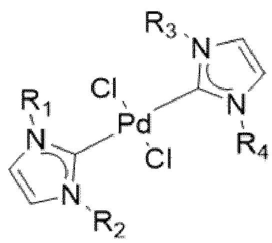

Formula III-1

in which :

$R_1$, $R_2$, $R_3$ and $R_4$ independently of one another represent a group selected from :

- $C_1$ to $C_{10}$ linear or branched alkyl,
- $C_3$ to $C_{10}$ cycloalkyl,
- $C_6$ to $C_{20}$ aryl or $C_3$ to $C_{20}$ heteroaryl, and
- $C_7$ to $C_{20}$ alkyl-aryl or $C_4$ to $C_{20}$ alkyl-heteroaryl,

said compound of Formula III-1 may be bonded to a support, in particular a polymer or silica, by at least one of the groups $R_1$ , $R_2$ , $R_3$ or $R_4$,
optionally one of the groups $R_1$ or $R_2$ can be linked to $R_3$ or $R_4$ and together represent a bidentate group comprising two *N-heterocyclic* carbene groups,
in particular said supported catalyst of Formula III-1 is selected from :

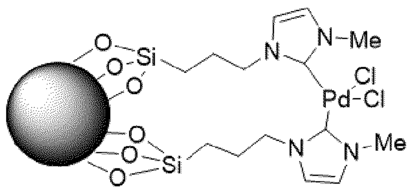

.

**11.** Use according to claim 1 of a Pt-NHC catalyst in a method for selectively preparing oxalates or oxamides from carbon monoxide, an oxidant, in particular molecular oxygen or air, and an alcohol or an amine respectively.

**12.** A method for preparing an oxalate compound or an oxamide compound comprising :

• a step A of contacting an alcohol or an amine, respectively, with :

- carbon monoxide, in particular used from 1.0 to 10.0 MPa, in particular 6.5 MPa,
- an oxidant, in particular oxygen or air, preferably oxygen used at 0.5 to 2.5 MPa,
- an M-NHC catalyst, in which M represents Pd or Pt and NHC represents an N-heterocyclic carbene group, comprising at least one M atom linked to at least one *N-heterocyclic* carbene ligand, and
- optionally a promoter, in particular selected from: tetrabutylammonium iodide ($Bu_4NI$), sodium iodide (NaI) and potassium iodide (KI), preferably tetrabutylammonium iodide,
- optionally a base, in particular selected from potassium carbonate ($K_2CO_3$), sodium carbonate ($Na_2CO_3$), potassium *tert-butylate (KotBu),* potassium phosphate ($K_3PO_4$) and triethylamine ($Et_3N$),
- optionally a solvent, in particular acetonitrile, toluene, 1,4-dioxane, tetrahydrofuran, ethanol, methanol and ethyl acetate, preferably acetonitrile and tetrahydrofuran,

to obtain a reaction medium,
• optionally a step B of heating said reaction medium,

to obtain the oxalate compound or the oxamide compound,
in particular comprising :

• a step A of contacting an alcohol or an amine, respectively, with :

- carbon monoxide,
- oxygen or air,
- a Pd-NHC catalyst,
- optionally a promoter,
- optionally a base,
- optionally a solvent,

to obtain a reaction medium,

• optionally a step B of heating said reaction medium,

to obtain the oxalate compound or the oxamide compound.

**13.** A method for the preparation according to claim 12 of an oxalate compound comprising :

• a step A of contacting an alcohol with :

- carbon monoxide,
- oxygen or air,
- a Pd-NHC catalyst, and
- optionally a promoter,
- optionally a base,
- optionally a solvent,

to obtain a reaction medium,

• a step B of heating said reaction medium, to obtain the oxalate compound, in particular at a temperature from 25 to 200°C, in particular from 60 to 110°C, preferably about 90°C.

**14.** A method for the preparation according to any of claims 12 or 13, of an oxalate compound of Formula 2, wherein step A comprises contacting an alcohol of Formula 1 :

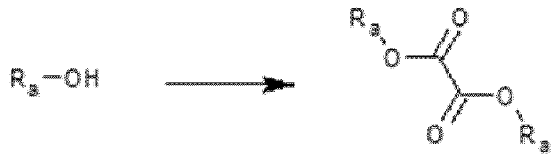

Formula 1 Formula 2

in which $R_a$ represents a group chosen from :

• $C_1$ to $C_{10}$ linear or branched alkyl, and
• $C_3$ to $C_{10}$ cycloalkyl

in particular the alcohol is chosen from methanol, ethanol and isopropanol.

**15.** A method for the preparation according to claim 12 of an oxamide compound comprising :

• a step A of contacting an amine with :

- carbon monoxide,
- oxygen or air,
- optionally a promoter,
- a Pd-NHC type catalyst,
- • a solvent, and
- optionally a base, in particular selected from $K_2CO_3$ or $Et_3N$,

to obtain a reaction medium comprising the oxamide,
in particular for the preparation of an oxamide compound of Formula 4, wherein step A comprises contacting an amine of Formula 3 :

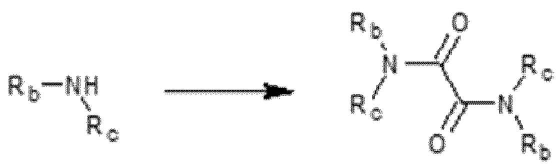

Formula 3 Formula 4

in which $R_b$ and $R_c$ independently of one another represent :

• a hydrogen atom,
• $C_1$ to $C_{20}$ linear or branched alkyl group
• $C_2$ to $C_{20}$ linear or branched alkenyl group
• $C_1$ to $C_{20}$ linear or branched heteroalkyl group, the heteroatom being in particular O or N,

at least one of the groups $R_b$ or $R_c$ being other than hydrogen,
$R_b$ and $R_c$ can form a cycle,
in particular the amine is chosen from: diethylamine, piperidine, pyrrolidine and morpholine.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 4005129 A
- US 4614832 A
- US 3393136 A
- US 4005130 A
- US 4005128 A


### Littérature non-brevet citée dans la description


- **N. MARION et al.** *Adv. Synth. Catal.*, 2007, vol. 349, 2380-2384
- **D.-H. LEE et al.** *Org. Lett.*, 2008, vol. 10, 1609-1612